# EUROPEAN PATENT SPECIFICATION

(11) **EP 3 387 011 B1**
(45) Date of publication and mention of the grant of the patent: **20.05.2020**
(21) Application number: 16822910.2
(22) Date of filing: 07.12.2016
(51) Int. Cl.: C07K 14/705, C12N 5/071

(54) **A RENAL CELL LINE WITH STABLE TRANSPORTER EXPRESSION**
RENALE ZELLLINIE MIT STABILER TRANSPORTEXPRESSION
LIGNÉE DE CELLULES RÉNALES AVEC UNE EXPRESSION DE TRANSPORT STABLE

(30) Priority: 08.12.2015 EP 15198421
(43) Date of publication of application: 17.10.2018
(73) Proprietor: Stichting Katholieke Universiteit, 6525 GA Nijmegen (NL)
(72) Inventor: WILMER, Martijn, 6525 EZ Nijmegen (NL); MASEREEUW, Rosalinde, 3584 CG Utrecht (NL)
(74) Representative: Nederlandsch Octrooibureau
(86) International application number: PCT/EP2016/080026
(87) International publication number: WO 2017/097815

(56) References cited:
- EP-A1- 2 496 687
- WO-A1-2015/069192
- BROWN C D A ET AL: "Characterisation of human tubular cell monolayers as a model of proximal tubular xenobiotic handling", TOXICOLOGY AND APPLIED PHARMACOLOGY, ACADEMIC PRESS, AMSTERDAM, NL, vol. 233, no. 3, 15 December 2008 (2008-12-15), pages 428-438, XP025678925, ISSN: 0041-008X, DOI: 10.1016/J.TAAP.2008.09.018 [retrieved on 2008-10-01]
- Anonymous: "(epi)genetics in pharmacology", , 24 April 2015 (2015-04-24), XP055264362, Retrieved from the Internet: URL:http://www.nvfarmaco.nl/assets/docs/Ab stracts_NVF_Voorjaarsdag_2015.pdf [retrieved on 2016-04-11]
- "Abstract Book - NfN Fall Symposium 2014", Abstracts - NfN Fall Symposium, 1 January 2014 (2014-01-01), XP055264376, Retrieved from the Internet: URL:http://www.nefro.nl/uploads/Er/e1/Ere1 8-CBMxI5GezCbqpivg/141001-Abstracts_NfN-Fa ll-Symposium-2014_FINALa.pdf [retrieved on 2016-04-11]
- NIESKENS TOM T ET AL: "A Human Renal Proximal Tubule Cell Line with Stable Organic Anion Transporter 1 and 3 Expression Predictive for Antiviral-Induced Toxicity", THE AAPS JOURNAL, SPRINGER US, NEW YORK, vol. 18, no. 2, 28 January 2016 (2016-01-28), pages 465-475, XP035642017, DOI: 10.1208/S12248-016-9871-8
- NIESKENS TOM T G ET AL: "Kidney-on-a-chip technology for renal proximal tubule tissue reconstruction", EUROPEAN JOURNAL OF PHARMACOLOGY, ELSEVIER SCIENCE, NL, vol. 790, 9 July 2016 (2016-07-09), pages 46-56, XP029758027, ISSN: 0014-2999, DOI: 10.1016/J.EJPHAR.2016.07.018

### SEQUENCE LISTING

<110> Stichting Katholieke Universiteit
<120> A renal cell line with stable transporter expression
<130> P6057046PCT
<150> EP15198421.8
   <151> 2015-12-08
<160> 28
<170> PatentIn version 3.3
<210> 1
   <211> 550
   <212> PRT
   <213> Homo sapiens
<400> 1
   Met Ala Phe Asn Asp Leu Leu Gln Gln Val Gly Gly Val Gly Arg Phe
   1               5                   10                  15
   Gln Gln Ile Gln Val Thr Leu Val Val Leu Pro Leu Leu Leu Met Ala
               20                  25                  30
   Ser His Asn Thr Leu Gln Asn Phe Thr Ala Ala Ile Pro Thr His His
           35                  40                  45
   Cys Arg Pro Pro Ala Asp Ala Asn Leu Ser Lys Asn Gly Gly Leu Glu
       50                  55                  60
   Val Trp Leu Pro Arg Asp Arg Gln Gly Gln Pro Glu Ser Cys Leu Arg
   65                  70                  75                  80
   Phe Thr Ser Pro Gln Trp Gly Leu Pro Phe Leu Asn Gly Thr Glu Ala
                   85                  90                  95
   Asn Gly Thr Gly Ala Thr Glu Pro Cys Thr Asp Gly Trp Ile Tyr Asp
               100                 105                 110
   Asn Ser Thr Phe Pro Ser Thr Ile Val Thr Glu Trp Asp Leu Val Cys
           115                 120                 125
   Ser His Arg Ala Leu Arg Gln Leu Ala Gln Ser Leu Tyr Met Val Gly
       130                 135                 140
   Val Leu Leu Gly Ala Met Val Phe Gly Tyr Leu Ala Asp Arg Leu Gly
   145                 150                 155                 160
   Arg Arg Lys Val Leu Ile Leu Asn Tyr Leu Gln Thr Ala Val Ser Gly
   165 170 175
   Thr Cys Ala Ala Phe Ala Pro Asn Phe Pro Ile Tyr Cys Ala Phe Arg
   180 185 190
   Leu Leu Ser Gly Met Ala Leu Ala Gly Ile Ser Leu Asn Cys Met Thr
   195 200 205
   Leu Asn Val Glu Trp Met Pro Ile His Thr Arg Ala Cys Val Gly Thr
   210 215 220
   Leu Ile Gly Tyr Val Tyr Ser Leu Gly Gln Phe Leu Leu Ala Gly Val
   225 230 235 240
   Ala Tyr Ala Val Pro His Trp Arg His Leu Gln Leu Leu Val Ser Ala
   245 250 255
   Pro Phe Phe Ala Phe Phe Ile Tyr Ser Trp Phe Phe Ile Glu Ser Ala
   260 265 270
   Arg Trp His Ser Ser Ser Gly Arg Leu Asp Leu Thr Leu Arg Ala Leu
   275 280 285
   Gln Arg Val Ala Arg Ile Asn Gly Lys Arg Glu Glu Gly Ala Lys Leu
   290 295 300
   Ser Met Glu Val Leu Arg Ala Ser Leu Gln Lys Glu Leu Thr Met Gly
   305 310 315 320
   Lys Gly Gln Ala Ser Ala Met Glu Leu Leu Arg Cys Pro Thr Leu Arg
   325 330 335
   His Leu Phe Leu Cys Leu Ser Met Leu Trp Phe Ala Thr Ser Phe Ala
   340 345 350
   Tyr Tyr Gly Leu Val Met Asp Leu Gln Gly Phe Gly Val Ser Ile Tyr
   355 360 365
   Leu Ile Gln Val Ile Phe Gly Ala Val Asp Leu Pro Ala Lys Leu Val
   370 375 380
   Gly Phe Leu Val Ile Asn Ser Leu Gly Arg Arg Pro Ala Gln Met Ala
   385 390 395 400
   Ala Leu Leu Leu Ala Gly Ile Cys Ile Leu Leu Asn Gly Val Ile Pro
   405 410 415
   Gln Asp Gln Ser Ile Val Arg Thr Ser Leu Ala Val Leu Gly Lys Gly
               420                 425                 430
   Cys Leu Ala Ala Ser Phe Asn Cys Ile Phe Leu Tyr Thr Gly Glu Leu
           435                 440                 445
   Tyr Pro Thr Met Ile Arg Gln Thr Gly Met Gly Met Gly Ser Thr Met
       450                 455                 460
   Ala Arg Val Gly Ser Ile Val Ser Pro Leu Val Ser Met Thr Ala Glu
   465                 470                 475                 480
   Leu Tyr Pro Ser Met Pro Leu Phe Ile Tyr Gly Ala Val Pro Val Ala
                   485                 490                 495
   Ala Ser Ala Val Thr Val Leu Leu Pro Glu Thr Leu Gly Gln Pro Leu
               500                 505                 510
   Pro Asp Thr Val Gln Asp Leu Glu Ser Arg Lys Gly Lys Gln Thr Arg
           515                 520                 525
   Gln Gln Gln Glu His Gln Lys Tyr Met Val Pro Leu Gln Ala Ser Ala
       530                 535                 540
   Gln Glu Lys Asn Gly Leu
   545                 550
<210> 2
   <211> 1653
   <212> DNA
   <213> Homo sapiens
<400> 2
   atggccttta atgacctcct gcagcaggtg gggggtgtcg gccgcttcca gcagatccag      60
   gtcaccctgg tggtcctccc cctgctcctg atggcttctc acaacaccct gcagaacttc     120
   actgctgcca tccctaccca ccactgccgc ccgcctgccg atgccaacct cagcaagaac     180
   ggggggctgg aggtctggct gccccgggac aggcaggggc agcctgagtc ctgcctccgc     240
   ttcacctccc cgcagtgggg actgcccttt ctcaatggca cagaagccaa tggcacaggg     300
   gccacagagc cctgcaccga tggctggatc tatgacaaca gcaccttccc atctaccatc     360
   gtgactgagt gggaccttgt gtgctctcac agggccctac gccagctggc ccagtccttg     420
   tacatggtgg gggtgctgct cggagccatg gtgttcggct accttgcaga caggctaggc     480
   cgccggaagg tactcatctt gaactacctg cagacagctg tgtcagggac ctgcgcagcc     540
   ttcgcaccca acttccccat ctactgcgcc ttccggctcc tctcgggcat ggctctggct     600
   ggcatctccc tcaactgcat gacactgaat gtggagtgga tgcccattca cacacgggcc     660
   tgcgtgggca ccttgattgg ctatgtctac agcctgggcc agttcctcct ggctggtgtg     720
   gcctacgctg tgccccactg gcgccacctg cagctactgg tctctgcgcc tttttttgcc     780
   ttcttcatct actcctggtt cttcattgag tcggcccgct ggcactcctc ctccgggagg     840
   ctggacctca ccctgagggc cctgcagaga gtcgcccgga tcaatgggaa gcgggaagaa     900
   ggagccaaat tgagtatgga ggtactccgg gccagtctgc agaaggagct gaccatgggc     960
   aaaggccagg catcggccat ggagctgctg cgctgcccca ccctccgcca cctcttcctc    1020
   tgcctctcca tgctgtggtt tgccactagc tttgcatact atgggctggt catggacctg    1080
   cagggctttg gagtcagcat ctacctaatc caggtgatct ttggtgctgt ggacctgcct    1140
   gccaagcttg tgggcttcct tgtcatcaac tccctgggtc gccggcctgc ccagatggct    1200
   gcactgctgc tggcaggcat ctgcatcctg ctcaatgggg tgatacccca ggaccagtcc    1260
   attgtccgaa cctctcttgc tgtgctgggg aagggttgtc tggctgcctc cttcaactgc    1320
   atcttcctgt atactgggga actgtatccc acaatgatcc ggcagacagg catgggaatg    1380
   ggcagcacca tggcccgagt gggcagcatc gtgagcccac tggtgagcat gactgccgag    1440
   ctctacccct ccatgcctct cttcatctac ggtgctgttc ctgtggccgc cagcgctgtc    1500
   actgtcctcc tgccagagac cctgggccag ccactgccag acacggtgca ggacctggag    1560
   agcaggaaag ggaaacagac gcgacagcaa caagagcacc agaagtatat ggtcccactg    1620
   caggcctcag cacaagagaa gaatggactc tag                                 1653
<210> 3
   <211> 542
   <212> PRT
   <213> Homo sapiens
<400> 3
   Met Thr Phe Ser Glu Ile Leu Asp Arg Val Gly Ser Met Gly His Phe
   1               5                   10                  15
   Gln Phe Leu His Val Ala Ile Leu Gly Leu Pro Ile Leu Asn Met Ala
               20                  25                  30
   Asn His Asn Leu Leu Gln Ile Phe Thr Ala Ala Thr Pro Val His His
           35                  40                  45
   Cys Arg Pro Pro His Asn Ala Ser Thr Gly Pro Trp Val Leu Pro Met
       50                  55                  60
   Gly Pro Asn Gly Lys Pro Glu Arg Cys Leu Arg Phe Val His Pro Pro
   65                  70                  75                  80
   Asn Ala Ser Leu Pro Asn Asp Thr Gln Arg Ala Met Glu Pro Cys Leu
   85 90 95
   Asp Gly Trp Val Tyr Asn Ser Thr Lys Asp Ser Ile Val Thr Glu Trp
   100 105 110
   Asp Leu Val Cys Asn Ser Asn Lys Leu Lys Glu Met Ala Gln Ser Ile
   115 120 125
   Phe Met Ala Gly Ile Leu Ile Gly Gly Leu Val Leu Gly Asp Leu Ser
   130 135 140
   Asp Arg Phe Gly Arg Arg Pro Ile Leu Thr Cys Ser Tyr Leu Leu Leu
   145 150 155 160
   Ala Ala Ser Gly Ser Gly Ala Ala Phe Ser Pro Thr Phe Pro Ile Tyr
   165 170 175
   Met Val Phe Arg Phe Leu Cys Gly Phe Gly Ile Ser Gly Ile Thr Leu
   180 185 190
   Ser Thr Val Ile Leu Asn Val Glu Trp Val Pro Thr Arg Met Arg Ala
   195 200 205
   Ile Met Ser Thr Ala Leu Gly Tyr Cys Tyr Thr Phe Gly Gln Phe Ile
   210 215 220
   Leu Pro Gly Leu Ala Tyr Ala Ile Pro Gln Trp Arg Trp Leu Gln Leu
   225 230 235 240
   Thr Val Ser Ile Pro Phe Phe Val Phe Phe Leu Ser Ser Trp Trp Thr
   245 250 255
   Pro Glu Ser Ile Arg Trp Leu Val Leu Ser Gly Lys Ser Ser Lys Ala
   260 265 270
   Leu Lys Ile Leu Arg Arg Val Ala Val Phe Asn Gly Lys Lys Glu Glu
   275 280 285
   Gly Glu Arg Leu Ser Leu Glu Glu Leu Lys Leu Asn Leu Gln Lys Glu
   290 295 300
   Ile Ser Leu Ala Lys Ala Lys Tyr Thr Ala Ser Asp Leu Phe Arg Ile
   305 310 315 320
   Pro Met Leu Arg Arg Met Thr Phe Cys Leu Ser Leu Ala Trp Phe Ala
   325 330 335
   Thr Gly Phe Ala Tyr Tyr Ser Leu Ala Met Gly Val Glu Glu Phe Gly
               340                 345                 350
   Val Asn Leu Tyr Ile Leu Gln Ile Ile Phe Gly Gly Val Asp Val Pro
           355                 360                 365
   Ala Lys Phe Ile Thr Ile Leu Ser Leu Ser Tyr Leu Gly Arg His Thr
       370                 375                 380
   Thr Gln Ala Ala Ala Leu Leu Leu Ala Gly Gly Ala Ile Leu Ala Leu
   385                 390                 395                 400
   Thr Phe Val Pro Leu Asp Leu Gln Thr Val Arg Thr Val Leu Ala Val
                   405                 410                 415
   Phe Gly Lys Gly Cys Leu Ser Ser Ser Phe Ser Cys Leu Phe Leu Tyr
               420                 425                 430
   Thr Ser Glu Leu Tyr Pro Thr Val Ile Arg Gln Thr Gly Met Gly Val
           435                 440                 445
   Ser Asn Leu Trp Thr Arg Val Gly Ser Met Val Ser Pro Leu Val Lys
       450                 455                 460
   Ile Thr Gly Glu Val Gln Pro Phe Ile Pro Asn Ile Ile Tyr Gly Ile
   465                 470                 475                 480
   Thr Ala Leu Leu Gly Gly Ser Ala Ala Leu Phe Leu Pro Glu Thr Leu
                   485                 490                 495
   Asn Gln Pro Leu Pro Glu Thr Ile Glu Asp Leu Glu Asn Trp Ser Leu
               500                 505                 510
   Arg Ala Lys Lys Pro Lys Gln Glu Pro Glu Val Glu Lys Ala Ser Gln
           515                 520                 525
   Arg Ile Pro Leu Gln Pro His Gly Pro Gly Leu Gly Ser Ser
       530                 535                 540
<210> 4
   <211> 1440
   <212> DNA
   <213> Homo sapiens
<400> 4
   atgaccttct cggagatcct ggaccgtgtg ggaagcatgg gccatttcca gttcctgcat 60
   gtagccatac tgggcctccc gatcctcaac atggccaacc acaacctgct gcagatcttc 120
   acagccgcca cccctgtcca ccactgtcgc ccgccccaca atgcctccac agggccttgg 180
   gtgctcccca tgggcccaaa tgggaagcct gagaggtgcc tccgttttgt acatccgccc 240
   aatgccagcc tgcccaatga cacccagagg gccatggagc catgcctgga tggctgggtc 300
   tacaacagca ccaaggactc cattgtgaca gagtgggact tggtgtgcaa ctccaacaaa 360
   ctgaaggaga tggcccagtc tatcttcatg gcaggtatac tgattggagg gctcgtgctt 420
   ggagacctgt ctgacaggtt tggccgcagg cccatcctga cctgcagcta cctgctgctg 480
   gcagccagcg gctccggtgc agccttcagc cccaccttcc ccatctacat ggtcttccgc 540
   ttcctgtgtg gctttggcat ctcaggcatt accctgagca ccgtcatctt gaatgtggaa 600
   tgggtgccta cccggatgcg ggccatcatg tcgacagcac tcgggtactg ctacaccttt 660
   ggccagttca ttctgcccgg cctggcctac gccatccccc agtggcgttg gctgcagtta 720
   actgtgtcca ttcccttctt cgtcttcttc ctatcatcct ggtggacacc agagtccata 780
   cgctggttgg tcttgtctgg aaagtcctcg aaggccctga agatactccg gcgggtggct 840
   gtcttcaatg gcaagaagga agagggagaa aggctcagct tggaggagct caaactcaac 900
   ctgcagaagg agatctcctt ggccaaggcc aagtacaccg caagtgacct gttccggata 960
   cccatgctgc gccgcatgac cttctgtctt tccctggcct ggtttgctac cggttttgcc 1020
   tactatagtt tggctatggg tgtggaagaa tttggagtca acctctacat cctccagatc 1080
   atctttggtg gggtcgatgt cccagccaag ttcatcacca tcctctcctt aagctacctg 1140
   ggccggcata ccactcaggc cgctgccctg ctcctggcag gaggggccat cttggctctc 1200
   acctttgtgc ccttggactt gcagaccgtg aggacagtat tggctgtgtt tgggaaggga 1260
   tgcctatcca gctccttcag ctgcctcttc ctctacacaa gtgaattata ccccacagtc 1320
   atcaggcaaa caggtatggg cgtaagtaac ctgtggaccc gcgtgggaag catggtgtcc 1380
   ccgctggtga aaatcacggg tgaggtacag cccttcatcc ccaatatcat ctacgggatc 1440
<210> 5
   <211> 555
   <212> PRT
   <213> Homo sapiens
<400> 5
   Met Pro Thr Thr Val Asp Asp Val Leu Glu His Gly Gly Glu Phe His
   1               5                   10                  15
   Phe Phe Gln Lys Gln Met Phe Phe Leu Leu Ala Leu Leu Ser Ala Thr
               20                  25                  30
   Phe Ala Pro Ile Tyr Val Gly Ile Val Phe Leu Gly Phe Thr Pro Asp
           35                  40                  45
   His Arg Cys Arg Ser Pro Gly Val Ala Glu Leu Ser Leu Arg Cys Gly
       50                  55                  60
   Trp Ser Pro Ala Glu Glu Leu Asn Tyr Thr Val Pro Gly Pro Gly Pro
   65                  70                  75                  80
   Ala Gly Glu Ala Ser Pro Arg Gln Cys Arg Arg Tyr Glu Val Asp Trp
                   85                  90                  95
   Asn Gln Ser Thr Phe Asp Cys Val Asp Pro Leu Ala Ser Leu Asp Thr
               100                 105                 110
   Asn Arg Ser Arg Leu Pro Leu Gly Pro Cys Arg Asp Gly Trp Val Tyr
           115                 120                 125
   Glu Thr Pro Gly Ser Ser Ile Val Thr Glu Phe Asn Leu Val Cys Ala
       130                 135                 140
   Asn Ser Trp Met Leu Asp Leu Phe Gln Ser Ser Val Asn Val Gly Phe
   145                 150                 155                 160
   Phe Ile Gly Ser Met Ser Ile Gly Tyr Ile Ala Asp Arg Phe Gly Arg
                   165                 170                 175
   Lys Leu Cys Leu Leu Thr Thr Val Leu Ile Asn Ala Ala Ala Gly Val
               180                 185                 190
   Leu Met Ala Ile Ser Pro Thr Tyr Thr Trp Met Leu Ile Phe Arg Leu
           195                 200                 205
   Ile Gln Gly Leu Val Ser Lys Ala Gly Trp Leu Ile Gly Tyr Ile Leu
       210                 215                 220
   Ile Thr Glu Phe Val Gly Arg Arg Tyr Arg Arg Thr Val Gly Ile Phe
   225                 230                 235                 240
   Tyr Gln Val Ala Tyr Thr Val Gly Leu Leu Val Leu Ala Gly Val Ala
                   245                 250                 255
   Tyr Ala Leu Pro His Trp Arg Trp Leu Gln Phe Thr Val Ser Leu Pro
               260                 265                 270
   Asn Phe Phe Phe Leu Leu Tyr Tyr Trp Cys Ile Pro Glu Ser Pro Arg
           275                 280                 285
   Trp Leu Ile Ser Gln Asn Lys Asn Ala Glu Ala Met Arg Ile Ile Lys
       290                 295                 300
   His Ile Ala Lys Lys Asn Gly Lys Ser Leu Pro Ala Ser Leu Gln Arg
   305                 310                 315                 320
   Leu Arg Leu Glu Glu Glu Thr Gly Lys Lys Leu Asn Pro Ser Phe Leu
                   325                 330                 335
   Asp Leu Val Arg Thr Pro Gln Ile Arg Lys His Thr Met Ile Leu Met
               340                 345                 350
   Tyr Asn Trp Phe Thr Ser Ser Val Leu Tyr Gln Gly Leu Ile Met His
           355                 360                 365
   Met Gly Leu Ala Gly Asp Asn Ile Tyr Leu Asp Phe Phe Tyr Ser Ala
       370                 375                 380
   Leu Val Glu Phe Pro Ala Ala Phe Met Ile Ile Leu Thr Ile Asp Arg
   385                 390                 395                 400
   Ile Gly Arg Arg Tyr Pro Trp Ala Ala Ser Asn Met Val Ala Gly Ala
                   405                 410                 415
   Ala Cys Leu Ala Ser Val Phe Ile Pro Gly Asp Leu Gln Trp Leu Lys
               420                 425                 430
   Ile Ile Ile Ser Cys Leu Gly Arg Met Gly Ile Thr Met Ala Tyr Glu
           435                 440                 445
   Ile Val Cys Leu Val Asn Ala Glu Leu Tyr Pro Thr Phe Ile Arg Asn
       450                 455                 460
   Leu Gly Val His Ile Cys Ser Ser Met Cys Asp Ile Gly Gly Ile Ile
   465                 470                 475                 480
   Thr Pro Phe Leu Val Tyr Arg Leu Thr Asn Ile Trp Leu Glu Leu Pro
                   485                 490                 495
   Leu Met Val Phe Gly Val Leu Gly Leu Val Ala Gly Gly Leu Val Leu
               500                 505                 510
   Leu Leu Pro Glu Thr Lys Gly Lys Ala Leu Pro Glu Thr Ile Glu Glu
           515                 520                 525
   Ala Glu Asn Met Gln Arg Pro Arg Lys Asn Lys Glu Lys Met Ile Tyr
       530                 535                 540
   Leu Gln Val Gln Lys Leu Asp Ile Pro Leu Asn
   545 550 555
<210> 6
   <211> 1668
   <212> DNA
   <213> Homo sapiens
<400> 6
<210> 7
   <211> 724
   <212> PRT
   <213> Homo sapiens
<400> 7
   Met Lys Ser Ala Lys Gly Ile Glu Asn Leu Ala Phe Val Pro Ser Ser
   1               5                   10                  15
   Pro Asp Ile Leu Arg Arg Leu Ser Ala Ser Pro Ser Gln Ile Glu Val
               20                  25                  30
   Ser Ala Leu Ser Ser Asp Pro Gln Arg Glu Asn Ser Gln Pro Gln Glu
           35                  40                  45
   Leu Gln Lys Pro Gln Glu Pro Gln Lys Ser Pro Glu Pro Ser Leu Pro
       50                  55                  60
   Ser Ala Pro Pro Asn Val Ser Glu Glu Lys Leu Arg Ser Leu Ser Leu
   65                  70                  75                  80
   Ser Glu Phe Glu Glu Gly Ser Tyr Gly Trp Arg Asn Phe His Pro Gln
                   85                  90                  95
   Cys Leu Gln Arg Cys Asn Thr Pro Gly Gly Phe Leu Leu His Tyr Cys
               100                 105                 110
   Leu Leu Ala Val Thr Gln Gly Ile Val Val Asn Gly Leu Val Asn Ile
           115                 120                 125
   Ser Ile Ser Thr Val Glu Lys Arg Tyr Glu Met Lys Ser Ser Leu Thr
       130                 135                 140
   Gly Leu Ile Ser Ser Ser Tyr Asp Ile Ser Phe Cys Leu Leu Ser Leu
   145                 150                 155                 160
   Phe Val Ser Phe Phe Gly Glu Arg Gly His Lys Pro Arg Trp Leu Ala
                   165                 170                 175
   Phe Ala Ala Phe Met Ile Gly Leu Gly Ala Leu Val Phe Ser Leu Pro
               180                 185                 190
   Gln Phe Phe Ser Gly Glu Tyr Lys Leu Gly Ser Leu Phe Glu Asp Thr
           195                 200                 205
   Cys Val Thr Thr Arg Asn Ser Thr Ser Cys Thr Ser Ser Thr Ser Ser
       210                 215                 220
   Leu Ser Asn Tyr Leu Tyr Val Phe Ile Leu Gly Gln Leu Leu Leu Gly
   225                 230                 235                 240
   Ala Gly Gly Thr Pro Leu Tyr Thr Leu Gly Thr Ala Phe Leu Asp Asp
                   245                 250                 255
   Ser Val Pro Thr His Lys Ser Ser Leu Tyr Ile Gly Thr Gly Tyr Ala
               260                 265                 270
   Met Ser Ile Leu Gly Pro Ala Ile Gly Tyr Val Leu Gly Gly Gln Leu
           275                 280                 285
   Leu Thr Ile Tyr Ile Asp Val Ala Met Gly Glu Ser Thr Asp Val Thr
       290                 295                 300
   Glu Asp Asp Pro Arg Trp Leu Gly Ala Trp Trp Ile Gly Phe Leu Leu
   305                 310                 315                 320
   Ser Trp Ile Phe Ala Trp Ser Leu Ile Ile Pro Phe Ser Cys Phe Pro
                   325                 330                 335
   Lys His Leu Pro Gly Thr Ala Glu Ile Gln Ala Gly Lys Thr Ser Gln
               340                 345                 350
   Ala His Gln Ser Asn Ser Asn Ala Asp Val Lys Phe Gly Lys Ser Ile
           355                 360                 365
   Lys Asp Phe Pro Ala Ala Leu Lys Asn Leu Met Lys Asn Ala Val Phe
       370                 375                 380
   Met Cys Leu Val Leu Ser Thr Ser Ser Glu Ala Leu Ile Thr Thr Gly
   385                 390                 395                 400
   Phe Ala Thr Phe Leu Pro Lys Phe Ile Glu Asn Gln Phe Gly Leu Thr
                   405                 410                 415
   Ser Ser Phe Ala Ala Thr Leu Gly Gly Ala Val Leu Ile Pro Gly Ala
               420                 425                 430
   Ala Leu Gly Gln Ile Leu Gly Gly Phe Leu Val Ser Lys Phe Arg Met
           435                 440                 445
   Thr Cys Lys Asn Thr Met Lys Phe Ala Leu Phe Thr Ser Gly Val Ala
       450                 455                 460
   Leu Thr Leu Ser Phe Val Phe Met Tyr Ala Lys Cys Glu Asn Glu Pro
   465                 470                 475                 480
   Phe Ala Gly Val Ser Glu Ser Tyr Asn Gly Thr Gly Glu Leu Gly Asn
                   485                 490                 495
   Leu Ile Ala Pro Cys Asn Ala Asn Cys Asn Cys Ser Arg Ser Tyr Tyr
               500                 505                 510
   Tyr Pro Val Cys Gly Asp Gly Val Gln Tyr Phe Ser Pro Cys Phe Ala
           515                 520                 525
   Gly Cys Ser Asn Pro Val Ala His Arg Lys Pro Lys Val Tyr Tyr Asn
       530                 535                 540
   Cys Ser Cys Ile Glu Arg Lys Thr Glu Ile Thr Ser Thr Ala Glu Thr
   545                 550                 555                 560
   Phe Gly Phe Glu Ala Lys Ala Gly Lys Cys Glu Thr His Cys Ala Lys
                   565                 570                 575
   Leu Pro Ile Phe Leu Cys Ile Phe Phe Ile Val Ile Ile Phe Thr Phe
               580                 585                 590
   Met Ala Gly Thr Pro Ile Thr Val Ser Ile Leu Arg Cys Val Asn His
           595                 600                 605
   Arg Gln Arg Ser Leu Ala Leu Gly Ile Gln Phe Met Val Leu Arg Leu
       610                 615                 620
   Leu Gly Thr Ile Pro Gly Pro Ile Ile Phe Gly Phe Thr Ile Asp Ser
   625                 630                 635                 640
   Thr Cys Ile Leu Trp Asp Ile Asn Asp Cys Gly Ile Lys Gly Ala Cys
                   645                 650                 655
   Trp Ile Tyr Asp Asn Ile Lys Met Ala His Met Leu Val Ala Ile Ser
               660                 665                 670
   Val Thr Cys Lys Val Ile Thr Met Phe Phe Asn Gly Phe Ala Ile Phe
           675                 680                 685
   Leu Tyr Lys Pro Pro Pro Ser Ala Thr Asp Val Ser Phe His Lys Glu
       690                 695                 700
   Asn Ala Val Val Thr Asn Val Leu Ala Glu Gln Asp Leu Asn Lys Ile
   705                 710                 715                 720
   Val Lys Glu Gly
<210> 8
   <211> 2175
   <212> DNA
   <213> Homo sapiens
<400> 8
   atgaagagcg ccaaaggtat tgagaacttg gcttttgtcc cctccagccc agacatcctg      60
   cgccgcttgt ctgcgtcgcc ctcccaaatc gaagtctctg ccttgtcctc tgacccccaa     120
   agagagaatt ctcagccaca ggagcttcag aagccccagg agccccagaa gtcaccagag     180
   ccatctctgc cttcagcccc tcccaatgtc tccgaagaga agctccggtc actgtcgctg     240
   tccgagtttg aggaggggtc ttacggctgg aggaacttcc atcctcaatg tctccagcgc     300
   tgcaacacac ctggaggctt tctgcttcac tactgcctct tggccgtcac gcaaggtatt     360
   gtagttaatg gcctagtaaa tattagcatt tccactgttg agaagcgtta tgaaatgaag     420
   agttccctga ctggcctgat ttcatcaagc tacgatattt cattctgttt gttgtcttta     480
   tttgtatcat tctttggtga aagaggacat aagccgagat ggcttgcatt tgcagccttt     540
   atgattggac tgggagcact tgtattctca ttgccacaat ttttcagtgg agaatataaa     600
   ttggggtctc tttttgaaga cacttgtgta acaacaagga atagcaccag ttgtacatct     660
   tcaacttctt cactttctaa ctacttgtat gtcttcatct tgggacaact attgctgggg     720
   gcaggaggaa ctcctcttta tactctggga acagcctttc ttgatgattc tgtgcccaca     780
   cacaagtctt ctctctatat aggaaccggt tatgctatgt caatcttagg ccctgctatt     840
   ggctatgtat tgggaggaca actgctaacc atatacattg atgttgctat gggagaaagc     900
   actgatgtca ctgaggatga tccgcgatgg ttgggagctt ggtggattgg gtttcttcta     960
   tcatggatct ttgcttggtc tttaataata cctttttctt gctttccaaa acatttacca    1020
   ggtacagcag aaattcaagc tggaaaaact tcccaggctc atcagagtaa tagtaatgca    1080
   gatgtgaaat ttggaaaaag tattaaagat tttccagctg ctctaaagaa tttgatgaag    1140
   aatgctgtct ttatgtgttt agttctatca acttcttcag aagccttaat tactactgga    1200
   tttgctacat ttttacctaa atttatagaa aatcaattcg gattgacatc cagcttcgca    1260
   gctactcttg gaggggctgt tttaattcct ggagctgctc tcggtcaaat tttaggtggc    1320
   ttccttgttt caaaattcag aatgacatgt aaaaacacaa tgaagtttgc actgttcaca    1380
   tctggagttg cacttacgct gagttttgta tttatgtatg ccaaatgtga aaatgagcca    1440
   tttgctggtg tatctgaatc atataatggg actggagaat tgggaaactt gatagcccct    1500
   tgtaatgcca attgtaactg ttcgcgatca tattattatc ctgtctgtgg agatggagtc    1560
   caatattttt ctccctgctt tgcaggctgt tcaaacccag ttgcacacag gaagccaaag 1620
   gtatattaca actgttcctg tattgaaagg aaaacagaaa taacatccac tgcagaaact 1680
   tttggttttg aagctaaagc tggaaaatgt gaaactcatt gtgcgaaact gcccatattc 1740
   ctttgcattt tctttattgt aattattttt acctttatgg ccggtactcc tataactgtg 1800
   tctatcctaa ggtgtgttaa tcacagacaa cggtccctag ccttgggaat acaatttatg 1860
   gtccttcgat tattagggac aattcctgga ccaattatat ttggtttcac aatagacagc 1920
   acatgtattc tttgggatat aaatgattgt ggaattaaag gagcttgctg gatttatgat 1980
   aacatcaaga tggcccatat gctagtagcc ataagtgtta cttgtaaagt tatcaccatg 2040
   ttcttcaatg gatttgcaat ctttttgtat aaaccacctc catcagccac agatgtgtca 2100
   tttcataaag agaatgcagt tgtgactaat gttttagcag aacaggatct caacaaaata 2160
   gtaaaagaag ggtga 2175
<210> 9
   <211> 1280
   <212> PRT
   <213> Homo sapiens
<400> 9
   Met Asp Leu Glu Gly Asp Arg Asn Gly Gly Ala Lys Lys Lys Asn Phe
   1               5                   10                  15
   Phe Lys Leu Asn Asn Lys Ser Glu Lys Asp Lys Lys Glu Lys Lys Pro
               20                  25                  30
   Thr Val Ser Val Phe Ser Met Phe Arg Tyr Ser Asn Trp Leu Asp Lys
           35                  40                  45
   Leu Tyr Met Val Val Gly Thr Leu Ala Ala Ile Ile His Gly Ala Gly
       50                  55                  60
   Leu Pro Leu Met Met Leu Val Phe Gly Glu Met Thr Asp Ile Phe Ala
   65                  70                  75                  80
   Asn Ala Gly Asn Leu Glu Asp Leu Met Ser Asn Ile Thr Asn Arg Ser
                   85                  90                  95
   Asp Ile Asn Asp Thr Gly Phe Phe Met Asn Leu Glu Glu Asp Met Thr
               100                 105                 110
   Arg Tyr Ala Tyr Tyr Tyr Ser Gly Ile Gly Ala Gly Val Leu Val Ala
           115                 120                 125
   Ala Tyr Ile Gln Val Ser Phe Trp Cys Leu Ala Ala Gly Arg Gln Ile
   130 135 140
   His Lys Ile Arg Lys Gln Phe Phe His Ala Ile Met Arg Gln Glu Ile
   145 150 155 160
   Gly Trp Phe Asp Val His Asp Val Gly Glu Leu Asn Thr Arg Leu Thr
   165 170 175
   Asp Asp Val Ser Lys Ile Asn Glu Gly Ile Gly Asp Lys Ile Gly Met
   180 185 190
   Phe Phe Gln Ser Met Ala Thr Phe Phe Thr Gly Phe Ile Val Gly Phe
   195 200 205
   Thr Arg Gly Trp Lys Leu Thr Leu Val Ile Leu Ala Ile Ser Pro Val
   210 215 220
   Leu Gly Leu Ser Ala Ala Val Trp Ala Lys Ile Leu Ser Ser Phe Thr
   225 230 235 240
   Asp Lys Glu Leu Leu Ala Tyr Ala Lys Ala Gly Ala Val Ala Glu Glu
   245 250 255
   Val Leu Ala Ala Ile Arg Thr Val Ile Ala Phe Gly Gly Gln Lys Lys
   260 265 270
   Glu Leu Glu Arg Tyr Asn Lys Asn Leu Glu Glu Ala Lys Arg Ile Gly
   275 280 285
   Ile Lys Lys Ala Ile Thr Ala Asn Ile Ser Ile Gly Ala Ala Phe Leu
   290 295 300
   Leu Ile Tyr Ala Ser Tyr Ala Leu Ala Phe Trp Tyr Gly Thr Thr Leu
   305 310 315 320
   Val Leu Ser Gly Glu Tyr Ser Ile Gly Gln Val Leu Thr Val Phe Phe
   325 330 335
   Ser Val Leu Ile Gly Ala Phe Ser Val Gly Gln Ala Ser Pro Ser Ile
   340 345 350
   Glu Ala Phe Ala Asn Ala Arg Gly Ala Ala Tyr Glu Ile Phe Lys Ile
   355 360 365
   Ile Asp Asn Lys Pro Ser Ile Asp Ser Tyr Ser Lys Ser Gly His Lys
   370 375 380
   Pro Asp Asn Ile Lys Gly Asn Leu Glu Phe Arg Asn Val His Phe Ser
   385 390 395 400
   Tyr Pro Ser Arg Lys Glu Val Lys Ile Leu Lys Gly Leu Asn Leu Lys
   405 410 415
   Val Gln Ser Gly Gln Thr Val Ala Leu Val Gly Asn Ser Gly Cys Gly
   420 425 430
   Lys Ser Thr Thr Val Gln Leu Met Gln Arg Leu Tyr Asp Pro Thr Glu
   435 440 445
   Gly Met Val Ser Val Asp Gly Gln Asp Ile Arg Thr Ile Asn Val Arg
   450 455 460
   Phe Leu Arg Glu Ile Ile Gly Val Val Ser Gln Glu Pro Val Leu Phe
   465 470 475 480
   Ala Thr Thr Ile Ala Glu Asn Ile Arg Tyr Gly Arg Glu Asn Val Thr
   485 490 495
   Met Asp Glu Ile Glu Lys Ala Val Lys Glu Ala Asn Ala Tyr Asp Phe
   500 505 510
   Ile Met Lys Leu Pro His Lys Phe Asp Thr Leu Val Gly Glu Arg Gly
   515 520 525
   Ala Gln Leu Ser Gly Gly Gln Lys Gln Arg Ile Ala Ile Ala Arg Ala
   530 535 540
   Leu Val Arg Asn Pro Lys Ile Leu Leu Leu Asp Glu Ala Thr Ser Ala
   545 550 555 560
   Leu Asp Thr Glu Ser Glu Ala Val Val Gln Val Ala Leu Asp Lys Ala
   565 570 575
   Arg Lys Gly Arg Thr Thr Ile Val Ile Ala His Arg Leu Ser Thr Val
   580 585 590
   Arg Asn Ala Asp Val Ile Ala Gly Phe Asp Asp Gly Val Ile Val Glu
   595 600 605
   Lys Gly Asn His Asp Glu Leu Met Lys Glu Lys Gly Ile Tyr Phe Lys
   610 615 620
   Leu Val Thr Met Gln Thr Ala Gly Asn Glu Val Glu Leu Glu Asn Ala
   625 630 635 640
   Ala Asp Glu Ser Lys Ser Glu Ile Asp Ala Leu Glu Met Ser Ser Asn
   645 650 655
   Asp Ser Arg Ser Ser Leu Ile Arg Lys Arg Ser Thr Arg Arg Ser Val
   660 665 670
   Arg Gly Ser Gln Ala Gln Asp Arg Lys Leu Ser Thr Lys Glu Ala Leu
   675 680 685
   Asp Glu Ser Ile Pro Pro Val Ser Phe Trp Arg Ile Met Lys Leu Asn
   690 695 700
   Leu Thr Glu Trp Pro Tyr Phe Val Val Gly Val Phe Cys Ala Ile Ile
   705 710 715 720
   Asn Gly Gly Leu Gln Pro Ala Phe Ala Ile Ile Phe Ser Lys Ile Ile
   725 730 735
   Gly Val Phe Thr Arg Ile Asp Asp Pro Glu Thr Lys Arg Gln Asn Ser
   740 745 750
   Asn Leu Phe Ser Leu Leu Phe Leu Ala Leu Gly Ile Ile Ser Phe Ile
   755 760 765
   Thr Phe Phe Leu Gln Gly Phe Thr Phe Gly Lys Ala Gly Glu Ile Leu
   770 775 780
   Thr Lys Arg Leu Arg Tyr Met Val Phe Arg Ser Met Leu Arg Gln Asp
   785 790 795 800
   Val Ser Trp Phe Asp Asp Pro Lys Asn Thr Thr Gly Ala Leu Thr Thr
   805 810 815
   Arg Leu Ala Asn Asp Ala Ala Gln Val Lys Gly Ala Ile Gly Ser Arg
   820 825 830
   Leu Ala Val Ile Thr Gln Asn Ile Ala Asn Leu Gly Thr Gly Ile Ile
   835 840 845
   Ile Ser Phe Ile Tyr Gly Trp Gln Leu Thr Leu Leu Leu Leu Ala Ile
   850 855 860
   Val Pro Ile Ile Ala Ile Ala Gly Val Val Glu Met Lys Met Leu Ser
   865 870 875 880
   Gly Gln Ala Leu Lys Asp Lys Lys Glu Leu Glu Gly Ser Gly Lys Ile
   885 890 895
   Ala Thr Glu Ala Ile Glu Asn Phe Arg Thr Val Val Ser Leu Thr Gln
               900                 905                 910
   Glu Gln Lys Phe Glu His Met Tyr Ala Gln Ser Leu Gln Val Pro Tyr
           915                 920                 925
   Arg Asn Ser Leu Arg Lys Ala His Ile Phe Gly Ile Thr Phe Ser Phe
       930                 935                 940
   Thr Gln Ala Met Met Tyr Phe Ser Tyr Ala Gly Cys Phe Arg Phe Gly
   945                 950                 955                 960
   Ala Tyr Leu Val Ala His Lys Leu Met Ser Phe Glu Asp Val Leu Leu
                   965                 970                 975
   Val Phe Ser Ala Val Val Phe Gly Ala Met Ala Val Gly Gln Val Ser
               980                 985                 990
   Ser Phe Ala Pro Asp Tyr Ala Lys  Ala Lys Ile Ser Ala  Ala His Ile
           995                 1000                 1005
   Ile Met  Ile Ile Glu Lys Thr  Pro Leu Ile Asp Ser  Tyr Ser Thr
       1010                 1015                 1020
   Glu Gly  Leu Met Pro Asn Thr  Leu Glu Gly Asn Val  Thr Phe Gly
       1025                 1030                 1035
   Glu Val  Val Phe Asn Tyr Pro  Thr Arg Pro Asp Ile  Pro Val Leu
       1040                 1045                 1050
   Gln Gly  Leu Ser Leu Glu Val  Lys Lys Gly Gln Thr  Leu Ala Leu
       1055                 1060                 1065
   Val Gly  Ser Ser Gly Cys Gly  Lys Ser Thr Val Val  Gln Leu Leu
       1070                 1075                 1080
   Glu Arg  Phe Tyr Asp Pro Leu  Ala Gly Lys Val Leu  Leu Asp Gly
       1085                 1090                 1095
   Lys Glu  Ile Lys Arg Leu Asn  Val Gln Trp Leu Arg  Ala His Leu
       1100                 1105                 1110
   Gly Ile  Val Ser Gln Glu Pro  Ile Leu Phe Asp Cys  Ser Ile Ala
       1115                 1120                 1125
   Glu Asn  Ile Ala Tyr Gly Asp  Asn Ser Arg Val Val  Ser Gln Glu
   1130 1135 1140
   Glu Ile Val Arg Ala Ala Lys Glu Ala Asn Ile His Ala Phe Ile
   1145 1150 1155
   Glu Ser Leu Pro Asn Lys Tyr Ser Thr Lys Val Gly Asp Lys Gly
   1160 1165 1170
   Thr Gln Leu Ser Gly Gly Gln Lys Gln Arg Ile Ala Ile Ala Arg
   1175 1180 1185
   Ala Leu Val Arg Gln Pro His Ile Leu Leu Leu Asp Glu Ala Thr
   1190 1195 1200
   Ser Ala Leu Asp Thr Glu Ser Glu Lys Val Val Gln Glu Ala Leu
   1205 1210 1215
   Asp Lys Ala Arg Glu Gly Arg Thr Cys Ile Val Ile Ala His Arg
   1220 1225 1230
   Leu Ser Thr Ile Gln Asn Ala Asp Leu Ile Val Val Phe Gln Asn
   1235 1240 1245
   Gly Arg Val Lys Glu His Gly Thr His Gln Gln Leu Leu Ala Gln
   1250 1255 1260
   Lys Gly Ile Tyr Phe Ser Met Val Ser Val Gln Ala Gly Thr Lys
   1265 1270 1275
   Arg Gln
   1280
<210> 10
   <211> 3843
   <212> DNA
   <213> Homo sapiens
<400> 10
   atggatcttg aaggggaccg caatggagga gcaaagaaga agaacttttt taaactgaac     60
   aataaaagtg aaaaagataa gaaggaaaag aaaccaactg tcagtgtatt ttcaatgttt    120
   cgctattcaa attggcttga caagttgtat atggtggtgg gaactttggc tgccatcatc    180
   catggggctg gacttcctct catgatgctg gtgtttggag aaatgacaga tatctttgca    240
   aatgcaggaa atttagaaga tctgatgtca aacatcacta atagaagtga tatcaatgat    300
   acagggttct tcatgaatct ggaggaagac atgaccaggt atgcctatta ttacagtgga    360
   attggtgctg gggtgctggt tgctgcttac attcaggttt cattttggtg cctggcagct    420
   ggaagacaaa tacacaaaat tagaaaacag ttttttcatg ctataatgcg acaggagata    480
   ggctggtttg atgtgcacga tgttggggag cttaacaccc gacttacaga tgatgtctcc    540
   aagattaatg aaggaattgg tgacaaaatt ggaatgttct ttcagtcaat ggcaacattt    600
   ttcactgggt ttatagtagg atttacacgt ggttggaagc taacccttgt gattttggcc    660
   atcagtcctg ttcttggact gtcagctgct gtctgggcaa agatactatc ttcatttact    720
   gataaagaac tcttagcgta tgcaaaagct ggagcagtag ctgaagaggt cttggcagca    780
   attagaactg tgattgcatt tggaggacaa aagaaagaac ttgaaaggta caacaaaaat    840
   ttagaagaag ctaaaagaat tgggataaag aaagctatta cagccaatat ttctataggt    900
   gctgctttcc tgctgatcta tgcatcttat gctctggcct tctggtatgg gaccaccttg    960
   gtcctctcag gggaatattc tattggacaa gtactcactg tattcttttc tgtattaatt   1020
   ggggctttta gtgttggaca ggcatctcca agcattgaag catttgcaaa tgcaagagga   1080
   gcagcttatg aaatcttcaa gataattgat aataagccaa gtattgacag ctattcgaag   1140
   agtgggcaca aaccagataa tattaaggga aatttggaat tcagaaatgt tcacttcagt   1200
   tacccatctc gaaaagaagt taagatcttg aagggtctga acctgaaggt gcagagtggg   1260
   cagacggtgg ccctggttgg aaacagtggc tgtgggaaga gcacaacagt ccagctgatg   1320
   cagaggctct atgaccccac agaggggatg gtcagtgttg atggacagga tattaggacc   1380
   ataaatgtaa ggtttctacg ggaaatcatt ggtgtggtga gtcaggaacc tgtattgttt   1440
   gccaccacga tagctgaaaa cattcgctat ggccgtgaaa atgtcaccat ggatgagatt   1500
   gagaaagctg tcaaggaagc caatgcctat gactttatca tgaaactgcc tcataaattt   1560
   gacaccctgg ttggagagag aggggcccag ttgagtggtg ggcagaagca gaggatcgcc   1620
   attgcacgtg ccctggttcg caaccccaag atcctcctgc tggatgaggc cacgtcagcc   1680
   ttggacacag aaagcgaagc agtggttcag gtggctctgg ataaggccag aaaaggtcgg   1740
   accaccattg tgatagctca tcgtttgtct acagttcgta atgctgacgt catcgctggt   1800
   ttcgatgatg gagtcattgt ggagaaagga aatcatgatg aactcatgaa agagaaaggc   1860
   atttacttca aacttgtcac aatgcagaca gcaggaaatg aagttgaatt agaaaatgca   1920
   gctgatgaat ccaaaagtga aattgatgcc ttggaaatgt cttcaaatga ttcaagatcc   1980
   agtctaataa gaaaaagatc aactcgtagg agtgtccgtg gatcacaagc ccaagacaga   2040
   aagcttagta ccaaagaggc tctggatgaa agtatacctc cagtttcctt ttggaggatt   2100
   atgaagctaa atttaactga atggccttat tttgttgttg gtgtattttg tgccattata   2160
   aatggaggcc tgcaaccagc atttgcaata atattttcaa agattatagg ggtttttaca   2220
   agaattgatg atcctgaaac aaaacgacag aatagtaact tgttttcact attgtttcta   2280
   gcccttggaa ttatttcttt tattacattt ttccttcagg gtttcacatt tggcaaagct   2340
   ggagagatcc tcaccaagcg gctccgatac atggttttcc gatccatgct cagacaggat 2400
   gtgagttggt ttgatgaccc taaaaacacc actggagcat tgactaccag gctcgccaat 2460
   gatgctgctc aagttaaagg ggctataggt tccaggcttg ctgtaattac ccagaatata 2520
   gcaaatcttg ggacaggaat aattatatcc ttcatctatg gttggcaact aacactgtta 2580
   ctcttagcaa ttgtacccat cattgcaata gcaggagttg ttgaaatgaa aatgttgtct 2640
   ggacaagcac tgaaagataa gaaagaacta gaaggttctg ggaagatcgc tactgaagca 2700
   atagaaaact tccgaaccgt tgtttctttg actcaggagc agaagtttga acatatgtat 2760
   gctcagagtt tgcaggtacc atacagaaac tctttgagga aagcacacat ctttggaatt 2820
   acattttcct tcacccaggc aatgatgtat ttttcctatg ctggatgttt ccggtttgga 2880
   gcctacttgg tggcacataa actcatgagc tttgaggatg ttctgttagt attttcagct 2940
   gttgtctttg gtgccatggc cgtggggcaa gtcagttcat ttgctcctga ctatgccaaa 3000
   gccaaaatat cagcagccca catcatcatg atcattgaaa aaaccccttt gattgacagc 3060
   tacagcacgg aaggcctaat gccgaacaca ttggaaggaa atgtcacatt tggtgaagtt 3120
   gtattcaact atcccacccg accggacatc ccagtgcttc agggactgag cctggaggtg 3180
   aagaagggcc agacgctggc tctggtgggc agcagtggct gtgggaagag cacagtggtc 3240
   cagctcctgg agcggttcta cgaccccttg gcagggaaag tgctgcttga tggcaaagaa 3300
   ataaagcgac tgaatgttca gtggctccga gcacacctgg gcatcgtgtc ccaggagccc 3360
   atcctgtttg actgcagcat tgctgagaac attgcctatg gagacaacag ccgggtggtg 3420
   tcacaggaag agattgtgag ggcagcaaag gaggccaaca tacatgcctt catcgagtca 3480
   ctgcctaata aatatagcac taaagtagga gacaaaggaa ctcagctctc tggtggccag 3540
   aaacaacgca ttgccatagc tcgtgccctt gttagacagc ctcatatttt gcttttggat 3600
   gaagccacgt cagctctgga tacagaaagt gaaaaggttg tccaagaagc cctggacaaa 3660
   gccagagaag gccgcacctg cattgtgatt gctcaccgcc tgtccaccat ccagaatgca 3720
   gacttaatag tggtgtttca gaatggcaga gtcaaggagc atggcacgca tcagcagctg 3780
   ctggcacaga aaggcatcta tttttcaatg gtcagtgtcc aggctggaac aaagcgccag 3840
   tga 3843
<210> 11
   <211> 655
   <212> PRT
   <213> Homo sapiens
<400> 11
   Met Ser Ser Ser Asn Val Glu Val Phe Ile Pro Val Ser Gln Gly Asn
   1 5 10 15
   Thr Asn Gly Phe Pro Ala Thr Ala Ser Asn Asp Leu Lys Ala Phe Thr
               20                  25                  30
   Glu Gly Ala Val Leu Ser Phe His Asn Ile Cys Tyr Arg Val Lys Leu
           35                  40                  45
   Lys Ser Gly Phe Leu Pro Cys Arg Lys Pro Val Glu Lys Glu Ile Leu
       50                  55                  60
   Ser Asn Ile Asn Gly Ile Met Lys Pro Gly Leu Asn Ala Ile Leu Gly
   65                  70                  75                  80
   Pro Thr Gly Gly Gly Lys Ser Ser Leu Leu Asp Val Leu Ala Ala Arg
                   85                  90                  95
   Lys Asp Pro Ser Gly Leu Ser Gly Asp Val Leu Ile Asn Gly Ala Pro
               100                 105                 110
   Arg Pro Ala Asn Phe Lys Cys Asn Ser Gly Tyr Val Val Gln Asp Asp
           115                 120                 125
   Val Val Met Gly Thr Leu Thr Val Arg Glu Asn Leu Gln Phe Ser Ala
       130                 135                 140
   Ala Leu Arg Leu Ala Thr Thr Met Thr Asn His Glu Lys Asn Glu Arg
   145                 150                 155                 160
   Ile Asn Arg Val Ile Gln Glu Leu Gly Leu Asp Lys Val Ala Asp Ser
                   165                 170                 175
   Lys Val Gly Thr Gln Phe Ile Arg Gly Val Ser Gly Gly Glu Arg Lys
               180                 185                 190
   Arg Thr Ser Ile Gly Met Glu Leu Ile Thr Asp Pro Ser Ile Leu Phe
           195                 200                 205
   Leu Asp Glu Pro Thr Thr Gly Leu Asp Ser Ser Thr Ala Asn Ala Val
       210                 215                 220
   Leu Leu Leu Leu Lys Arg Met Ser Lys Gln Gly Arg Thr Ile Ile Phe
   225                 230                 235                 240
   Ser Ile His Gln Pro Arg Tyr Ser Ile Phe Lys Leu Phe Asp Ser Leu
                   245                 250                 255
   Thr Leu Leu Ala Ser Gly Arg Leu Met Phe His Gly Pro Ala Gln Glu
   260 265 270
   Ala Leu Gly Tyr Phe Glu Ser Ala Gly Tyr His Cys Glu Ala Tyr Asn
   275 280 285
   Asn Pro Ala Asp Phe Phe Leu Asp Ile Ile Asn Gly Asp Ser Thr Ala
   290 295 300
   Val Ala Leu Asn Arg Glu Glu Asp Phe Lys Ala Thr Glu Ile Ile Glu
   305 310 315 320
   Pro Ser Lys Gln Asp Lys Pro Leu Ile Glu Lys Leu Ala Glu Ile Tyr
   325 330 335
   Val Asn Ser Ser Phe Tyr Lys Glu Thr Lys Ala Glu Leu His Gln Leu
   340 345 350
   Ser Gly Gly Glu Lys Lys Lys Lys Ile Thr Val Phe Lys Glu Ile Ser
   355 360 365
   Tyr Thr Thr Ser Phe Cys His Gln Leu Arg Trp Val Ser Lys Arg Ser
   370 375 380
   Phe Lys Asn Leu Leu Gly Asn Pro Gln Ala Ser Ile Ala Gln Ile Ile
   385 390 395 400
   Val Thr Val Val Leu Gly Leu Val Ile Gly Ala Ile Tyr Phe Gly Leu
   405 410 415
   Lys Asn Asp Ser Thr Gly Ile Gln Asn Arg Ala Gly Val Leu Phe Phe
   420 425 430
   Leu Thr Thr Asn Gln Cys Phe Ser Ser Val Ser Ala Val Glu Leu Phe
   435 440 445
   Val Val Glu Lys Lys Leu Phe Ile His Glu Tyr Ile Ser Gly Tyr Tyr
   450 455 460
   Arg Val Ser Ser Tyr Phe Leu Gly Lys Leu Leu Ser Asp Leu Leu Pro
   465 470 475 480
   Met Arg Met Leu Pro Ser Ile Ile Phe Thr Cys Ile Val Tyr Phe Met
   485 490 495
   Leu Gly Leu Lys Pro Lys Ala Asp Ala Phe Phe Val Met Met Phe Thr
   500 505 510
   Leu Met Met Val Ala Tyr Ser Ala Ser Ser Met Ala Leu Ala Ile Ala
           515                 520                 525
   Ala Gly Gln Ser Val Val Ser Val Ala Thr Leu Leu Met Thr Ile Cys
       530                 535                 540
   Phe Val Phe Met Met Ile Phe Ser Gly Leu Leu Val Asn Leu Thr Thr
   545                 550                 555                 560
   Ile Ala Ser Trp Leu Ser Trp Leu Gln Tyr Phe Ser Ile Pro Arg Tyr
                   565                 570                 575
   Gly Phe Thr Ala Leu Gln His Asn Glu Phe Leu Gly Gln Asn Phe Cys
               580                 585                 590
   Pro Gly Leu Asn Ala Thr Gly Asn Asn Pro Cys Asn Tyr Ala Thr Cys
           595                 600                 605
   Thr Gly Glu Glu Tyr Leu Val Lys Gln Gly Ile Asp Leu Ser Pro Trp
       610                 615                 620
   Gly Leu Trp Lys Asn His Val Ala Leu Ala Cys Met Ile Val Ile Phe
   625                 630                 635                 640
   Leu Thr Ile Ala Tyr Leu Lys Leu Leu Phe Leu Lys Lys Tyr Ser
                   645                 650                 655
<210> 12
   <211> 1967
   <212> DNA
   <213> Homo sapiens
<400> 12
   tgtcttccag taatgtcgaa gtttttatcc cagtgtcaca aggaaacacc aatggcttcc 60
   ccgcgacagc ttccaatgac ctgaaggcat ttactgaagg agctgtgtta agttttcata 120
   acatctgcta tcgagtaaaa ctgaagagtg gctttctacc ttgtcgaaaa ccagttgaga 180
   aagaaatatt atcgaatatc aatgggatca tgaaacctgg tctcaacgcc atcctgggac 240
   ccacaggtgg aggcaaatct tcgttattag atgtcttagc tgcaaggaaa gatccaagtg 300
   gattatctgg agatgttctg ataaatggag caccgcgacc tgccaatttc aaatgtaatt 360
   caggttacgt ggtacaagat gatgttgtga tgggcactct gacggtgaga gaaaacttac 420
   agttctcagc agctcttcgg cttgcaacaa ctatgacgaa tcatgaaaaa aacgaacgga 480
   ttaacagggt cattcaagag ttaggtctgg ataaagtggc agactccaag gttggaactc 540
   agtttatccg tggtgtgtct ggaggagaaa gaaaaaggac tagtatagga atggagctta 600
   tcactgatcc ttccatcttg ttcttggatg agcctacaac tggcttagac tcaagcacag 660
   caaatgctgt ccttttgctc ctgaaaagga tgtctaagca gggacgaaca atcatcttct 720
   ccattcatca gcctcgatat tccatcttca agttgtttga tagcctcacc ttattggcct 780
   caggaagact tatgttccac gggcctgctc aggaggcctt gggatacttt gaatcagctg 840
   gttatcactg tgaggcctat aataaccctg cagacttctt cttggacatc attaatggag 900
   attccactgc tgtggcatta aacagagaag aagactttaa agccacagag atcatagagc 960
   cttccaagca ggataagcca ctcatagaaa aattagcgga gatttatgtc aactcctcct 1020
   tctacaaaga gacaaaagct gaattacatc aactttccgg gggtgagaag aagaagaaga 1080
   tcacagtctt caaggagatc agctacacca cctccttctg tcatcaactc agatgggttt 1140
   ccaagcgttc attcaaaaac ttgctgggta atccccaggc ctctatagct cagatcattg 1200
   tcacagtcgt actgggactg gttataggtg ccatttactt tgggctaaaa aatgattcta 1260
   ctggaatcca gaacagagct ggggttctct tcttcctgac gaccaaccag tgtttcagca 1320
   gtgtttcagc cgtggaactc tttgtggtag agaagaagct cttcatacat gaatacatca 1380
   gcggatacta cagagtgtca tcttatttcc ttggaaaact gttatctgat ttattaccca 1440
   tgaggatgtt accaagtatt atatttacct gtatagtgta cttcatgtta ggattgaagc 1500
   caaaggcaga tgccttcttc gttatgatgt ttacccttat gatggtggct tattcagcca 1560
   gttccatggc actggccata gcagcaggtc agagtgtggt ttctgtagca acacttctca 1620
   tgaccatctg ttttgtgttt atgatgattt tttcaggtct gttggtcaat ctcacaacca 1680
   ttgcatcttg gctgtcatgg cttcagtact tcagcattcc acgatatgga tttacggctt 1740
   tgcagcataa tgaatttttg ggacaaaact tctgcccagg actcaatgca acaggaaaca 1800
   atccttgtaa ctatgcaaca tgtactggcg aagaatattt ggtaaagcag ggcatcgatc 1860
   tctcaccctg gggcttgtgg aagaatcacg tggccttggc ttgtatgatt gttattttcc 1920
   tcacaattgc ctacctgaaa ttgttatttc ttaaaaaata ttcttaa 1967
<210> 13
   <211> 1545
   <212> PRT
   <213> Homo sapiens
<400> 13
<210> 14
   <211> 4638
   <212> DNA
   <213> Homo sapiens
<400> 14
   atgctggaga agttctgcaa ctctactttt tggaattcct cattcctgga cagtccggag 60
   gcagacctgc cactttgttt tgagcaaact gttctggtgt ggattccctt gggctaccta 120
   tggctcctgg ccccctggca gcttctccac gtgtataaat ccaggaccaa gagatcctct 180
   accaccaaac tctatcttgc taagcaggta ttcgttggtt ttcttcttat tctagcagcc 240
   atagagctgg cccttgtact cacagaagac tctggacaag ccacagtccc tgctgttcga 300
   tataccaatc caagcctcta cctaggcaca tggctcctgg ttttgctgat ccaatacagc 360
   agacaatggt gtgtacagaa aaactcctgg ttcctgtccc tattctggat tctctcgata 420
   ctctgtggca ctttccaatt tcagactctg atccggacac tcttacaggg tgacaattct 480
   aatctagcct actcctgcct gttcttcatc tcctacggat tccagatcct gatcctgatc 540
   ttttcagcat tttcagaaaa taatgagtca tcaaataatc catcatccat agcttcattc 600
   ctgagtagca ttacctacag ctggtatgac agcatcattc tgaaaggcta caagcgtcct 660
   ctgacactcg aggatgtctg ggaagttgat gaagagatga aaaccaagac attagtgagc 720
   aagtttgaaa cgcacatgaa gagagagctg cagaaagcca ggcgggcact ccagagacgg 780
   caggagaaga gctcccagca gaactctgga gccaggctgc ctggcttgaa caagaatcag 840
   agtcaaagcc aagatgccct tgtcctggaa gatgttgaaa agaaaaaaaa gaagtctggg 900
   accaaaaaag atgttccaaa atcctggttg atgaaggctc tgttcaaaac tttctacatg 960
   gtgctcctga aatcattcct actgaagcta gtgaatgaca tcttcacgtt tgtgagtcct 1020
   cagctgctga aattgctgat ctcctttgca agtgaccgtg acacatattt gtggattgga 1080
   tatctctgtg caatcctctt attcactgcg gctctcattc agtctttctg ccttcagtgt 1140
   tatttccaac tgtgcttcaa gctgggtgta aaagtacgga cagctatcat ggcttctgta 1200
   tataagaagg cattgaccct atccaacttg gccaggaagg agtacaccgt tggagaaaca 1260
   gtgaacctga tgtctgtgga tgcccagaag ctcatggatg tgaccaactt catgcacatg 1320
   ctgtggtcaa gtgttctaca gattgtctta tctatcttct tcctatggag agagttggga 1380
   ccctcagtct tagcaggtgt tggggtgatg gtgcttgtaa tcccaattaa tgcgatactg 1440
   tccaccaaga gtaagaccat tcaggtcaaa aatatgaaga ataaagacaa acgtttaaag 1500
   atcatgaatg agattcttag tggaatcaag atcctgaaat attttgcctg ggaaccttca 1560
   ttcagagacc aagtacaaaa cctccggaag aaagagctca agaacctgct ggcctttagt 1620
   caactacagt gtgtagtaat attcgtcttc cagttaactc cagtcctggt atctgtggtc 1680
   acattttctg tttatgtcct ggtggatagc aacaatattt tggatgcaca aaaggccttc 1740
   acctccatta ccctcttcaa tatcctgcgc tttcccctga gcatgcttcc catgatgatc 1800
   tcctccatgc tccaggccag tgtttccaca gagcggctag agaagtactt gggaggggat 1860
   gacttggaca catctgccat tcgacatgac tgcaattttg acaaagccat gcagttttct 1920
   gaggcctcct ttacctggga acatgattcg gaagccacag tccgagatgt gaacctggac 1980
   attatggcag gccaacttgt ggctgtgata ggccctgtcg gctctgggaa atcctccttg 2040
   atatcagcca tgctgggaga aatggaaaat gtccacgggc acatcaccat caagggcacc 2100
   actgcctatg tcccacagca gtcctggatt cagaatggca ccataaagga caacatcctt 2160
   tttggaacag agtttaatga aaagaggtac cagcaagtac tggaggcctg tgctctcctc 2220
   ccagacttgg aaatgctgcc tggaggagat ttggctgaga ttggagagaa gggtataaat 2280
   cttagtgggg gtcagaagca gcggatcagc ctggccagag ctacctacca aaatttagac 2340
   atctatcttc tagatgaccc cctgtctgca gtggatgctc atgtaggaaa acatattttt 2400
   aataaggtct tgggccccaa tggcctgttg aaaggcaaga ctcgactctt ggttacacat 2460
   agcatgcact ttcttcctca agtggatgag attgtagttc tggggaatgg aacaattgta 2520
   gagaaaggat cctacagtgc tctcctggcc aaaaaaggag agtttgctaa gaatctgaag 2580
   acatttctaa gacatacagg ccctgaagag gaagccacag tccatgatgg cagtgaagaa 2640
   gaagacgatg actatgggct gatatccagt gtggaagaga tccccgaaga tgcagcctcc 2700
   ataaccatga gaagagagaa cagctttcgt cgaacactta gccgcagttc taggtccaat 2760
   ggcaggcatc tgaagtccct gagaaactcc ttgaaaactc ggaatgtgaa tagcctgaag 2820
   gaagacgaag aactagtgaa aggacaaaaa ctaattaaga aggaattcat agaaactgga 2880
   aaggtgaagt tctccatcta cctggagtac ctacaagcaa taggattgtt ttcgatattc 2940
   ttcatcatcc ttgcgtttgt gatgaattct gtggctttta ttggatccaa cctctggctc 3000
   agtgcttgga ccagtgactc taaaatcttc aatagcaccg actatccagc atctcagagg 3060
   gacatgagag ttggagtcta cggagctctg ggattagccc aaggtatatt tgtgttcata 3120
   gcacatttct ggagtgcctt tggtttcgtc catgcatcaa atatcttgca caagcaactg 3180
   ctgaacaata tccttcgagc acctatgaga ttttttgaca caacacccac aggccggatt 3240
   gtgaacaggt ttgccggcga tatttccaca gtggatgaca ccctgcctca gtccttgcgc 3300
   agctggatta catgcttcct ggggataatc agcacccttg tcatgatctg catggccact 3360
   cctgtcttca ccatcatcgt cattcctctt ggcattattt atgtatctgt tcagatgttt 3420
   tatgtgtcta cctcccgcca gctgaggcgt ctggactctg tcaccaggtc cccaatctac 3480
   tctcacttca gcgagaccgt atcaggtttg ccagttatcc gtgcctttga gcaccagcag 3540
   cgatttctga aacacaatga ggtgaggatt gacaccaacc agaaatgtgt cttttcctgg 3600
   atcacctcca acaggtggct tgcaattcgc ctggagctgg ttgggaacct gactgtcttc 3660
   ttttcagcct tgatgatggt tatttataga gataccctaa gtggggacac tgttggcttt 3720
   gttctgtcca atgcactcaa tatcacacaa accctgaact ggctggtgag gatgacatca 3780
   gaaatagaga ccaacattgt ggctgttgag cgaataactg agtacacaaa agtggaaaat 3840
   gaggcaccct gggtgactga taagaggcct ccgccagatt ggcccagcaa aggcaagatc 3900
   cagtttaaca actaccaagt gcggtaccga cctgagctgg atctggtcct cagagggatc 3960
   acttgtgaca tcggtagcat ggagaagatt ggtgtggtgg gcaggacagg agctggaaag 4020
   tcatccctca caaactgcct cttcagaatc ttagaggctg ccggtggtca gattatcatt 4080
   gatggagtag atattgcttc cattgggctc cacgacctcc gagagaagct gaccatcatc 4140
   ccccaggacc ccatcctgtt ctctggaagc ctgaggatga atctcgaccc tttcaacaac 4200
   tactcagatg aggagatttg gaaggccttg gagctggctc acctcaagtc ttttgtggcc 4260
   agcctgcaac ttgggttatc ccacgaagtg acagaggctg gtggcaacct gagcataggc 4320
   cagaggcagc tgctgtgcct gggcagggct ctgcttcgga aatccaagat cctggtcctg 4380
   gatgaggcca ctgctgcggt ggatctagag acagacaacc tcattcagac gaccatccaa 4440
   aacgagttcg cccactgcac agtgatcacc atcgcccaca ggctgcacac catcatggac 4500
   agtgacaagg taatggtcct agacaacggg aagattatag agtgcggcag ccctgaagaa 4560
   ctgctacaaa tccctggacc cttttacttt atggctaagg aagctggcat tgagaatgtg 4620
   aacagcacaa aattctag 4638
<210> 15
   <211> 1325
   <212> PRT
   <213> Homo sapiens
<400> 15
   Met Leu Pro Val Tyr Gln Glu Val Lys Pro Asn Pro Leu Gln Asp Ala
   1               5                   10                  15
   Asn Leu Cys Ser Arg Val Phe Phe Trp Trp Leu Asn Pro Leu Phe Lys
               20                  25                  30
   Ile Gly His Lys Arg Arg Leu Glu Glu Asp Asp Met Tyr Ser Val Leu
           35                  40                  45
   Pro Glu Asp Arg Ser Gln His Leu Gly Glu Glu Leu Gln Gly Phe Trp
       50                  55                  60
   Asp Lys Glu Val Leu Arg Ala Glu Asn Asp Ala Gln Lys Pro Ser Leu
   65                  70                  75                  80
   Thr Arg Ala Ile Ile Lys Cys Tyr Trp Lys Ser Tyr Leu Val Leu Gly
                   85                  90                  95
   Ile Phe Thr Leu Ile Glu Glu Ser Ala Lys Val Ile Gln Pro Ile Phe
               100                 105                 110
   Leu Gly Lys Ile Ile Asn Tyr Phe Glu Asn Tyr Asp Pro Met Asp Ser
           115                 120                 125
   Val Ala Leu Asn Thr Ala Tyr Ala Tyr Ala Thr Val Leu Thr Phe Cys
       130                 135                 140
   Thr Leu Ile Leu Ala Ile Leu His His Leu Tyr Phe Tyr His Val Gln
   145                 150                 155                 160
   Cys Ala Gly Met Arg Leu Arg Val Ala Met Cys His Met Ile Tyr Arg
                   165                 170                 175
   Lys Ala Leu Arg Leu Ser Asn Met Ala Met Gly Lys Thr Thr Thr Gly
               180                 185                 190
   Gln Ile Val Asn Leu Leu Ser Asn Asp Val Asn Lys Phe Asp Gln Val
           195                 200                 205
   Thr Val Phe Leu His Phe Leu Trp Ala Gly Pro Leu Gln Ala Ile Ala
       210                 215                 220
   Val Thr Ala Leu Leu Trp Met Glu Ile Gly Ile Ser Cys Leu Ala Gly
   225                 230                 235                 240
   Met Ala Val Leu Ile Ile Leu Leu Pro Leu Gln Ser Cys Phe Gly Lys
                   245                 250                 255
   Leu Phe Ser Ser Leu Arg Ser Lys Thr Ala Thr Phe Thr Asp Ala Arg
               260                 265                 270
   Ile Arg Thr Met Asn Glu Val Ile Thr Gly Ile Arg Ile Ile Lys Met
           275                 280                 285
   Tyr Ala Trp Glu Lys Ser Phe Ser Asn Leu Ile Thr Asn Leu Arg Lys
       290                 295                 300
   Lys Glu Ile Ser Lys Ile Leu Arg Ser Ser Cys Leu Arg Gly Met Asn
   305 310 315 320
   Leu Ala Ser Phe Phe Ser Ala Ser Lys Ile Ile Val Phe Val Thr Phe
   325 330 335
   Thr Thr Tyr Val Leu Leu Gly Ser Val Ile Thr Ala Ser Arg Val Phe
   340 345 350
   Val Ala Val Thr Leu Tyr Gly Ala Val Arg Leu Thr Val Thr Leu Phe
   355 360 365
   Phe Pro Ser Ala Ile Glu Arg Val Ser Glu Ala Ile Val Ser Ile Arg
   370 375 380
   Arg Ile Gln Thr Phe Leu Leu Leu Asp Glu Ile Ser Gln Arg Asn Arg
   385 390 395 400
   Gln Leu Pro Ser Asp Gly Lys Lys Met Val His Val Gln Asp Phe Thr
   405 410 415
   Ala Phe Trp Asp Lys Ala Ser Glu Thr Pro Thr Leu Gln Gly Leu Ser
   420 425 430
   Phe Thr Val Arg Pro Gly Glu Leu Leu Ala Val Val Gly Pro Val Gly
   435 440 445
   Ala Gly Lys Ser Ser Leu Leu Ser Ala Val Leu Gly Glu Leu Ala Pro
   450 455 460
   Ser His Gly Leu Val Ser Val His Gly Arg Ile Ala Tyr Val Ser Gln
   465 470 475 480
   Gln Pro Trp Val Phe Ser Gly Thr Leu Arg Ser Asn Ile Leu Phe Gly
   485 490 495
   Lys Lys Tyr Glu Lys Glu Arg Tyr Glu Lys Val Ile Lys Ala Cys Ala
   500 505 510
   Leu Lys Lys Asp Leu Gln Leu Leu Glu Asp Gly Asp Leu Thr Val Ile
   515 520 525
   Gly Asp Arg Gly Thr Thr Leu Ser Gly Gly Gln Lys Ala Arg Val Asn
   530 535 540
   Leu Ala Arg Ala Val Tyr Gln Asp Ala Asp Ile Tyr Leu Leu Asp Asp
   545 550 555 560
   Pro Leu Ser Ala Val Asp Ala Glu Val Ser Arg His Leu Phe Glu Leu
   565 570 575
   Cys Ile Cys Gln Ile Leu His Glu Lys Ile Thr Ile Leu Val Thr His
   580 585 590
   Gln Leu Gln Tyr Leu Lys Ala Ala Ser Gln Ile Leu Ile Leu Lys Asp
   595 600 605
   Gly Lys Met Val Gln Lys Gly Thr Tyr Thr Glu Phe Leu Lys Ser Gly
   610 615 620
   Ile Asp Phe Gly Ser Leu Leu Lys Lys Asp Asn Glu Glu Ser Glu Gln
   625 630 635 640
   Pro Pro Val Pro Gly Thr Pro Thr Leu Arg Asn Arg Thr Phe Ser Glu
   645 650 655
   Ser Ser Val Trp Ser Gln Gln Ser Ser Arg Pro Ser Leu Lys Asp Gly
   660 665 670
   Ala Leu Glu Ser Gln Asp Thr Glu Asn Val Pro Val Thr Leu Ser Glu
   675 680 685
   Glu Asn Arg Ser Glu Gly Lys Val Gly Phe Gln Ala Tyr Lys Asn Tyr
   690 695 700
   Phe Arg Ala Gly Ala His Trp Ile Val Phe Ile Phe Leu Ile Leu Leu
   705 710 715 720
   Asn Thr Ala Ala Gln Val Ala Tyr Val Leu Gln Asp Trp Trp Leu Ser
   725 730 735
   Tyr Trp Ala Asn Lys Gln Ser Met Leu Asn Val Thr Val Asn Gly Gly
   740 745 750
   Gly Asn Val Thr Glu Lys Leu Asp Leu Asn Trp Tyr Leu Gly Ile Tyr
   755 760 765
   Ser Gly Leu Thr Val Ala Thr Val Leu Phe Gly Ile Ala Arg Ser Leu
   770 775 780
   Leu Val Phe Tyr Val Leu Val Asn Ser Ser Gln Thr Leu His Asn Lys
   785 790 795 800
   Met Phe Glu Ser Ile Leu Lys Ala Pro Val Leu Phe Phe Asp Arg Asn
   805 810 815
   Pro Ile Gly Arg Ile Leu Asn Arg Phe Ser Lys Asp Ile Gly His Leu
   820 825 830
   Asp Asp Leu Leu Pro Leu Thr Phe Leu Asp Phe Ile Gln Thr Leu Leu
   835 840 845
   Gln Val Val Gly Val Val Ser Val Ala Val Ala Val Ile Pro Trp Ile
   850 855 860
   Ala Ile Pro Leu Val Pro Leu Gly Ile Ile Phe Ile Phe Leu Arg Arg
   865 870 875 880
   Tyr Phe Leu Glu Thr Ser Arg Asp Val Lys Arg Leu Glu Ser Thr Thr
   885 890 895
   Arg Ser Pro Val Phe Ser His Leu Ser Ser Ser Leu Gln Gly Leu Trp
   900 905 910
   Thr Ile Arg Ala Tyr Lys Ala Glu Glu Arg Cys Gln Glu Leu Phe Asp
   915 920 925
   Ala His Gln Asp Leu His Ser Glu Ala Trp Phe Leu Phe Leu Thr Thr
   930 935 940
   Ser Arg Trp Phe Ala Val Arg Leu Asp Ala Ile Cys Ala Met Phe Val
   945 950 955 960
   Ile Ile Val Ala Phe Gly Ser Leu Ile Leu Ala Lys Thr Leu Asp Ala
   965 970 975
   Gly Gln Val Gly Leu Ala Leu Ser Tyr Ala Leu Thr Leu Met Gly Met
   980 985 990
   Phe Gln Trp Cys Val Arg Gln Ser Ala Glu Val Glu Asn Met Met Ile
   995 1000 1005
   Ser Val Glu Arg Val Ile Glu Tyr Thr Asp Leu Glu Lys Glu Ala
   1010 1015 1020
   Pro Trp Glu Tyr Gln Lys Arg Pro Pro Pro Ala Trp Pro His Glu
   1025 1030 1035
   Gly Val Ile Ile Phe Asp Asn Val Asn Phe Met Tyr Ser Pro Gly
   1040 1045 1050
   Gly Pro Leu Val Leu Lys His Leu Thr Ala Leu Ile Lys Ser Gln
   1055 1060 1065
   Glu Lys Val Gly Ile Val Gly Arg Thr Gly Ala Gly Lys Ser Ser
   1070 1075 1080
   Leu Ile Ser Ala Leu Phe Arg Leu Ser Glu Pro Glu Gly Lys Ile
   1085 1090 1095
   Trp Ile Asp Lys Ile Leu Thr Thr Glu Ile Gly Leu His Asp Leu
   1100 1105 1110
   Arg Lys Lys Met Ser Ile Ile Pro Gln Glu Pro Val Leu Phe Thr
   1115 1120 1125
   Gly Thr Met Arg Lys Asn Leu Asp Pro Phe Asn Glu His Thr Asp
   1130 1135 1140
   Glu Glu Leu Trp Asn Ala Leu Gln Glu Val Gln Leu Lys Glu Thr
   1145 1150 1155
   Ile Glu Asp Leu Pro Gly Lys Met Asp Thr Glu Leu Ala Glu Ser
   1160 1165 1170
   Gly Ser Asn Phe Ser Val Gly Gln Arg Gln Leu Val Cys Leu Ala
   1175 1180 1185
   Arg Ala Ile Leu Arg Lys Asn Gln Ile Leu Ile Ile Asp Glu Ala
   1190 1195 1200
   Thr Ala Asn Val Asp Pro Arg Thr Asp Glu Leu Ile Gln Lys Lys
   1205 1210 1215
   Ile Arg Glu Lys Phe Ala His Cys Thr Val Leu Thr Ile Ala His
   1220 1225 1230
   Arg Leu Asn Thr Ile Ile Asp Ser Asp Lys Ile Met Val Leu Asp
   1235 1240 1245
   Ser Gly Arg Leu Lys Glu Tyr Asp Glu Pro Tyr Val Leu Leu Gln
   1250 1255 1260
   Asn Lys Glu Ser Leu Phe Tyr Lys Met Val Gln Gln Leu Gly Lys
   1265 1270 1275
   Ala Glu Ala Ala Ala Leu Thr Glu Thr Ala Lys Gln Val Tyr Phe
   1280 1285 1290
   Lys Arg Asn Tyr Pro His Ile Gly His Thr Asp His Met Val Thr
       1295                1300                1305
   Asn Thr Ser Asn Gly Gln Pro Ser Thr Leu Thr Ile Phe Glu Thr
       1310                1315                1320
   Ala Leu
       1325
<210> 16
   <211> 3978
   <212> DNA
   <213> Homo sapiens
<400> 16
<210> 17
   <211> 570
   <212> PRT
   <213> Homo sapiens
<400> 17
   Met Glu Ala Pro Glu Glu Pro Ala Pro Val Arg Gly Gly Pro Glu Ala
   1               5                   10                  15
   Thr Leu Glu Val Arg Gly Ser Arg Cys Leu Arg Leu Ser Ala Phe Arg
               20                  25                  30
   Glu Glu Leu Arg Ala Leu Leu Val Leu Ala Gly Pro Ala Phe Leu Val
           35                  40                  45
   Gln Leu Met Val Phe Leu Ile Ser Phe Ile Ser Ser Val Phe Cys Gly
       50                  55                  60
   His Leu Gly Lys Leu Glu Leu Asp Ala Val Thr Leu Ala Ile Ala Val
   65                  70                  75                  80
   Ile Asn Val Thr Gly Val Ser Val Gly Phe Gly Leu Ser Ser Ala Cys
                   85                  90                  95
   Asp Thr Leu Ile Ser Gln Thr Tyr Gly Ser Gln Asn Leu Lys His Val
               100                 105                 110
   Gly Val Ile Leu Gln Arg Ser Ala Leu Val Leu Leu Leu Cys Cys Phe
           115                 120                 125
   Pro Cys Trp Ala Leu Phe Leu Asn Thr Gln His Ile Leu Leu Leu Phe
       130                 135                 140
   Arg Gln Asp Pro Asp Val Ser Arg Leu Thr Gln Thr Tyr Val Thr Ile
   145                 150                 155                 160
   Phe Ile Pro Ala Leu Pro Ala Thr Phe Leu Tyr Met Leu Gln Val Lys
                   165                 170                 175
   Tyr Leu Leu Asn Gln Gly Ile Val Leu Pro Gln Ile Val Thr Gly Val
               180                 185                 190
   Ala Ala Asn Leu Val Asn Ala Leu Ala Asn Tyr Leu Phe Leu His Gln
           195                 200                 205
   Leu His Leu Gly Val Ile Gly Ser Ala Leu Ala Asn Leu Ile Ser Gln
       210                 215                 220
   Tyr Thr Leu Ala Leu Leu Leu Phe Leu Tyr Ile Leu Gly Lys Lys Leu
   225                 230                 235                 240
   His Gln Ala Thr Trp Gly Gly Trp Ser Leu Glu Cys Leu Gln Asp Trp
                   245                 250                 255
   Ala Ser Phe Leu Arg Leu Ala Ile Pro Ser Met Leu Met Leu Cys Met
               260                 265                 270
   Glu Trp Trp Ala Tyr Glu Val Gly Ser Phe Leu Ser Gly Ile Leu Gly
           275                 280                 285
   Met Val Glu Leu Gly Ala Gln Ser Ile Val Tyr Glu Leu Ala Ile Ile
       290                 295                 300
   Val Tyr Met Val Pro Ala Gly Phe Ser Val Ala Ala Ser Val Arg Val
   305                 310                 315                 320
   Gly Asn Ala Leu Gly Ala Gly Asp Met Glu Gln Ala Arg Lys Ser Ser
                   325                 330                 335
   Thr Val Ser Leu Leu Ile Thr Val Leu Phe Ala Val Ala Phe Ser Val
               340                 345                 350
   Leu Leu Leu Ser Cys Lys Asp His Val Gly Tyr Ile Phe Thr Thr Asp
           355                 360                 365
   Arg Asp Ile Ile Asn Leu Val Ala Gln Val Val Pro Ile Tyr Ala Val
       370                 375                 380
   Ser His Leu Phe Glu Ala Leu Ala Cys Thr Ser Gly Gly Val Leu Arg
   385                 390                 395                 400
   Gly Ser Gly Asn Gln Lys Val Gly Ala Ile Val Asn Thr Ile Gly Tyr
                   405                 410                 415
   Tyr Val Val Gly Leu Pro Ile Gly Ile Ala Leu Met Phe Ala Thr Thr
               420                 425                 430
   Leu Gly Val Met Gly Leu Trp Ser Gly Ile Ile Ile Cys Thr Val Phe
           435                 440                 445
   Gln Ala Val Cys Phe Leu Gly Phe Ile Ile Gln Leu Asn Trp Lys Lys
       450                 455                 460
   Ala Cys Gln Gln Ala Gln Val His Ala Asn Leu Lys Val Asn Asn Val
   465                 470                 475                 480
   Pro Arg Ser Gly Asn Ser Ala Leu Pro Gln Asp Pro Leu His Pro Gly
                   485                 490                 495
   Cys Pro Glu Asn Leu Glu Gly Ile Leu Thr Asn Asp Val Gly Lys Thr
               500                 505                 510
   Gly Glu Pro Gln Ser Asp Gln Gln Met Arg Gln Glu Glu Pro Leu Pro
           515                 520                 525
   Glu His Pro Gln Asp Gly Ala Lys Leu Ser Arg Lys Gln Leu Val Leu
       530                 535                 540
   Arg Arg Gly Leu Leu Leu Leu Gly Val Phe Leu Ile Leu Leu Val Gly
   545                 550                 555                 560
   Ile Leu Val Arg Phe Tyr Val Arg Ile Gln
                   565                 570
<210> 18
   <211> 1713
   <212> DNA
   <213> Homo sapiens
<400> 18
   atggaagctc ctgaggagcc cgcgccagtg cgcggaggcc cggaggccac ccttgaggtc 60
   cgtgggtcgc gctgcttgcg gctgtccgcc ttccgagaag agctgcgggc gctcttggtc 120
   ctggctggcc ccgcgttctt ggttcagctg atggtgttcc tgatcagctt cataagctcc 180
   gtgttctgtg gccacctggg caagctggag ctggatgcag tcacgctggc aatcgcggtt 240
   atcaatgtca ctggtgtctc agtgggattc ggcttatctt ctgcctgtga caccctcatc 300
   tcccagacgt acgggagcca gaacctgaag cacgtgggcg tgatcctgca gcggagtgcg 360
   ctcgtcctgc tcctctgctg cttcccctgc tgggcgctct ttctcaacac ccagcacatc 420
   ctgctgctct tcaggcagga cccagatgtg tccaggctta cccagaccta tgtcacgatc 480
   ttcattccag ctcttcctgc aacctttctt tatatgttac aagttaaata tttgctcaac 540
   cagggaattg tactgcccca gatcgtaact ggagttgcag ccaaccttgt caatgccctc 600
   gccaactatc tgtttctcca tcaactgcat cttggggtga taggctctgc actggcaaac 660
   ttgatttccc agtacaccct ggctctactc ctctttctct acatcctcgg gaaaaaactg 720
   catcaagcta catggggagg ctggtccctc gagtgcctgc aggactgggc ctccttcctc 780
   cgcctggcca tccccagcat gctcatgctg tgcatggagt ggtgggccta tgaggtcggg 840
   agcttcctca gtggcatcct cggcatggtg gagctgggcg ctcagtccat cgtgtatgaa 900
   ctggccatca ttgtgtacat ggtccctgca ggcttcagtg tggctgccag tgtccgggta 960
   ggaaacgctc tgggtgctgg agacatggag caggcacgga agtcctctac cgtttccctg 1020
   ctgattacag tgctctttgc tgtagccttc agtgtcctgc tgttaagctg taaggatcac 1080
   gtggggtaca tttttactac cgaccgagac atcattaatc tggtggctca ggtggttcca 1140
   atttatgctg tttcccacct ctttgaagct cttgcttgca cgagtggtgg tgttctgagg 1200
   gggagtggaa atcagaaggt tggagccatt gtgaatacca ttgggtacta tgtggttggc 1260
   ctccccatcg ggatcgcgct gatgtttgca accacacttg gagtgatggg tctgtggtca 1320
   gggatcatca tctgtacagt ctttcaagct gtgtgttttc taggctttat tattcagcta 1380
   aattggaaaa aagcctgtca gcaggctcag gtacacgcca atttgaaagt aaacaacgtg 1440
   cctcggagtg ggaattctgc tctccctcag gatccgcttc acccagggtg ccctgaaaac 1500
   cttgaaggaa ttttaacgaa cgatgttgga aagacaggcg agcctcagtc agatcagcag 1560
   atgcgccaag aagaaccttt gccggaacat ccacaggacg gcgctaaatt gtccaggaaa 1620
   cagctggtgc tgcggcgagg gcttctgctc ctgggggtct tcttaatctt gctggtgggg 1680
   attttagtga gattctatgt cagaattcag tga 1713
<210> 19
   <211> 580
   <212> PRT
   <213> Homo sapiens
<400> 19
   Met Asp Ser Leu Gln Asp Thr Val Ala Leu Asp His Gly Gly Cys Cys
   1               5                   10                  15
   Pro Ala Leu Ser Arg Leu Val Pro Arg Gly Phe Gly Thr Glu Met Trp
               20                  25                  30
   Thr Leu Phe Ala Leu Ser Gly Pro Leu Phe Leu Phe Gln Val Leu Thr
           35                  40                  45
   Phe Met Ile Tyr Ile Val Ser Thr Val Phe Cys Gly His Leu Gly Lys
       50                  55                  60
   Val Glu Leu Ala Ser Val Thr Leu Ala Val Ala Phe Val Asn Val Cys
   65                  70                  75                  80
   Gly Val Ser Val Gly Val Gly Leu Ser Ser Ala Cys Asp Thr Leu Met
                   85                  90                  95
   Ser Gln Ser Phe Gly Ser Pro Asn Lys Lys His Val Gly Val Ile Leu
               100                 105                 110
   Gln Arg Gly Ala Leu Val Leu Leu Leu Cys Cys Leu Pro Cys Trp Ala
           115                 120                 125
   Leu Phe Leu Asn Thr Gln His Ile Leu Leu Leu Phe Arg Gln Asp Pro
       130                 135                 140
   Asp Val Ser Arg Leu Thr Gln Asp Tyr Val Met Ile Phe Ile Pro Gly
   145                 150                 155                 160
   Leu Pro Val Ile Phe Leu Tyr Asn Leu Leu Ala Lys Tyr Leu Gln Asn
                   165                 170                 175
   Gln Lys Ile Thr Trp Pro Gln Val Leu Ser Gly Val Val Gly Asn Cys
               180                 185                 190
   Val Asn Gly Val Ala Asn Tyr Ala Leu Val Ser Val Leu Asn Leu Gly
           195                 200                 205
   Val Arg Gly Ser Ala Tyr Ala Asn Ile Ile Ser Gln Phe Ala Gln Thr
       210                 215                 220
   Val Phe Leu Leu Leu Tyr Ile Val Leu Lys Lys Leu His Leu Glu Thr
   225                 230                 235                 240
   Trp Ala Gly Trp Ser Ser Gln Cys Leu Gln Asp Trp Gly Pro Phe Phe
                   245                 250                 255
   Ser Leu Ala Val Pro Ser Met Leu Met Ile Cys Val Glu Trp Trp Ala
   260 265 270
   Tyr Glu Ile Gly Ser Phe Leu Met Gly Leu Leu Ser Val Val Asp Leu
   275 280 285
   Ser Ala Gln Ala Val Ile Tyr Glu Val Ala Thr Val Thr Tyr Met Arg
   290 295 300
   His Ser His Arg Leu Ala Tyr Ala Ala His Val Thr Arg Ile Pro Leu
   305 310 315 320
   Gly Leu Ser Ile Gly Val Cys Val Arg Val Gly Met Ala Leu Gly Ala
   325 330 335
   Ala Asp Thr Val Gln Ala Lys Arg Ser Ala Val Ser Gly Val Leu Ser
   340 345 350
   Ile Val Gly Ile Ser Leu Val Leu Gly Thr Leu Ile Ser Ile Leu Lys
   355 360 365
   Asn Gln Leu Gly His Ile Phe Thr Asn Asp Glu Asp Val Ile Ala Leu
   370 375 380
   Val Ser Gln Val Leu Pro Val Tyr Ser Val Phe His Val Phe Glu Ala
   385 390 395 400
   Ile Cys Cys Val Tyr Gly Gly Val Leu Arg Gly Thr Gly Lys Gln Ala
   405 410 415
   Phe Gly Ala Ala Val Asn Ala Ile Thr Tyr Tyr Ile Ile Gly Leu Pro
   420 425 430
   Leu Gly Ile Leu Leu Thr Phe Val Val Arg Met Arg Ile Met Gly Leu
   435 440 445
   Trp Leu Gly Met Leu Ala Cys Val Phe Leu Ala Thr Ala Ala Phe Val
   450 455 460
   Ala Tyr Thr Ala Arg Leu Asp Trp Lys Leu Ala Ala Glu Glu Ala Lys
   465 470 475 480
   Lys His Ser Gly Arg Gln Gln Gln Gln Arg Ala Glu Ser Thr Ala Thr
   485 490 495
   Arg Pro Gly Pro Glu Lys Ala Val Leu Ser Ser Val Ala Thr Gly Ser
   500 505 510
   Ser Pro Gly Ile Thr Leu Thr Thr Tyr Ser Arg Ser Glu Cys His Val
           515                 520                 525
   Asp Phe Phe Arg Thr Pro Glu Glu Ala His Ala Leu Ser Ala Pro Thr
       530                 535                 540
   Ser Arg Leu Ser Val Lys Gln Leu Val Ile Arg Arg Gly Ala Ala Leu
   545                 550                 555                 560
   Gly Ala Ala Ser Ala Thr Leu Met Val Gly Leu Thr Val Arg Ile Leu
                   565                 570                 575
   Ala Thr Arg His
               580
<210> 20
   <211> 1743
   <212> DNA
   <213> Homo sapiens
<400> 20
   atggacagcc tccaggacac agtggccctg gaccatgggg gctgctgccc tgccctcagc      60
   aggctggttc ccagaggctt tgggactgag atgtggactc tctttgccct ttctggaccc     120
   ctgttcctgt tccaggtgct gacttttatg atctacatcg tgagcactgt gttctgcggg     180
   cacctgggca aggtggagct ggcatcggtg accctcgcgg tggcctttgt caatgtctgc     240
   ggagtttctg taggagttgg tttgtcttcg gcatgtgaca ccttgatgtc tcagagcttc     300
   ggcagcccca acaagaagca cgtgggcgtg atcctgcagc ggggcgcgct ggtcctgctc     360
   ctctgctgcc tcccttgctg ggcgctcttc ctcaacaccc agcacatcct gctgctcttc     420
   cggcaggacc cggacgtgtc caggttgacc caggactatg taatgatttt cattccagga     480
   cttccggtga tttttcttta caatctgctg gcaaaatatt tgcaaaatca gaagatcacc     540
   tggccccaag tcctcagtgg tgtggtgggc aactgtgtca acggtgtggc caactatgcc     600
   ctggtttctg tgctgaacct gggggtcagg ggctccgcct atgccaacat catctcccag     660
   tttgcacaga ccgtcttcct ccttctctac attgtgctga agaagctgca cctggagacg     720
   tgggcaggtt ggtccagcca gtgcctgcag gactggggcc ccttcttctc cctggctgtc     780
   cccagcatgc tcatgatctg tgttgagtgg tgggcctatg agatcgggag cttcctcatg     840
   gggctgctca gtgtggtgga tctctctgcc caggctgtca tctacgaggt ggccactgtg     900
   acctacatga gacacagcca tcgtcttgct tatgcagccc atgtcactcg gattcccttg     960
   gggctcagca tcggggtctg tgtccgagtg gggatggctc tgggggctgc ggatactgtg    1020
   caggccaagc gctcggccgt ctcgggcgtg ctcagcatag ttggcatttc cctggtcctg    1080
   ggcaccctga taagcatcct gaaaaatcag ctggggcata tttttaccaa tgatgaagat 1140
   gtcattgccc tggtgagcca ggtcttgccg gtttatagtg tctttcacgt gtttgaggcc 1200
   atctgttgtg tctatggcgg agttctgaga ggaactggga agcaggcctt tggtgccgct 1260
   gtgaatgcca tcacatatta catcatcggc ctaccactgg gcatccttct gacctttgtg 1320
   gtcagaatga gaatcatggg cctctggctg ggcatgctgg cctgtgtctt cctggcaact 1380
   gctgcctttg ttgcttatac tgcccggctg gactggaagc ttgctgcaga ggaggctaag 1440
   aaacattcag gccggcagca gcagcagaga gcagagagca ctgcaaccag acctgggcct 1500
   gagaaagcag tcctatcttc agtggctaca ggcagttccc ctggcattac cttgacaacg 1560
   tattcaaggt ctgagtgcca cgtggacttc ttcaggactc cagaggaggc ccacgccctt 1620
   tcagctccta ccagcagact atcagtgaaa cagctggtca tccgccgtgg ggctgctctg 1680
   ggggcggcgt cagccacact gatggtgggg ctcacggtca ggatcctagc caccaggcac 1740
   tag 1743
<210> 21
   <211> 639
   <212> PRT
   <213> Homo sapiens
<400> 21
<210> 22
   <211> 1920
   <212> DNA
   <213> Homo sapiens
<400> 22
   atgttgtcct acggagagag gctggggtcc cctgctgtct ccccactccc agtccgtggg 60
   gggcatgtga tgcgagggac ggcctttgcc tacgtgccca gccctcaggt cctacacagg 120
   atcccgggga cctctgccta tgccttcccc agcctgggcc ctgtggccct tgctgagcac 180
   acctgcccct gtggggaggt cctggagcgc catgaaccac tgcctgccaa gctggccctg 240
   gaggaggagc agaagccaga gtccaggctg gtccccaagc tgcgccaggc tggcgccatg 300
   ctgctcaagg tgccactgat gctcaccttc ctctacctct tcgtctgctc cctggacatg 360
   ctcagctcgg ccttccagct ggctggaggg aaggtggctg gtgacatctt caaggataac 420
   gccatcctgt ccaacccggt ggccgggctg gtggtgggga tcctggtgac cgtgctggtg 480
   cagagctcca gcacctccac atccatcatc gtcagcatgg tctcctctgg cttgctggag 540
   gtgagctctg ccatccccat catcatgggc tccaacatcg gcacctctgt caccaacacc 600
   atcgtggccc tgatgcaggc gggggacagg actgacttcc ggcgggcctt cgcgggggcc 660
   acggtgcatg actgctttaa ctggctgtca gtgctggtcc tgctgcccct ggaggctgcc 720
   actggctacc tgcaccacat cactcgactt gtggtggcct ccttcaacat ccatggtggc 780
   cgtgatgctc ctgacctgct caagatcatc acagagccct tcacgaagct catcatccag 840
   ctggacgagt ctgtgataac cagcattgcc actggtgatg agtccctgag gaaccacagt 900
   ctcatccaga tctggtgcca cccagactcc ttacaggctc ccacctccat gtccagagca 960
   gaggccaact ccagccagac ccttggaaat gccaccatgg agaaatgcaa ccacatcttt 1020
   gtggacactg gcctaccgga cctggctgtg gggctcatcc tgctggcagg atccctggtg 1080
   ctgctgtgca cctgcctcat cctcctagtc aagatgctca actccctgct caagggccaa 1140
   gtggccaagg tcatccagaa ggtcatcaat acggacttcc ctgccccctt cacctgggtc 1200
   acaggctact ttgccatggt ggtgggcgcc agcatgacct tcgtggtcca gagcagttct 1260
   gtgttcacct cggccatcac cccactcatc ggtcttggtg tgatcagcat tgagagggcc 1320
   tacccgctca cactgggttc caacatcggc accaccacca cggccatcct ggctgccctg 1380
   gccagcccca gggagaagct gtccagcgct ttccagattg ccctctgtca cttcttcttc 1440
   aacatctcgg gtatccttct gtggtacccg gtgccctgca cacgcctgcc catccgcatg 1500
   gccaaggcgc tggggaaacg cacggccaag taccgctggt ttgccgtcct ctatctcctt 1560
   gtctgcttcc tgctgctgcc ctcactggtg tttggcatct ccatggcagg ctggcaggtc 1620
   atggtaggtg tgggcacgcc cttcggggcc ctgctggcct tcgtggtgct catcaatgtc 1680
   ctgcagagtc ggagtcccgg gcacctgccc aagtggttac agacatggga cttcctgcct 1740
   cgctggatgc actccctgaa gcccctggac cacctcatca cccgcgccac cctatgctgt 1800
   gccaggcctg agccccgctc acccccgctg ccccccaggg tcttcctgga ggagctaccc 1860
   cctgccacac cctccccccg tcttgcactg cctgctcacc acaatgccac ccgcctctag 1920
<210> 23
   <211> 599
   <212> PRT
   <213> Homo sapiens
<400> 23
<210> 24
   <211> 1800
   <212> DNA
   <213> Homo sapiens
<400> 24
   atgccgagtt cccttcccgg cagccaggtc ccccacccca ctctggacgc ggttgaccta 60
   gtggaaaaga ctctgaggaa tgaagggacc tccagttctg ctccagtctt ggaggaaggg 120
   gacacagacc cctggaccct ccctcagctg aaggacacaa gccagccctg gaaagagctc 180
   cgcgtggccg gcaggctgcg ccgcgtggcc ggcagcgtcc tcaaggcctg cgggctcctc 240
   ggcagcctgt acttcttcat ctgctctctg gacgtcctca gctccgcctt ccagctgctg 300
   ggcagcaaag tggccggaga catcttcaag gacaacgtgg tgctgtccaa ccctgtggct 360
   ggactggtca ttggcgtgct ggtcacagcc ctggtgcaga gttccagcac gtcctcctcc 420
   atcgtggtca gcatggtggc tgctaagctg ctgactgtcc gggtgtctgt gcccatcatc 480
   atgggtgtca acgtaggcac atccatcacc agcaccctgg tctcaatggc gcagtcaggg 540
   gaccgggatg aatttcagag ggctttcagc ggctcggcgg tgcacgggat cttcaactgg 600
   ctcacagtgc tggtcctgct gccactggag agcgccacgg ccctgctgga gaggctaagt 660
   gagctagccc tgggtgccgc cagcctgaca cccagggcgc aggcgcccga catcctcaag 720
   gtgctgacga agccgctcac acacctcatc gtgcagttgg actccgacat gatcatgagc 780
   agtgccacag gcaacgccac taacagcagt ctcattaagc actggtgcgg caccacgggg 840
   cagccgaccc aggagaacag cagctgtggc gccttcggcc cgtgcacaga gaagaacagc 900
   acagccccgg cggacaggct gccctgccgc cacctgtttg cgggcacgga gctcacggac 960
   ctggccgtgg gctgcatcct gctggccggc tccctgctgg tgctctgcgg ctgcctggtc 1020
   ctcatagtca agctgctcaa ctctgtgctg cgcggccgcg tggcccaggt cgtgaggaca 1080
   gtcatcaatg cggacttccc cttcccgctg ggctggctcg gcggctacct ggccgtcctc 1140
   gcgggcgccg gcctgacctt cgcactgcag agcagcagcg tcttcacggc ggccgtcgtg 1200
   cccctcatgg gggtcggggt gatcagtctg gaccgggcgt accccctctt actgggctcc 1260
   aacatcggca ccactaccac agccctgctg gctgccctgg ccagccccgc agacaggatg 1320
   ctcagcgccc tgcaggtcgc cctcatccac ttcttcttca acctggccgg catcctgctg 1380
   tggtacctgg tgcctgcact gcggctgccc atcccgctgg ccaggcactt cggggtggtg 1440
   accgcccgtt accgctgggt ggctggggtc tacctgctgc tcggattcct gctgctgccc 1500
   ctggcggcct tcgggctctc cctggcaggg ggcatggagc tggccgctgt cgggggtccc 1560
   ctggtggggc tggtgctcct cgtcatcctg gttactgtcc tgcagcggcg ccggccggcc 1620
   tggctgcctg tccgcctgcg ctcctgggcc tggctccccg tctggctcca ttctctggag 1680
   ccctgggacc gcctggtgac ccgctgctgc ccctgcaacg tctgcagccc cccgaaggcc 1740
   accaccaaag aggcctactg ctacgagaac cctgagatct tggcctccca gcagttgtga 1800
<210> 25
   <211> 8754
   <212> DNA
   <213> Artificial
<220>
   <223> Synthetic lentiviral construct
<400> 25
   cgatgccgcc gttgacattg attattgact agttattaat agtaatcaat tacggggtca 60
   ttagttcata gcccatatat ggagttccgc gttacataac ttacggtaaa tggcccgcct 120
   ggctgaccgc ccaacgaccc ccgcccattg acgtcaataa tgacgtatgt tcccatagta 180
   acgccaatag ggactttcca ttgacgtcaa tgggtggagt atttacggta aactgcccac    240
   ttggcagtac atcaagtgta tcatatgcca agtacgcccc ctattgacgt caatgacggt    300
   aaatggcccg cctggcatta tgcccagtac atgaccttat gggactttcc tacttggcag    360
   tacatctacg tattagtcat cgctattacc atggtgatgc ggttttggca gtacatcaat    420
   gggcgtggat agcggtttga ctcacgggga tttccaagtc tccaccccat tgacgtcaat    480
   gggagtttgt tttggcacca aaatcaacgg gactttccaa aatgtcgtaa caactccgcc    540
   ccattgacgc aaatgggcgg taggcgtgta cggtgggagg tctatataag cagagctctc    600
   cctatcagtg atagagatct ccctatcagt gatagagatc gtcgacgagc tcgtttagtg    660
   aaccgtcaga tcgcctggag acgccatcca cgctgttttg acctccatag aagacaccgg    720
   gaccgatcca gcctccggcc gccgaattct gcagatatca acaagtttgt acaaaaaagc    780
   aggctccacc atggccttta atgacctcct gcagcaggtg gggggtgtcg gccgcttcca    840
   gcagatccag gtcaccctgg tggtcctccc cctgctcctg atggcttctc acaacaccct    900
   gcagaacttc actgctgcca tccctaccca ccactgccgc ccgcctgccg atgccaacct    960
   cagcaagaac ggggggctgg aggtctggct gccccgggac aggcaggggc agcctgagtc   1020
   ctgcctccgc ttcacctccc cgcagtgggg actgcccttt ctcaatggca cagaagccaa   1080
   tggcacaggg gccacagagc cctgcaccga tggctggatc tatgacaaca gcaccttccc   1140
   atctaccatc gtgactgagt gggaccttgt gtgctctcac agggccctac gccagctggc   1200
   ccagtccttg tacatggtgg gggtgctgct cggagccatg gtgttcggct accttgcaga   1260
   caggctaggc cgccggaagg tactcatctt gaactacctg cagacagctg tgtcagggac   1320
   ctgcgcagcc ttcgcaccca acttccccat ctactgcgcc ttccggctcc tctcgggcat   1380
   ggctctggct ggcatctccc tcaactgcat gacactgaat gtggagtgga tgcccattca   1440
   cacacgggcc tgcgtgggca ccttgattgg ctatgtctac agcctgggcc agttcctcct   1500
   ggctggtgtg gcctacgctg tgccccactg gcgccacctg cagctactgg tctctgcgcc   1560
   tttttttgcc ttcttcatct actcctggtt cttcattgag tcggcccgct ggcactcctc   1620
   ctccgggagg ctggacctca ccctgagggc cctgcagaga gtcgcccgga tcaatgggaa   1680
   gcgggaagaa ggagccaaat tgagtatgga ggtactccgg gccagtctgc agaaggagct   1740
   gaccatgggc aaaggccagg catcggccat ggagctgctg cgctgcccca ccctccgcca   1800
   cctcttcctc tgcctctcca tgctgtggtt tgccactagc tttgcatact atgggctggt   1860
   catggacctg cagggctttg gagtcagcat ctacctaatc caggtgatct ttggtgctgt   1920
   ggacctgcct gccaagcttg tgggcttcct tgtcatcaac tccctgggtc gccggcctgc   1980
   ccagatggct gcactgctgc tggcaggcat ctgcatcctg ctcaatgggg tgatacccca   2040
   ggaccagtcc attgtccgaa cctctcttgc tgtgctgggg aagggttgtc tggctgcctc 2100
   cttcaactgc atcttcctgt atactgggga actgtatccc acaatgatcc ggcagacagg 2160
   catgggaatg ggcagcacca tggcccgagt gggcagcatc gtgagcccac tggtgagcat 2220
   gactgccgag ctctacccct ccatgcctct cttcatctac ggtgctgttc ctgtggccgc 2280
   cagcgctgtc actgtcctcc tgccagagac cctgggccag ccactgccag acacggtgca 2340
   ggacctggag agcaggaaag ggaaacagac gcgacagcaa caagagcacc agaagtatat 2400
   ggtcccactg caggcctcag cacaagagaa gaatggactc tgagacccag ctttcttgta 2460
   caaagtggtt gatatccagc acagtggcgg ccgctcgagt ctagagggcc cgcggttcga 2520
   aggtaagcct atccctaacc ctctcctcgg tctcgattct acgcgtaccg gttagtaatg 2580
   agtttggaat taattctgtg gaatgtgtgt cagttagggt gtggaaagtc cccaggctcc 2640
   ccaggcaggc agaagtatgc aaagcatgca tctcaattag tcagcaacca ggtgtggaaa 2700
   gtccccaggc tccccagcag gcagaagtat gcaaagcatg catctcaatt agtcagcaac 2760
   catagtcccg cccctaactc cgcccatccc gcccctaact ccgcccagtt ccgcccattc 2820
   tccgccccat ggctgactaa ttttttttat ttatgcagag gccgaggccg cctctgcctc 2880
   tgagctattc cagaagtagt gaggaggctt ttttggaggc ctaggctttt gcaaaaagct 2940
   ccccctgttg acaattaatc atcggcatag tatatcggca tagtataata cgacaaggtg 3000
   aggaactaaa ccatggccaa gttgaccagt gccgttccgg tgctcaccgc gcgcgacgtc 3060
   gccggagcgg tcgagttctg gaccgaccgg ctcgggttct cccgggactt cgtggaggac 3120
   gacttcgccg gtgtggtccg ggacgacgtg accctgttca tcagcgcggt ccaggaccag 3180
   gtggtgccgg acaacaccct ggcctgggtg tgggtgcgcg gcctggacga gctgtacgcc 3240
   gagtggtcgg aggtcgtgtc cacgaacttc cgggacgcct ccgggccggc catgaccgag 3300
   atcggcgagc agccgtgggg gcgggagttc gccctgcgcg acccggccgg caactgcgtg 3360
   cacttcgtgg ccgaggagca ggactgacac gtgctacgag atttaaatgg tacctttaag 3420
   accaatgact tacaaggcag ctgtagatct tagccacttt ttaaaagaaa aggggggact 3480
   ggaagggcta attcactccc aacgaagaca agatctgctt tttgcttgta ctgggtctct 3540
   ctggttagac cagatctgag cctgggagct ctctggctaa ctagggaacc cactgcttaa 3600
   gcctcaataa agcttgcctt gagtgcttca agtagtgtgt gcccgtctgt tgtgtgactc 3660
   tggtaactag agatccctca gaccctttta gtcagtgtgg aaaatctcta gcagtagtag 3720
   ttcatgtcat cttattattc agtatttata acttgcaaag aaatgaatat cagagagtga 3780
   gaggaacttg tttattgcag cttataatgg ttacaaataa agcaatagca tcacaaattt 3840
   cacaaataaa gcattttttt cactgcattc tagttgtggt ttgtccaaac tcatcaatgt 3900
   atcttatcat gtctggctct agctatcccg cccctaactc cgcccatccc gcccctaact 3960
   ccgcccagtt ccgcccattc tccgccccat ggctgactaa ttttttttat ttatgcagag 4020
   gccgaggccg cctcggcctc tgagctattc cagaagtagt gaggaggctt ttttggaggc 4080
   ctagggacgt acccaattcg ccctatagtg agtcgtatta cgcgcgctca ctggccgtcg 4140
   ttttacaacg tcgtgactgg gaaaaccctg gcgttaccca acttaatcgc cttgcagcac 4200
   atcccccttt cgccagctgg cgtaatagcg aagaggcccg caccgatcgc ccttcccaac 4260
   agttgcgcag cctgaatggc gaatgggacg cgccctgtag cggcgcatta agcgcggcgg 4320
   gtgtggtggt tacgcgcagc gtgaccgcta cacttgccag cgccctagcg cccgctcctt 4380
   tcgctttctt cccttccttt ctcgccacgt tcgccggctt tccccgtcaa gctctaaatc 4440
   gggggctccc tttagggttc cgatttagtg ctttacggca cctcgacccc aaaaaacttg 4500
   attagggtga tggttcacgt agtgggccat cgccctgata gacggttttt cgccctttga 4560
   cgttggagtc cacgttcttt aatagtggac tcttgttcca aactggaaca acactcaacc 4620
   ctatctcggt ctattctttt gatttataag ggattttgcc gatttcggcc tattggttaa 4680
   aaaatgagct gatttaacaa aaatttaacg cgaattttaa caaaatatta acgcttacaa 4740
   tttaggtggc acttttcggg gaaatgtgcg cggaacccct atttgtttat ttttctaaat 4800
   acattcaaat atgtatccgc tcatgagaca ataaccctga taaatgcttc aataatattg 4860
   aaaaaggaag agtatgagta ttcaacattt ccgtgtcgcc cttattccct tttttgcggc 4920
   attttgcctt cctgtttttg ctcacccaga aacgctggtg aaagtaaaag atgctgaaga 4980
   tcagttgggt gcacgagtgg gttacatcga actggatctc aacagcggta agatccttga 5040
   gagttttcgc cccgaagaac gttttccaat gatgagcact tttaaagttc tgctatgtgg 5100
   cgcggtatta tcccgtattg acgccgggca agagcaactc ggtcgccgca tacactattc 5160
   tcagaatgac ttggttgagt actcaccagt cacagaaaag catcttacgg atggcatgac 5220
   agtaagagaa ttatgcagtg ctgccataac catgagtgat aacactgcgg ccaacttact 5280
   tctgacaacg atcggaggac cgaaggagct aaccgctttt ttgcacaaca tgggggatca 5340
   tgtaactcgc cttgatcgtt gggaaccgga gctgaatgaa gccataccaa acgacgagcg 5400
   tgacaccacg atgcctgtag caatggcaac aacgttgcgc aaactattaa ctggcgaact 5460
   acttactcta gcttcccggc aacaattaat agactggatg gaggcggata aagttgcagg 5520
   accacttctg cgctcggccc ttccggctgg ctggtttatt gctgataaat ctggagccgg 5580
   tgagcgtggg tctcgcggta tcattgcagc actggggcca gatggtaagc cctcccgtat 5640
   cgtagttatc tacacgacgg ggagtcaggc aactatggat gaacgaaata gacagatcgc 5700
   tgagataggt gcctcactga ttaagcattg gtaactgtca gaccaagttt actcatatat 5760
   actttagatt gatttaaaac ttcattttta atttaaaagg atctaggtga agatcctttt 5820
   tgataatctc atgaccaaaa tcccttaacg tgagttttcg ttccactgag cgtcagaccc 5880
   cgtagaaaag atcaaaggat cttcttgaga tccttttttt ctgcgcgtaa tctgctgctt 5940
   gcaaacaaaa aaaccaccgc taccagcggt ggtttgtttg ccggatcaag agctaccaac 6000
   tctttttccg aaggtaactg gcttcagcag agcgcagata ccaaatactg ttcttctagt 6060
   gtagccgtag ttaggccacc acttcaagaa ctctgtagca ccgcctacat acctcgctct 6120
   gctaatcctg ttaccagtgg ctgctgccag tggcgataag tcgtgtctta ccgggttgga 6180
   ctcaagacga tagttaccgg ataaggcgca gcggtcgggc tgaacggggg gttcgtgcac 6240
   acagcccagc ttggagcgaa cgacctacac cgaactgaga tacctacagc gtgagctatg 6300
   agaaagcgcc acgcttcccg aagggagaaa ggcggacagg tatccggtaa gcggcagggt 6360
   cggaacagga gagcgcacga gggagcttcc agggggaaac gcctggtatc tttatagtcc 6420
   tgtcgggttt cgccacctct gacttgagcg tcgatttttg tgatgctcgt caggggggcg 6480
   gagcctatgg aaaaacgcca gcaacgcggc ctttttacgg ttcctggcct tttgctggcc 6540
   ttttgctcac atgttctttc ctgcgttatc ccctgattct gtggataacc gtattaccgc 6600
   ctttgagtga gctgataccg ctcgccgcag ccgaacgacc gagcgcagcg agtcagtgag 6660
   cgaggaagcg gaagagcgcc caatacgcaa accgcctctc cccgcgcgtt ggccgattca 6720
   ttaatgcagc tggcacgaca ggtttcccga ctggaaagcg ggcagtgagc gcaacgcaat 6780
   taatgtgagt tagctcactc attaggcacc ccaggcttta cactttatgc ttccggctcg 6840
   tatgttgtgt ggaattgtga gcggataaca atttcacaca ggaaacagct atgaccatga 6900
   ttacgccaag cgcgcaatta accctcacta aagggaacaa aagctggagc tgcaagctta 6960
   atgtagtctt atgcaatact cttgtagtct tgcaacatgg taacgatgag ttagcaacat 7020
   gccttacaag gagagaaaaa gcaccgtgca tgccgattgg tggaagtaag gtggtacgat 7080
   cgtgccttat taggaaggca acagacgggt ctgacatgga ttggacgaac cactgaattg 7140
   ccgcattgca gagatattgt atttaagtgc ctagctcgat acataaacgg gtctctctgg 7200
   ttagaccaga tctgagcctg ggagctctct ggctaactag ggaacccact gcttaagcct 7260
   caataaagct tgccttgagt gcttcaagta gtgtgtgccc gtctgttgtg tgactctggt 7320
   aactagagat ccctcagacc cttttagtca gtgtggaaaa tctctagcag tggcgcccga 7380
   acagggactt gaaagcgaaa gggaaaccag aggagctctc tcgacgcagg actcggcttg 7440
   ctgaagcgcg cacggcaaga ggcgaggggc ggcgactggt gagtacgcca aaaattttga 7500
   ctagcggagg ctagaaggag agagatgggt gcgagagcgt cagtattaag cgggggagaa 7560
   ttagatcgcg atgggaaaaa attcggttaa ggccaggggg aaagaaaaaa tataaattaa 7620
   aacatatagt atgggcaagc agggagctag aacgattcgc agttaatcct ggcctgttag 7680
   aaacatcaga aggctgtaga caaatactgg gacagctaca accatccctt cagacaggat 7740
   cagaagaact tagatcatta tataatacag tagcaaccct ctattgtgtg catcaaagga 7800
   tagagataaa agacaccaag gaagctttag acaagataga ggaagagcaa aacaaaagta 7860
   agaccaccgc acagcaagcg gccgctgatc ttcagacctg gaggaggaga tatgagggac 7920
   aattggagaa gtgaattata taaatataaa gtagtaaaaa ttgaaccatt aggagtagca 7980
   cccaccaagg caaagagaag agtggtgcag agagaaaaaa gagcagtggg aataggagct 8040
   ttgttccttg ggttcttggg agcagcagga agcactatgg gcgcagcgtc aatgacgctg 8100
   acggtacagg ccagacaatt attgtctggt atagtgcagc agcagaacaa tttgctgagg 8160
   gctattgagg cgcaacagca tctgttgcaa ctcacagtct ggggcatcaa gcagctccag 8220
   gcaagaatcc tggctgtgga aagataccta aaggatcaac agctcctggg gatttggggt 8280
   tgctctggaa aactcatttg caccactgct gtgccttgga atgctagttg gagtaataaa 8340
   tctctggaac agatttggaa tcacacgacc tggatggagt gggacagaga aattaacaat 8400
   tacacaagct taatacactc cttaattgaa gaatcgcaaa accagcaaga aaagaatgaa 8460
   caagaattat tggaattaga taaatgggca agtttgtgga attggtttaa cataacaaat 8520
   tggctgtggt atataaaatt attcataatg atagtaggag gcttggtagg tttaagaata 8580
   gtttttgctg tactttctat agtgaataga gttaggcagg gatattcacc attatcgttt 8640
   cagacccacc tcccaacccc gaggggaccc gacaggcccg aaggaataga agaagaaggt 8700
   ggagagagag acagagacag atccattcga ttagtgaacg gatctcgacg gtat 8754
<210> 26
   <211> 8730
   <212> DNA
   <213> Artificial
<220>
   <223> Synthetic lentiviral construct
<400> 26
   cgatgccgcc gttgacattg attattgact agttattaat agtaatcaat tacggggtca     60
   ttagttcata gcccatatat ggagttccgc gttacataac ttacggtaaa tggcccgcct    120
   ggctgaccgc ccaacgaccc ccgcccattg acgtcaataa tgacgtatgt tcccatagta    180
   acgccaatag ggactttcca ttgacgtcaa tgggtggagt atttacggta aactgcccac    240
   ttggcagtac atcaagtgta tcatatgcca agtacgcccc ctattgacgt caatgacggt    300
   aaatggcccg cctggcatta tgcccagtac atgaccttat gggactttcc tacttggcag    360
   tacatctacg tattagtcat cgctattacc atggtgatgc ggttttggca gtacatcaat    420
   gggcgtggat agcggtttga ctcacgggga tttccaagtc tccaccccat tgacgtcaat    480
   gggagtttgt tttggcacca aaatcaacgg gactttccaa aatgtcgtaa caactccgcc    540
   ccattgacgc aaatgggcgg taggcgtgta cggtgggagg tctatataag cagagctctc 600
   cctatcagtg atagagatct ccctatcagt gatagagatc gtcgacgagc tcgtttagtg 660
   aaccgtcaga tcgcctggag acgccatcca cgctgttttg acctccatag aagacaccgg 720
   gaccgatcca gcctccggcc gccgaattct gcagatatca acaagtttgt acaaaaaagc 780
   aggctccacc atgaccttct cggagatcct ggaccgtgtg ggaagcatgg gccatttcca 840
   gttcctgcat gtagccatac tgggcctccc gatcctcaac atggccaacc acaacctgct 900
   gcagatcttc acagccgcca cccctgtcca ccactgtcgc ccgccccaca atgcctccac 960
   agggccttgg gtgctcccca tgggcccaaa tgggaagcct gagaggtgcc tccgttttgt 1020
   acatccgccc aatgccagcc tgcccaatga cacccagagg gccatggagc catgcctgga 1080
   tggctgggtc tacaacagca ccaaggactc cattgtgaca gagtgggact tggtgtgcaa 1140
   ctccaacaaa ctgaaggaga tggcccagtc tatcttcatg gcaggtatac tgattggagg 1200
   gctcgtgctt ggagacctgt ctgacaggtt tggccgcagg cccatcctga cctgcagcta 1260
   cctgctgctg gcagccagcg gctccggtgc agccttcagc cccaccttcc ccatctacat 1320
   ggtcttccgc ttcctgtgtg gctttggcat ctcaggcatt accctgagca ccgtcatctt 1380
   gaatgtggaa tgggtgccta cccggatgcg ggccatcatg tcgacagcac tcgggtactg 1440
   ctacaccttt ggccagttca ttctgcccgg cctggcctac gccatccccc agtggcgttg 1500
   gctgcagtta actgtgtcca ttcccttctt cgtcttcttc ctatcatcct ggtggacacc 1560
   agagtccata cgctggttgg tcttgtctgg aaagtcctcg aaggccctga agatactccg 1620
   gcgggtggct gtcttcaatg gcaagaagga agagggagaa aggctcagct tggaggagct 1680
   caaactcaac ctgcagaagg agatctcctt ggccaaggcc aagtacaccg caagtgacct 1740
   gttccggata cccatgctgc gccgcatgac cttctgtctt tccctggcct ggtttgctac 1800
   cggttttgcc tactatagtt tggctatggg tgtggaagaa tttggagtca acctctacat 1860
   cctccagatc atctttggtg gggtcgatgt cccagccaag ttcatcacca tcctctcctt 1920
   aagctacctg ggccggcata ccactcaggc cgctgccctg ctcctggcag gaggggccat 1980
   cttggctctc acctttgtgc ccttggactt gcagaccgtg aggacagtat tggctgtgtt 2040
   tgggaaggga tgcctatcca gctccttcag ctgcctcttc ctctacacaa gtgaattata 2100
   ccccacagtc atcaggcaaa caggtatggg cgtaagtaac ctgtggaccc gcgtgggaag 2160
   catggtgtcc ccgctggtga aaatcacggg tgaggtacag cccttcatcc ccaatatcat 2220
   ctacgggatc accgccctcc tcgggggcag tgctgccctc ttcctgcctg agaccctgaa 2280
   tcagcccttg ccagagacta tcgaagacct ggaaaactgg tccctgcggg caaagaagcc 2340
   aaagcaggag ccagaggtgg aaaaggcctc ccagaggatc cctctacagc ctcacggacc 2400
   aggcctgggc tccagctgag acccagcttt cttgtacaaa gtggttgata tccagcacag 2460
   tggcggccgc tcgagtctag agggcccgcg gttcgaaggt aagcctatcc ctaaccctct   2520
   cctcggtctc gattctacgc gtaccggtta gtaatgagtt tggaattaat tctgtggaat   2580
   gtgtgtcagt tagggtgtgg aaagtcccca ggctccccag gcaggcagaa gtatgcaaag   2640
   catgcatctc aattagtcag caaccaggtg tggaaagtcc ccaggctccc cagcaggcag   2700
   aagtatgcaa agcatgcatc tcaattagtc agcaaccata gtcccgcccc taactccgcc   2760
   catcccgccc ctaactccgc ccagttccgc ccattctccg ccccatggct gactaatttt   2820
   ttttatttat gcagaggccg aggccgcctc tgcctctgag ctattccaga agtagtgagg   2880
   aggctttttt ggaggcctag gcttttgcaa aaagctcccc ctgttgacaa ttaatcatcg   2940
   gcatagtata tcggcatagt ataatacgac aaggtgagga actaaaccat ggccaagttg   3000
   accagtgccg ttccggtgct caccgcgcgc gacgtcgccg gagcggtcga gttctggacc   3060
   gaccggctcg ggttctcccg ggacttcgtg gaggacgact tcgccggtgt ggtccgggac   3120
   gacgtgaccc tgttcatcag cgcggtccag gaccaggtgg tgccggacaa caccctggcc   3180
   tgggtgtggg tgcgcggcct ggacgagctg tacgccgagt ggtcggaggt cgtgtccacg   3240
   aacttccggg acgcctccgg gccggccatg accgagatcg gcgagcagcc gtgggggcgg   3300
   gagttcgccc tgcgcgaccc ggccggcaac tgcgtgcact tcgtggccga ggagcaggac   3360
   tgacacgtgc tacgagattt aaatggtacc tttaagacca atgacttaca aggcagctgt   3420
   agatcttagc cactttttaa aagaaaaggg gggactggaa gggctaattc actcccaacg   3480
   aagacaagat ctgctttttg cttgtactgg gtctctctgg ttagaccaga tctgagcctg   3540
   ggagctctct ggctaactag ggaacccact gcttaagcct caataaagct tgccttgagt   3600
   gcttcaagta gtgtgtgccc gtctgttgtg tgactctggt aactagagat ccctcagacc   3660
   cttttagtca gtgtggaaaa tctctagcag tagtagttca tgtcatctta ttattcagta   3720
   tttataactt gcaaagaaat gaatatcaga gagtgagagg aacttgttta ttgcagctta   3780
   taatggttac aaataaagca atagcatcac aaatttcaca aataaagcat ttttttcact   3840
   gcattctagt tgtggtttgt ccaaactcat caatgtatct tatcatgtct ggctctagct   3900
   atcccgcccc taactccgcc catcccgccc ctaactccgc ccagttccgc ccattctccg   3960
   ccccatggct gactaatttt ttttatttat gcagaggccg aggccgcctc ggcctctgag   4020
   ctattccaga agtagtgagg aggctttttt ggaggcctag ggacgtaccc aattcgccct   4080
   atagtgagtc gtattacgcg cgctcactgg ccgtcgtttt acaacgtcgt gactgggaaa   4140
   accctggcgt tacccaactt aatcgccttg cagcacatcc ccctttcgcc agctggcgta   4200
   atagcgaaga ggcccgcacc gatcgccctt cccaacagtt gcgcagcctg aatggcgaat   4260
   gggacgcgcc ctgtagcggc gcattaagcg cggcgggtgt ggtggttacg cgcagcgtga   4320
   ccgctacact tgccagcgcc ctagcgcccg ctcctttcgc tttcttccct tcctttctcg 4380
   ccacgttcgc cggctttccc cgtcaagctc taaatcgggg gctcccttta gggttccgat 4440
   ttagtgcttt acggcacctc gaccccaaaa aacttgatta gggtgatggt tcacgtagtg 4500
   ggccatcgcc ctgatagacg gtttttcgcc ctttgacgtt ggagtccacg ttctttaata 4560
   gtggactctt gttccaaact ggaacaacac tcaaccctat ctcggtctat tcttttgatt 4620
   tataagggat tttgccgatt tcggcctatt ggttaaaaaa tgagctgatt taacaaaaat 4680
   ttaacgcgaa ttttaacaaa atattaacgc ttacaattta ggtggcactt ttcggggaaa 4740
   tgtgcgcgga acccctattt gtttattttt ctaaatacat tcaaatatgt atccgctcat 4800
   gagacaataa ccctgataaa tgcttcaata atattgaaaa aggaagagta tgagtattca 4860
   acatttccgt gtcgccctta ttcccttttt tgcggcattt tgccttcctg tttttgctca 4920
   cccagaaacg ctggtgaaag taaaagatgc tgaagatcag ttgggtgcac gagtgggtta 4980
   catcgaactg gatctcaaca gcggtaagat ccttgagagt tttcgccccg aagaacgttt 5040
   tccaatgatg agcactttta aagttctgct atgtggcgcg gtattatccc gtattgacgc 5100
   cgggcaagag caactcggtc gccgcataca ctattctcag aatgacttgg ttgagtactc 5160
   accagtcaca gaaaagcatc ttacggatgg catgacagta agagaattat gcagtgctgc 5220
   cataaccatg agtgataaca ctgcggccaa cttacttctg acaacgatcg gaggaccgaa 5280
   ggagctaacc gcttttttgc acaacatggg ggatcatgta actcgccttg atcgttggga 5340
   accggagctg aatgaagcca taccaaacga cgagcgtgac accacgatgc ctgtagcaat 5400
   ggcaacaacg ttgcgcaaac tattaactgg cgaactactt actctagctt cccggcaaca 5460
   attaatagac tggatggagg cggataaagt tgcaggacca cttctgcgct cggcccttcc 5520
   ggctggctgg tttattgctg ataaatctgg agccggtgag cgtgggtctc gcggtatcat 5580
   tgcagcactg gggccagatg gtaagccctc ccgtatcgta gttatctaca cgacggggag 5640
   tcaggcaact atggatgaac gaaatagaca gatcgctgag ataggtgcct cactgattaa 5700
   gcattggtaa ctgtcagacc aagtttactc atatatactt tagattgatt taaaacttca 5760
   tttttaattt aaaaggatct aggtgaagat cctttttgat aatctcatga ccaaaatccc 5820
   ttaacgtgag ttttcgttcc actgagcgtc agaccccgta gaaaagatca aaggatcttc 5880
   ttgagatcct ttttttctgc gcgtaatctg ctgcttgcaa acaaaaaaac caccgctacc 5940
   agcggtggtt tgtttgccgg atcaagagct accaactctt tttccgaagg taactggctt 6000
   cagcagagcg cagataccaa atactgttct tctagtgtag ccgtagttag gccaccactt 6060
   caagaactct gtagcaccgc ctacatacct cgctctgcta atcctgttac cagtggctgc 6120
   tgccagtggc gataagtcgt gtcttaccgg gttggactca agacgatagt taccggataa 6180
   ggcgcagcgg tcgggctgaa cggggggttc gtgcacacag cccagcttgg agcgaacgac 6240
   ctacaccgaa ctgagatacc tacagcgtga gctatgagaa agcgccacgc ttcccgaagg 6300
   gagaaaggcg gacaggtatc cggtaagcgg cagggtcgga acaggagagc gcacgaggga 6360
   gcttccaggg ggaaacgcct ggtatcttta tagtcctgtc gggtttcgcc acctctgact 6420
   tgagcgtcga tttttgtgat gctcgtcagg ggggcggagc ctatggaaaa acgccagcaa 6480
   cgcggccttt ttacggttcc tggccttttg ctggcctttt gctcacatgt tctttcctgc 6540
   gttatcccct gattctgtgg ataaccgtat taccgccttt gagtgagctg ataccgctcg 6600
   ccgcagccga acgaccgagc gcagcgagtc agtgagcgag gaagcggaag agcgcccaat 6660
   acgcaaaccg cctctccccg cgcgttggcc gattcattaa tgcagctggc acgacaggtt 6720
   tcccgactgg aaagcgggca gtgagcgcaa cgcaattaat gtgagttagc tcactcatta 6780
   ggcaccccag gctttacact ttatgcttcc ggctcgtatg ttgtgtggaa ttgtgagcgg 6840
   ataacaattt cacacaggaa acagctatga ccatgattac gccaagcgcg caattaaccc 6900
   tcactaaagg gaacaaaagc tggagctgca agcttaatgt agtcttatgc aatactcttg 6960
   tagtcttgca acatggtaac gatgagttag caacatgcct tacaaggaga gaaaaagcac 7020
   cgtgcatgcc gattggtgga agtaaggtgg tacgatcgtg ccttattagg aaggcaacag 7080
   acgggtctga catggattgg acgaaccact gaattgccgc attgcagaga tattgtattt 7140
   aagtgcctag ctcgatacat aaacgggtct ctctggttag accagatctg agcctgggag 7200
   ctctctggct aactagggaa cccactgctt aagcctcaat aaagcttgcc ttgagtgctt 7260
   caagtagtgt gtgcccgtct gttgtgtgac tctggtaact agagatccct cagacccttt 7320
   tagtcagtgt ggaaaatctc tagcagtggc gcccgaacag ggacttgaaa gcgaaaggga 7380
   aaccagagga gctctctcga cgcaggactc ggcttgctga agcgcgcacg gcaagaggcg 7440
   aggggcggcg actggtgagt acgccaaaaa ttttgactag cggaggctag aaggagagag 7500
   atgggtgcga gagcgtcagt attaagcggg ggagaattag atcgcgatgg gaaaaaattc 7560
   ggttaaggcc agggggaaag aaaaaatata aattaaaaca tatagtatgg gcaagcaggg 7620
   agctagaacg attcgcagtt aatcctggcc tgttagaaac atcagaaggc tgtagacaaa 7680
   tactgggaca gctacaacca tcccttcaga caggatcaga agaacttaga tcattatata 7740
   atacagtagc aaccctctat tgtgtgcatc aaaggataga gataaaagac accaaggaag 7800
   ctttagacaa gatagaggaa gagcaaaaca aaagtaagac caccgcacag caagcggccg 7860
   ctgatcttca gacctggagg aggagatatg agggacaatt ggagaagtga attatataaa 7920
   tataaagtag taaaaattga accattagga gtagcaccca ccaaggcaaa gagaagagtg 7980
   gtgcagagag aaaaaagagc agtgggaata ggagctttgt tccttgggtt cttgggagca 8040
   gcaggaagca ctatgggcgc agcgtcaatg acgctgacgg tacaggccag acaattattg 8100
   tctggtatag tgcagcagca gaacaatttg ctgagggcta ttgaggcgca acagcatctg   8160
   ttgcaactca cagtctgggg catcaagcag ctccaggcaa gaatcctggc tgtggaaaga   8220
   tacctaaagg atcaacagct cctggggatt tggggttgct ctggaaaact catttgcacc   8280
   actgctgtgc cttggaatgc tagttggagt aataaatctc tggaacagat ttggaatcac   8340
   acgacctgga tggagtggga cagagaaatt aacaattaca caagcttaat acactcctta   8400
   attgaagaat cgcaaaacca gcaagaaaag aatgaacaag aattattgga attagataaa   8460
   tgggcaagtt tgtggaattg gtttaacata acaaattggc tgtggtatat aaaattattc   8520
   ataatgatag taggaggctt ggtaggttta agaatagttt ttgctgtact ttctatagtg   8580
   aatagagtta ggcagggata ttcaccatta tcgtttcaga cccacctccc aaccccgagg   8640
   ggacccgaca ggcccgaagg aatagaagaa gaaggtggag agagagacag agacagatcc   8700
   attcgattag tgaacggatc tcgacggtat                                    8730
<210> 27
   <211> 38
   <212> DNA
   <213> Artificial
<220>
   <223> Synthetic primer
<400> 27
   gccgccatcg atgccgccgt tgacattgat tattgact 38
<210> 28
   <211> 39
   <212> DNA
   <213> Artificial
<220>
   <223> Synthetic primer
<400> 28
   ggcggcgaat tcggcggccg gaggctggat cggtcccgg 39

## Description

### FIELD OF THE INVENTION

The invention relates to the field of pharmacology, specifically the field of drug-drug interactions and nephrotoxicity. An engineered, stable cell line of human renal cells is provided that allows screening for drug-drug interactions and nephrotoxicity.

### BACKGROUND OF THE INVENTION

The renal proximal tubules play a major role in eliminating waste products from the body. Such waste products include drugs and their metabolites. The active secretion and reabsorption mechanisms of the renal proximal tubules, together with their biotransformation capacity, makes the proximal tubule cells exceptionally sensitive to drug-induced toxicity and to subsequent acute kidney injury (AKI) (Tiong et al., 2014). The process of renal drug elimination may be further affected by concomitant treatment with other drugs, that is: by treatment with more than one drug at the same time, which can lead to clinically relevant drug-drug interactions (DDI). In the context of DDI, drugs are known to sometimes be withdrawn from the market, denied regulatory approval, or discontinued from clinical development because of their victim potential (which means they are the object of a drug-drug interaction) or their perpetrator potential (which means they are the precipitating cause of the drug interaction). Regardless of a drug being a perpetrator or a victim, nephrotoxicity of a compound as a result of DDI is a significant cause of drug candidate attrition during pharmaceutical development, because it is often recognized only during the clinical stages of development: the translation from in vitro and animal studies to human studies lacks sufficient predictivity (Redfern et al., 2010; Guengerich, 2011).
The renal elimination mechanism of xenobiotics can roughly be divided into two major pathways: the organic anion system and the organic cation system. As a first step in elimination of organic anions in humans, active tubular uptake is mediated by two transport polypeptides: organic anion transporter 1 (OAT1), which is also known as "solute carrier family 22 member 6" (SLC22A6), and organic anion transporter 3 (OAT3), which is also known as "solute carrier family 22 member 8" (SLC22A8). These transporter polypeptides are present at the basolateral side, which is the blood-facing side (Wang & Sweet, 2013). As transporter polypeptides, OAT1 and OAT3 are characterized by their high affinity and capacity, and as a consequence they are major players in the potential development of drug-induced nephrotoxicity (Burckhardt & Burckhardt, 2011). After uptake of anionic compounds, their secretion into the tubular lumen is facilitated by apically expressed efflux transporters, such as multidrug resistance proteins 2 and 4 (MRP2 and MRP4; also known as ABCC2 and ABCC4, respectively) and breast cancer resistance protein (BCRP; ABCG2) (Masereeuw & Russel, 2010). In parallel, the renal elimination of organic cations in the human proximal tubular epithelium is facilitated by basolateral uptake, predominantly via the organic cation transporter 2 (OCT2), also known as "solute carrier family 22 member 2" (SLC22A2), and subsequent apical efflux via multidrug and toxin extrusion proteins 1 and 2-K (MATE1 and MATE2-K; also known as SLC47A1 and SLC47A2)(Motohashi & Inui, 2013) and P-glycoprotein (P-gp, also known as ABCB1)(Konig et al., 2013).
The polyspecific nature of many drug transporters implies that a wide range of substrates can be accepted, which contributes to the relatively high sensitivity to potential toxicity observed in the tissue that expresses said transporters. This sensitivity is especially apparent for organic anions, as this class comprises the majority of drugs that are excreted by the kidneys. Drug-induced nephrotoxicity related to the proximal tubular epithelium caused by this class of compounds has been described broadly, including descriptions for the acyclic nucleotide phosphonates adefovir, cidofovir and tenofovir (Izzedine et al., 2009; Lewis et al., 2003). These antiretroviral compounds are used, amongst other things, for the treatment of HIV, hepatitis B and cytomegalovirus infections, and the compounds function as nucleotide analog reverse transcriptase inhibitors (NtRTIs)(De Clerq, 2004). The exact mechanism of antiviral-induced renal toxicity is still under debate (Tourret et al., 2013), but the involvement of OATs in the uptake of many antivirals has been widely acknowledged (Kohler et al., 2011; Takeda et al., 2002; Ciglar et al., 1999). To prevent NtRTI-induced nephrotoxicity, their uptake can be inhibited by co-administration of an OAT1 inhibitor, such as probenecid (Lacy et al., 1998). As with many other diseases, current antiviral therapy, for example in HIV infections, is often based on polypharmacy. Increased plasma concentrations and systemic toxicity have been observed for didanosine co-administration with tenofovir in anti-HIV triple therapy, possibly by DDI at the site of OAT1 where that DDI limited renal excretion (Kearney et al., 2004). Together, polypharmacy can optimise the life-span of infected patients, but this strategy simultaneously increases the risk for DDIs and it demands personalized evaluation of the benefit/risk ratio for each drug (Vigouroux et al., 2014).
As described above, there is a need for an improved method to predict drug-drug interactions (DDI) or to predict nephrotoxicity. A model with sufficient predictive value for drug-induced nephrotoxicity should closely reflect the in vivo processes involved in renal drug handling. Such a model should probably be an in vitro model, and is highly desirable. A more specific description of such a highly desirable in vitro model is a cell-based model, which should comprise a proximal tubule epithelium that stably expresses a broad range of functional transporters such as OAT1 or OAT3, and metabolic enzymes, because transporters and enzymes act in concert in renal drug elimination *in vivo* (Gundert-Remy et al., 2014). Methods that do not take all of these factors into account might lack predictive value. In pharmacology and toxicology, the availability of a cell model of human origin expressing a broad range of functional transporters is of paramount importance. As such, the need for an improved method for predicting DDI or nephrotoxicity could be seen as the need for a suitable cell-based model that stably expresses functional transporters.

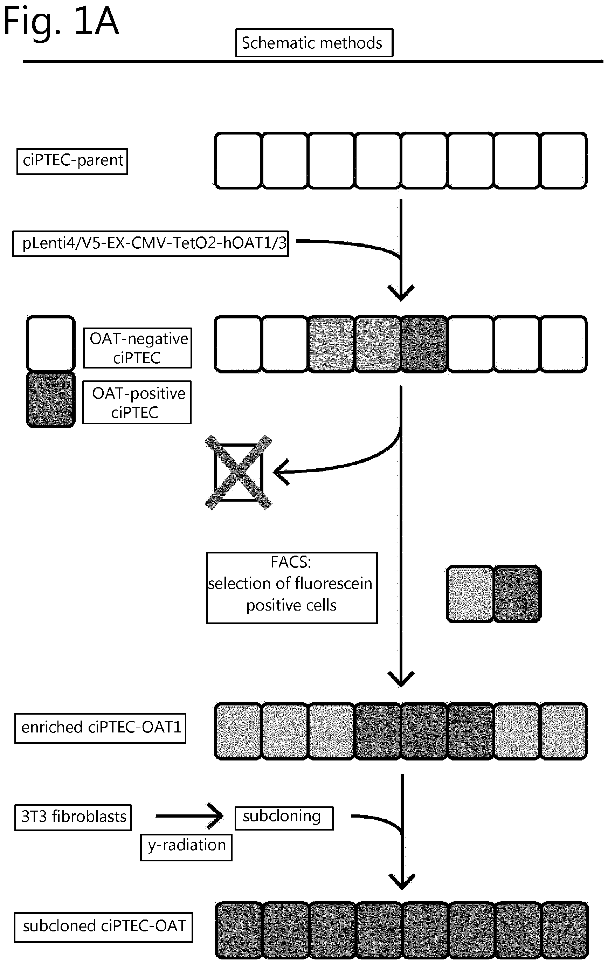

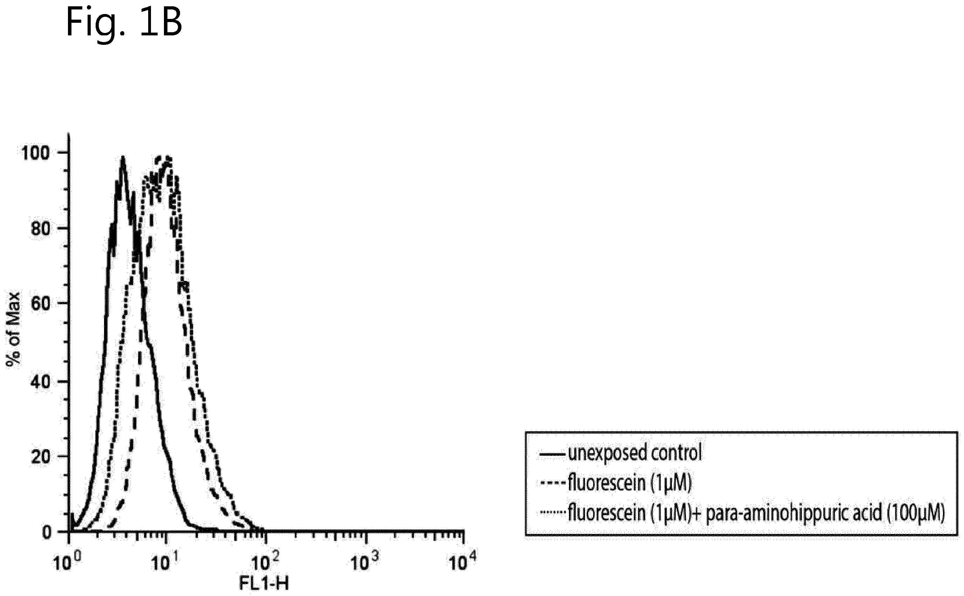

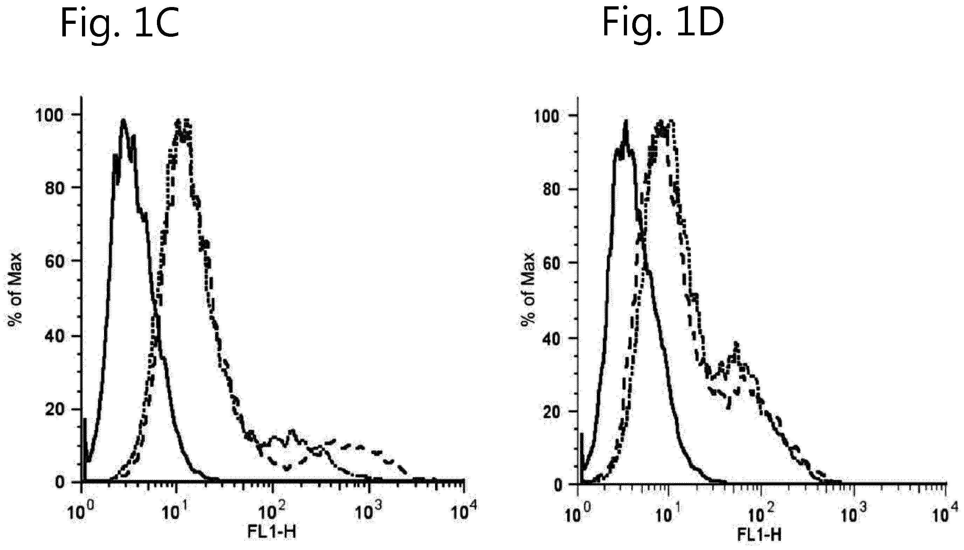

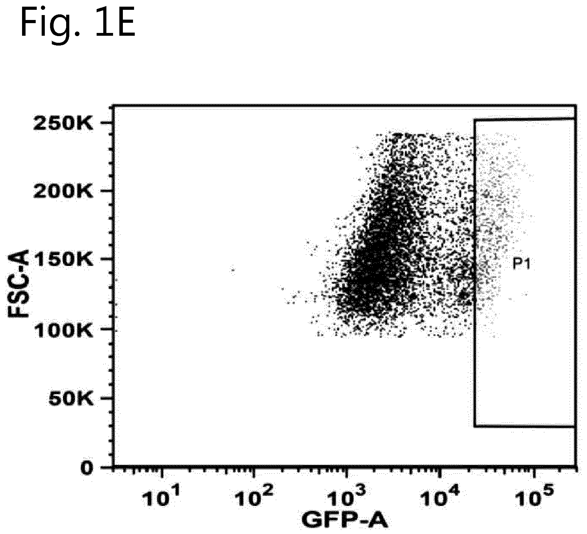

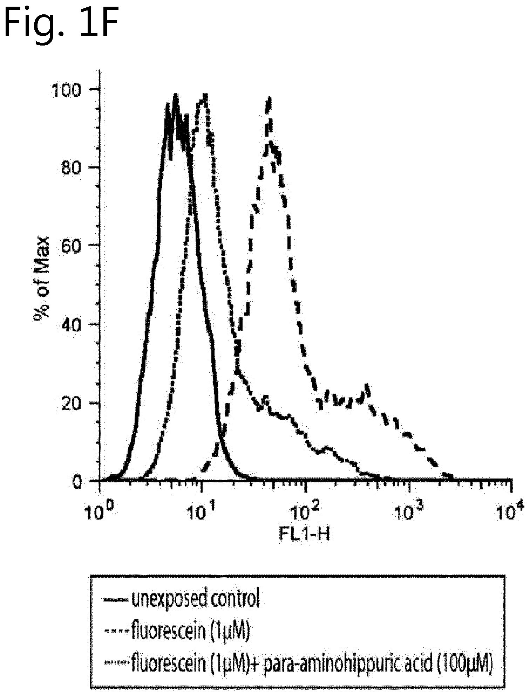

A major problem that is known for primary renal cell cultures that are often used in uptake studies, is that they quickly go into senescence (Ahlin et al., 2009). Conditionally immortalized proximal tubule epithelial cells (ciPTEC) are known as a preclinical in vitro prediction model that does not suffer from this problem (Wilmer et al., 2010). This model has been validated in the past to be highly predictive for studying DDI at the site of organic cation transporter 2 (OCT2) (Schophuizen et al., 2013), and it was shown to endogenously exhibit metabolic enzymes (Mutsaers et al., 2013) together with a panel of functional efflux transporters such as p-glycoprotein (Wilmer et al., 2010; Jansen et al., 2014). However, the expression of other transporters such as OAT1 and OAT3 is rapidly lost in culture (Jansen et al., 2014). OAT1 and OAT3 are important influx transporters in proximal tubular cells and determinants in the excretion of a variety of organic anions, including waste products from normal metabolism and drugs. Unfortunately, these transporters are absent on gene, protein and functional levels in ciPTEC (Figure 1). Although the expression of OATs has been observed in primary proximal tubular cells (Brown et al., 2008), the levels decrease dramatically during the first days of culturing and are lost after cell passaging. This phenomenon has already been described in 1990 by Miller (Miller, 1990) and has, as of yet, not been solved.
Abstract book of "(Epi)genetics in pharmacology") (page 7) describes the use of ciPTEC-OAT1 cells cultured on HFM as a first step to develop bioartifical kidneys. The abstract on page 13 describes ciPTEC cells overexpressing OAT1 or OAT3. The Abstract book of "NfN Fall symposium 2014" contains one abstract disclosing the use of ciPTEC cells transfected with OAT1 and their use in the prediction of drug-induced tubular toxicity. WO2015/069192 describes a method for differentiation of iPSC into "PTC-like" cells. Said PTC-like cells express AQP1, PEPTI, OAT3 and GLUT1 after 8 days in culture. EP2496687 describes ciPTEC expressing the organic anion transporter MRP4/ABCC4 in combination with expression and activity of OCT2 and Pgp. Stable expression of OAT1 and/or OAT3 has not been demonstrated.
Stable expression of these OATs in renal cell lines is not only of importance for studying regenerative nephrology, but is also of great value for drug development in pharmaceutical industry. The co-expression of functional drug transporters at both the apical and basolateral site is required for the further development of improved transcellular transport assays. Such assays would increase our understanding of renal excretion mechanisms, superior to the study of single transporters in the prior art expression models. The fact that for various transporters no stable expression is known in a valid model system, is a major hurdle. For this reason, there is a need for an improved method for predicting DDI or nephrotoxicity, preferably a suitable cell-based model that stably expresses a functional OAT1 or OAT 3 and further functional transporters.

### SUMMARY OF THE INVENTION

In an aspect, the present disclosure provides for a human proximal tubule epithelial cell (PTEC) that stably expresses a functional organic anion transporter (OAT) when cultured. In an embodiment of this aspect, said cell is conditionally immortalized (ciPTEC), preferably the cell is derived from ciPTEC DSM ACC 3019 or is derived from a passage or isolate thereof. In a further embodiment the cell comprises an organic anion transporter selected from the group consisting of OAT1 and OAT3. In a further embodiment, the cell further expresses at least one other relevant transporter, preferably a renal transporter, more preferably a renal transporter selected from the group consisting of SLC22A2 (OCT2), SLCO4C1 (OATP-H), ABCB1 (PgP), ABCG2 (BCRP), ABCC2 (MRP2), ABCC4 (MRP4), SLC47A1 (MATE1), SLC47A2 (MATE2-K), SLC34A1 (NaPi IIa), and SLC34A3 (NaPi IIc), or a multitude thereof. The cells of the invention are ciPTEC.OAT1.4B2 DSM ACC3279 or a passage or isolate thereof and ciPTEC.OAT3.3C1 DSM ACC3280 or passage or isolate thereof. Cells of this aspect provide a useful cell-based model that can stably expresses a functional OAT1 or OAT 3 and further functional transporters, with relevant polarization.

In a second aspect, the present invention provides for a method for the production of a human proximal tubule epithelial cell that stably expresses an organic anion transporter when cultured. In general, this method is for the production of cells of the first aspect of the invention. The method comprises transducing a population of proximal tubule epithelial cells by a lentiviral particle comprising an expression construct that comprises a nucleotide sequence having at least 50% sequence identity with SEQ ID NO: 2 or with SEQ ID NO: 4, optionally enriching the transduced population obtained in (i), preferably by using fluorescence activated cell sorting (FACS), and iii) subcloning the transduced population obtained in (i) of (ii) by selecting and isolating single cells and expanding these by culture.

In a third aspect, a method for analysis of a substance is provided. Said method can be *in vitro* or *ex vivo* and comprises contacting said substance with at least one cell according to the first aspect, preferably with a mature monolayer of said cells. In an embodiment of this aspect, the method is for determining the nephrotoxicity of said substance, and preferably further comprises the subsequent analysis of cell viability, preferably by analysis of cellular dehydrogenase capacity. In a further embodiment, the method is for the functional analysis of the interaction of said substance with a transporter, preferably a renal transporter, and wherein said contacting preferably is in the presence of a labeled anionic transporter substrate, preferably a radiolabeled or a fluorescently labeled anionic transporter substrate. In a further embodiment, the method further comprises determining the drug-drug interaction of said substance. In a further embodiment, the method further comprises determining whether said substance is a substrate or an inhibitor of a transporter involved in a clinically relevant drug-drug interaction. The method of this aspect provides relevant, useful, and reliable results due to the relevant expression of functional transporters by the cell of the invention, which is used in this method. Results provided by methods that use other cells can show greater variation with real clinical outcomes.

In a fourth aspect, a kit of parts is provided that comprises a cell according to the invention as defined in claim 1, and instructions for use.

### DETAILED DESCRIPTION OF THE INVENTION

The present invention relates to a human cell model that allows prediction of drug-induced nephrotoxicity and DDI of organic anions. Transporters OAT1 and OAT3 were expressed in ciPTEC by transduction followed by a selection procedure. Surprisingly, the function of both transporters (OAT1 and OAT3) was found to be stable upon prolonged culturing of the cells. This characteristic of the invention allows screening for DDI using known pharmacological substrates for and/or inhibitors of OAT1 and/or OAT3. The invention also relates to the use of said cells. Said use shows that OAT-mediated uptake in ciPTEC is a key determinant in antiviral-induced cytotoxicity. This underscores that ciPTEC-OAT1 and ciPTEC-OAT3 are valuable tools for drug-induced toxicity screening.
To improve prediction of the nephrotoxic potential of novel chemical entities and to mechanistically understand the pathways associated with drug-induced toxicity, highly predictive and validated translational models are required. In the present disclosure, such a robust human-based cell model with intact proximal tubular characteristics is described. Stable OAT1 and OAT3 expression in the human renal cell line ciPTEC allows studying reproducible drug-drug interactions (DDI) for a panel of model substrates and antiviral compounds. As illustrations of this, functional OAT1 and OAT3 transport activity was demonstrated to be associated with drug-induced toxicity of the antivirals adefovir, cidofovir and tenofovir. These findings indicate that the presently disclosed model can predict drug-induced nephrotoxicity, and the findings underscore that functional expression of influx transporters is pivotal in the prediction of drug-induced renal toxicity.
Many reports related to studying drug-OAT interactions describe the use of non-polarized overexpression systems, such as Chinese hamster ovary (CHO) cells, the human cervical epitheloid carcinoma cell line HeLa, or human epithelial kidney (HEK) 293 cells, which are highly relevant for studying interactions at the single transporter level but which have a poor overall predictivity due to their simplicity (Cihlar & Ho, 2000; Mandikova et al., 2013). Since proximal tubule cells are the main site of adverse drug effects in the kidney, this cell type would be preferred for in vitro assays investigating drug-induced nephrotoxicity (Tiong et al., 2014). Human primary proximal tubule cells reflect in vivo toxicological responses best, but lack reproducibility and robustness due to high donor-to-donor variability and limited availability. Moreover, primary cells lose their proximal tubular phenotype upon culturing, and OAT1-4, P-glycoprotein and MRP expressions were found to be rapidly decreased (Lash et al., 2006; Brown et al., 2008). A down-regulation in OATs' expression upon culturing can be part of a survival mechanism. This problem hampers development of good model cell lines. To extend the life span of human proximal tubular cells and to provide a robust model for drug screening, immortalized primary kidney cells have been developed, yet none have demonstrated functional OATs despite retained gene expressions (Wilmer et al., 2010; Wieser et al., 2008; Aschauer et al., 2014).
The current disclosure demonstrates a human model with stable expression and functionality of OAT1 and OAT3 for up to 29 passages. This was analyzed by fluorescein uptake. Experimental values obtained for DDI of model compounds correlated well with published data, confirming PAH has a higher inhibitory potency for OAT1 compared to OAT3, whereas the inhibitory potencies of estrone sulfate, probenecid and furosemide were clearly higher for OAT3. ciPTEC-OAT3 inhibition by cimetidine was found well within predetermined ranges (Khamdang et al., 20014; the IC₅₀ value of cimetidine in ciPTEC-OAT1 is about 5 fold higher as described earlier, which may be explained by the fact that the earlier study used different substrates: the OAT1-substrate PAH which was used in the earlier study has a lower affinity for OAT1 when compared to fluorescein, which is used in the present disclosure.) The effects of prototypic inhibitor compounds on drug transport are promising with respect to the application of ciPTEC as a tool to study drug-induced nephrotoxicity. The concept was validated with a selected a panel of clinically relevant antivirals with various pharmacokinetic parameters.

DDIs are a major concern in for example anti-HIV therapy, which often includes co-administration of multiple antivirals. Adefovir, cidofovir, tenofovir and zidovudine DDI were evaluated at the site of OAT1 and OAT3. The affinities of adefovir, cidofovir and tenofovir were higher for OAT1 than for OAT3, in agreement with previous studies in CHO cells overexpressing hOAT1 and hOAT3 (Cihlar et al., 2009). The Drug-drug-interaction (DDI) index is a concept that has been used to determine the potential of clinical DDIs and drug-induced toxicities (Huang et al., 2012; Wang & Sweet, 2012). It allows extrapolating in vitro observations to clinical settings (Kimura et al., 2005; Cihlar et al., 2009). In the present disclosure, IC₅₀ values of less than 10 times the maximal free plasma concentration (C_{max,u}/IC₅₀>0.1), were found for adefovir, cidofovir and zidovudine, indicating these antivirals are likely to inhibit OAT1 and OAT3 at clinically relevant concentrations.

The clinical relevance and impact on drug safety of OAT transporters are well acknowledged by regulatory authorities and the pharmaceutical industry (Nigam, 2015). Both the FDA and the European Medicines Agency (EMA) have issued guidance documents, outlining that OAT interactions should be studied for new compounds (European Medicines Agency, 2010; Huang & Zhang, 2012). Furthermore, the International Transporter Consortium (ITC) provided decision trees to determine whether a drug candidate may be a substrate (victim) or an inhibitor (perpetrator) of transporters involved in clinically relevant DDI (International Transporter Consortium, doi: 10.1038/nrd3028, 2010). Consequently, pharmaceutical industry started a quest for reliable and high-throughput in vitro models that mimic the human kidney with improved prediction of drug-induced nephrotoxicity and a decrease in use of animals in research (McGuinness, 2014). Current preclinical tests for prediction of nephrotoxicity are mainly based on animal (rodent) models. These models provide information about systemic toxicity in living organisms, but they bear high costs, are time intensive and remain an ethical issue. Their clinical predictive value is limited due to inherent interspecies differences in drug disposition and their use emphasizes the urgent need for human based models that closely resemble the human kidney physiology (Chu et al., 2013; Burckhardt & Burckhardt, 2011). In vitro models in combination with high-throughput automated systems for toxicity read-outs can become major steps forward in drug safety screening, for which the ciPTEC model provides a suitable cellular basis (Bhatia & Ingber, 2014).

This disclosure reports the first human PTEC model with stable expression and functionality of OAT1 and OAT3, allowing screening for drug-induced nephrotoxicity and DDI. ciPTEC-OAT1 and ciPTEC-OAT3 are valuable tools for drug-induced toxicity screening that can improve translation of in vitro findings to clinical research and which can decrease the use of animal studies in the preclinical stages of drug development. The PTEC model according to the invention is not only applicable for OAT compounds, but also for OCT2, Pgp, MRP4 and BCRP, which are simultaneously expressed with OAT1 or OAT3. These further transporters are not functional in other OAT over-expressing models known in the art (Schophuizen et al., 2013; Mutsaers et al., 2013; Wilmer et al., 2010; Jansen et al., 2014; Jansen et al., 2014; Brown et al., 2008; Miller, 1990), making the model of the invention unique and broadly applicable with improved *in vitro* to *in vivo* prediction.

### Cell

In a first aspect, the invention provides for a human proximal tubule epithelial cell (PTEC) that stably expresses a functional organic anion transporter (OAT) when cultured, wherein the cell is the one as defined in the claims. Proximal tubule epithelial cells (PTECs) are cells that, in healthy subjects, are comprised in the renal proximal tubule, where they are in contact with blood and with the tubular lumen. PTECs grow in an anisotropic or polarised manner, exhibiting a basolateral side and and apical side. Said basolateral side is the blood-facing side, where PTECs anisotropically express uptake transporters, which will be later defined herein. Said apical side faces the tubular lumen, and comprises apically expressed efflux transporters, such as multidrug resistance proteins, which will be later defined herein.
In this context, the culturing of cells is the growing and/or the maintaining of said cells under conducive circumstances, with periodic passage of the cells. Cell passage can involve culling the population of cells, and refreshing the medium that covers the cells, and detaching the cells from any substrate, sometimes to move said cells to a new or different substrate. Stable expression (or stably expressing, used herein interchangeably) is the expression of a polypeptide that is not transient, or that is not lost during culturing. In this context, stable expression of a polypeptide is expression that leads to the detectable presence or activity of said polypeptide for at least ten passages. In preferred embodiments, stable expression is considered to be expression of a polypeptide that leads to the detectable presence or activity of said polypeptide for at least 11, 12, 13, 14, 15, 16, 17, 18, 19, 20, 21, 22, 23, 24, 25, 26, 27, 28, 29, 30, or more passages. A functional polypeptide is a polypeptide that can be detected to perform the function that it has in the physiology of a cell. Alternately, a functional polypeptide is a polypeptide that has can be detected to perform its desired function when such a function is known in the art. In preferred embodiments of this aspect, a functional polypeptide exhibits at least 1% of its usual presence or activity, where the usual presence or activity is known to a person skilled in the art, or can be assessed by a person skilled in the art. In more preferred embodiments, a functional polypeptide exhibits at least 5%, 10%, 15%, 20%, 25%, 30%, 35%, 40%, 45%, 50%, 55%, 60%, 65%, 70%, 75%, 80%, 85%, 90%, 95%, or more of its presence or activity.
An organic anion transporter (OAT) is a polypeptide that functions as a transporter. In this context, a transporter is a polypeptide that can also be referred to as a transmembrane pump, and it serves the function of moving substances in relation to a cell, preferably from outside the cell to inside the cell. OATs are generally present at the basolateral membrane of PTEC. OATs generally transport organic anions. Examples of OATs are organic anion transporter 1 (OAT1), which is also known as "solute carrier family 22 member 6" (SLC22A6), and organic anion transporter 3 (OAT3), which is also known as "solute carrier family 22 member 8" (SLC22A8). The transporter polypeptides OAT1 and OAT3 are characterized by their high affinity and capacity. A further OAT is OAT4. Transporter polypeptides generally perform their function in concert with further transporter polypeptides. For instance, a substance can be imported by one transporter, and subsequently exported by another substance. It follows that to assess a transporter, or to assess a substance and its interaction with transporters, model systems have to be used that do not merely express one or just a few transporters. In other words, models should not entail overexpression of a transporter in an otherwise 'empty' model. The advantage of a human proximal tubular background for PTEC is that it expresses other relevant transporters such as OCT2, MRP4, BCRP etc. It is to be understood that expression of a functional OAT is to be construed as the expression of OAT in such a manner that the expressing cell exhibits increased uptake of ingestion of organic anions, or that transport of organic anions from outside the cell to inside the cell is increased compared to a cell that does not express functional OAT. It is also to be understood that stable expression of OAT is to be construed as the lasting expression of OAT by cells, in the sense that said expression is still detectable after multiple passages.

In an embodiment, the human proximal tubule epithelial cell (PTEC) that stably expresses a functional organic anion transporter (OAT) when cultured is conditionally immortalized (ciPTEC), preferably said cell is derived from ciPTEC DSM ACC 3019 or is derived from a passage or isolate thereof. ciPTEC DSM ACC 3019 (deposited at DSMZ - German Collection of Microorganisms and Cell Cultures; Inhoffenstrasse 7 B; 38124 Braunschweig; Germany) is extensively described in EP2496687B1. Conditional immortalization is a technique that delays or avoids the effects of limited proliferation capacities when cultured in a specific condition, in the present case culturing at 33° C. To overcome the limited availability of PTEC, which stems from its rapid senescence and loss of functionality in culturing, immortalization steps can be applied (Wilmer et al. 2005). Infection by using both the temperature-sensitive mutant U19tsA58 of SV40 large T antigen (SV40T) and the essential catalytic subunit of human telomerase (hTERT) is known to be effective for the development of conditionally immortalized cells (O'Hare et al. 2001; Saleem et al. 2002; Satchell et al. 2006). Transfection with SV40T allows cells to proliferate at a permissive low temperature of 33° C, hence immortalization at 33° C, whereas the inactivation of the large T antigen at 37° C causes changes in gene expression (Stamps et al. 1994). The hTERT vector expresses telomerase activity to maintain telomere length, preventing the occurrence of replicative senescence (Bodnar et al. 1998). Using a non-invasive technique of obtaining renal material from urine, a conditionally immortalized human PTEC (ciPTEC) can be generated. Such cell line can be maintained for at least 45 passages and presents proximal tubule characteristics. In ciPTEC, amongst other things the uptake of albumin and phosphate and the activities of the ATP-binding cassette (ABC) transporter P-glycoprotein (Pgp/MDR1/ABCB1) and organic cation transporter 2 (OCT2, SLC22A2) are intact. A preferred ciPTEC is derived from ciPTEC DSM ACC 3019 or derived from a passage or isolate thereof (EP2496687B1).

In an embodiment, in the human PTEC or in the human ciPTEC according to the disclosure, stably expressing a functional OAT when cultured, said OAT is selected from the group consisting of:
i) a polypeptide having at least 50%, 55%, 60%, 65%, 70%, 75%, 80%, 85%, 90%, 91%, 92%, 93%, 94%, 95%, 96%, 97%, 98%, 99% or most preferably 100% sequence identity or similarity with SEQ ID NO: 1 (organic anion transporter 1 (OAT1)), or encoded by a nucleotide sequence having at least 90% sequence identity with SEQ ID NO: 2,and
ii) a polypeptide having at least 50%, 55%, 60%, 65%, 70%, 75%, 80%, 85%, 90%, 91%, 92%, 93%, 94%, 95%, 96%, 97%, 98%, 99% or most preferably 100% sequence identity or similarity with SEQ ID NO: 3 (organic anion transporter 3 (OAT3)), or encoded by a nucleotide sequence having at least 50%, 55%, 60%, 65%, 70%, 75%, 80%, 85%, 90%, 91%, 92%, 93%, 94%, 95%, 96%, 97%, 98%, 99% or most preferably 100% sequence identity with SEQ ID NO: 4.
OAT1 is described above, and SEQ ID NO: 1 represents the amino acid sequence of naturally occurring OAT1. SEQ ID NO: 2 represents a nucleic acid sequence that encodes the polypeptide represented by SEQ ID NO: 1. OAT3 is described above, and SEQ ID NO: 3 represents the amino acid sequence of naturally occurring OAT3. SEQ ID NO:4 represents a nucleic acid sequence that encodes the polypeptide represented by SEQ ID NO: 3
In an embodiment, the human PTEC or the human ciPTEC according to the disclosure that stably expresses a functional OAT when cultured, further expresses at least one other relevant transporter, preferably a renal transporter, more preferably a renal transporter selected from the group consisting of SLC22A2 (OCT2), SLCO4C1 (OATP-H), ABCB1 (PgP), ABCG2 (BCRP), ABCC2 (MRP2), ABCC4 (MRP4), SLC47A1 (MATE1), SLC47A2 (MATE2-K), SLC34A1 (NaPi IIa), and SLC34A3 (NaPi IIc). A preferred renal transporter is a drug transporter such as an influx transporter selected from the group consisting of OCT2 and OATP-H and such as an efflux transporter selected from the group consisting of Pgp, BCRP, MRP2, MRP4, MATE1 and MATE2-k. Another preferred renal transporter is an ion transporter selected from the group consisting of NaPi IIa and NaP iIIc.
Preferably at least one, two, three or four drug transporters are expressed in the human PTEC or the human ciPTEC according to the invention and at least one, two or three ion transporters are expressed in the human PTEC or the human ciPTEC according to the disclosure.
A renal transporter is a transporter that is relevant for the physiology of a kidney. A renal transporter is a transporter that is expressed and/or active in the kidney, and that contributes to what is known in the art as renal function. Examples of such function is the transport of a substance from blood to the inside of a renal cell, or the transport of a substance from inside a renal cell to the tubular lumen. Preferred renal transporters are Organic Cation Transporter 2 (also known as SLC22A2 or OCT2, the polypeptide of which is preferably represented by SEQ ID NO: 5, which in turn is preferably encoded by the nucleotide represented by SEQ ID NO: 6), Organic Anion Transporter H (also known as SLCO4C1 or OATP-H, the polypeptide of which is preferably represented by SEQ ID NO:7, which in turn is preferably encoded by the nucleotide represented by SEQ ID NO: 8), P-glycoprotein (also known as ABCB1 or PgP , the polypeptide of which is preferably represented by SEQ ID NO: 9, which in turn is preferably encoded by the nucleotide represented by SEQ ID NO: 10), ATP-binding cassette sub-family G member 2 (also known as ABCG2, or BCRP or CDw338, the polypeptide of which is preferably represented by SEQ ID NO: 11, which in turn is preferably encoded by the nucleotide represented by SEQ ID NO: 12), Multidrug resistance protein 2 (also known as ABCC2, or MRP2, the polypeptide of which is preferably represented by SEQ ID NO: 13, which in turn is preferably encoded by the nucleotide represented by SEQ ID NO: 14), Multidrug resistance protein 4 (also known as ABCC4, or MRP4, the polypeptide of which is preferably represented by SEQ ID NO: 15, which in turn is preferably encoded by the nucleotide represented by SEQ ID NO: 16), Multidrug and toxin extrusion protein 1 (also known as SLC47A1, or MATE1, the polypeptide of which is preferably represented by SEQ ID NO: 17, which in turn is preferably encoded by the nucleotide represented by SEQ ID NO: 18), Multidrug and toxin extrusion protein 2-K (also known as SLC47A2, or MATE2-K, or MATE2K, the polypeptide of which is preferably represented by SEQ ID NO: 19, which in turn is preferably encoded by the nucleotide represented by SEQ ID NO: 20), Sodium/phosphate cotransporter (also known as SLC34A1, or NaPi IIa, the polypeptide of which is preferably represented by SEQ ID NO: 21, which in turn is preferably encoded by the nucleotide represented by SEQ ID NO: 22), and Sodium-dependent phosphate transport protein 2C (also known as SLC34A3, NaPi IIc, the polypeptide of which is preferably represented by SEQ ID NO: 23, which in turn is preferably encoded by the nucleotide represented by SEQ ID NO: 24). Transporter polypeptides as described above can be susceptible to certain variations, such as polymorphisms. Such variants are recognized and understood as such by a person skilled in the art, and said variants are encompassed by the invention. Accordingly, preferred transporter polypeptides are polypeptides having at least 50%, 55%, 60%, 65%, 70%, 75%, 80%, 85%, 90%, 91%, 92%, 93%, 94%, 95%, 96%, 97%, 98%, 99% or most preferably 100% sequence identity or similarity with either SEQ ID NO: 5, 7, 9, 11, 13, 15, 17, 19, 21 or SEQ ID NO: 23; preferably encoded by polynucleotides having at least 50%, 55%, 60%, 65%, 70%, 75%, 80%, 85%, 90%, 91%, 92%, 93%, 94%, 95%, 96%, 97%, 98%, 99% or most preferably 100% sequence identity or similarity with either SEQ ID NO: 6, 8, 10, 12, 14, 16, 18, 20, 22 or SEQ ID NO: 24.
The human ciPTEC and PTEC according to the invention as listed here above expressing at least one further relevant transporter are advantageous because they can be considered to match the natural physiological environment of the transporter that is to be investigated or that is to be used to assess characteristics of other substances. As an example, a cell that would only express one specific import transporter and not an efflux transporter might not offer a reliable model for said import transporter, because the import transporter might lose functionality due to accumulation of substrates inside the cell. In a natural situation, the import transporter could be expressed against a background of other transporters, amongst which could be efflux transporters. In such a case, substrate inhibition due to intracellular accumulation would not affect import transporter functionality. Also, substance accumulation due to a lack of further transporter activity might result in perceived toxicity levels of said substance that are artificially high, because said substance is not exported, which would normally reduce its perceived toxicity. This leads to the conclusion that transporter model systems that do not comprise further relevant transporters can be considered to provide results that cannot reliably be translated into a clinical context.
A human ciPTEC or a PTEC according to the invention that stably expresses a functional OAT and expresses another functional renal transporter, offers a desirable model system that allows the use or analysis of OAT in a context that closely matches naturally occurring renal PTEC, which offers clinical significance.

Preferably, a human ciPTEC or PTEC that stably expresses functional OAT when cultured is obtainable by a method according to the invention comprising the following steps:
i) transducing a population of proximal tubule epithelial cells by a lentiviral particle comprising an expression construct that comprises a nucleotide sequence having at least 50%, 55%, 60%, 65%, 70%, 75%, 80%, 85%, 90%, 91%, 92%, 93%, 94%, 95%, 96%, 97%, 98%, 99% or most preferably 100% sequence identity with SEQ ID NO: 2 or with SEQ ID NO: 4,
ii) optionally enriching the transduced population obtained in (i), preferably by using fluorescence activated cell sorting (FACS), and
iii) subcloning the transduced population obtained in (i) of (ii) by selecting and isolating single cells and expanding these by culture.
In this context, transducing a cell is the transformation of a cell by integrating at least one nucleic acid as disclosed herein, which transformation may be carried out by any suitable known means which have been widely described in the specialist literature and in particular in the references cited in the present application, more particularly by a lentiviral particle comprising an expression construct as described below.
Lentiviral particles are particles that can function as a gene delivery vector. They are related to the *Retroviridae* family of RNA viruses and feature reverse transcriptase enzymes and integrase enzymes that allow the genetic cargo of the particles to be integrated in the genome of a host cell. Lentiviral transduction is a technique that is known to the skilled person, and that is described in the literature. More details of lentiviral transduction are provided in the examples. The methods of lentiviral transduction listed in the examples are preferred methods in the embodiments of the invention.
In this context, an expression construct is a nucleic acid construct comprising a nucleic acid molecule that has a nucleotide sequence as identified herein. An expression construct may comprise a first nucleic acid sequence encoding a polypeptide such as OAT, possibly further comprising a further nucleic acid sequence. An expression construct can be an expression vector, which can comprise any nucleic acid sequence. Preferably, an expression vector comprises a nucleotide sequence according to the invention, which is operably linked to one or more control sequences, which direct the production or expression of the encoded polypeptide in a cell, a subject, or a cell-free expression system. An expression vector may be seen as a recombinant expression vector. This vector can be constituted by a plasmid, a cosmid, a bacteriophage or a virus which is transformed by introducing a nucleic acid molecule as disclosed herein. Such transformation vectors according to the host organism to be transformed are well known to those skilled in the art and widely described in the literature.
Enriching of a population of cells of interest is a technique that is known in the art. A non-limiting example of a method for enriching a population of cells is to select and isolate desired cells using a fluorescence activated cell sorter (FACS). By culturing the isolated cells that each have the desired characteristic, the ensuing population of cells can be enriched for that desired characteristic.

Subcloning a population is to be interpreted as selecting and/or isolating cells, preferably single cells, that exhibit desirable characteristics, subsequently culturing said cells to expand their number. This allows the provision of populations that predominantly, preferably entirely, consist of cells that share the same characteristics as the originator cell or cells.
A preferred method of subcloning is the method described in the examples herein. Preferably, the ciPTEC obtainable is derived from ciPTEC DSM ACC 3019 or is derived from a passage or isolate thereof.

Preferably, the human PTEC or human ciPTEC that stably expresses functional OAT when cultured, is obtainable by a method according to the invention comprising the following steps:
i) transducing a population of proximal tubule epithelial cells by a lentiviral particle comprising an expression construct that has at least 50%, 55%, 60%, 65%, 70%, 75%, 80%, 85%, 90%, 91%, 92%, 93%, 94%, 95%, 96%, 97%, 98%, 99% or most preferably 100% sequence identity with an expression construct selected from the group consisting of:
   1) pLenti4/V5-EX-CMV-TetO2-hOAT1 (SEQ ID NO: 25), and
   2) pLenti4/V5-EX-CMV-TetO2-hOAT3 (SEQ ID NO: 26).,
ii) optionally enriching the transduced population obtained in (i), preferably by using fluorescence activated cell sorting (FACS), and
iii) subcloning the transduced population obtained in (i) of (ii) by selecting and isolating single cells and expanding these by culture. The features of this method are preferably those as described earlier herein.
Preferably, a human ciPTEC or PTEC according to the invention that stably expresses functional OAT when cultured is ciPTEC.OAT1.4B2 DSM ACC3279 at DSMZ - German Collection of Microorganisms and Cell Cultures; Inhoffenstrasse 7 B; 38124 Braunschweig; Germany, or a passage or isolate thereof that stably expresses functional OAT1 when cultured.
Preferably, a human ciPTEC or PTEC according to the invention that stably expresses functional OAT when cultured is ciPTEC.OAT3.3C1 DSM ACC3280 at DSMZ, or a passage or isolate thereof that stably expresses functional OAT3 when cultured.

The human ciPTEC according to the invention is for use in any method that is described elsewhere herein and for any use described elsewhere herein.

### Method

In a second aspect there is provided a method for the production of a human proximal tubule epithelial cell that stably expresses an organic anion transporter when cultured, said method comprising:
i) transducing a population of proximal tubule epithelial cells by a lentiviral particle comprising an expression construct that comprises a nucleotide sequence having at least 50%, 55%, 60%, 65%, 70%, 75%, 80%, 85%, 90%, 91%, 92%, 93%, 94%, 95%, 96%, 97%, 98%, 99% or most preferably 100% sequence identity with SEQ ID NO: 2 or with SEQ ID NO: 4,
ii) optionally enriching the transduced population obtained in (i), preferably by using fluorescence activated cell sorting (FACS), and
iii) subcloning the transduced population obtained in (i) of (ii) by selecting and isolating single cells and expanding these by culture.
Said method is herein referred to as a method according to the invention.
The features of the method are preferably the features described in the first aspect of the invention.
Preferably, in said method according to the invention, the expression construct has at least 50%, 55%, 60%, 65%, 70%, 75%, 80%, 85%, 90%, 91%, 92%, 93%, 94%, 95%, 96%, 97%, 98%, 99% or most preferably 100% sequence identity with an expression construct selected from the group consisting of:
i) pLenti4/V5-EX-CMV-TetO2-hOAT1 (SEQ ID NO: 25), and
ii) pLenti4/V5-EX-CMV-TetO2-hOAT3 (SEQ ID NO: 26).

In a third aspect, the invention provides for an *in vitro* or *ex vivo* method for analysis of a substance or of a composition, comprising contacting said substance with at least one cell according to the invention, preferably with a monolayer of a multiplicity of said cell, more preferably a mature monolayer of a multiplicity of said cell.
The features of the method are preferably the features described in the first aspect of the invention. In this context, 'substance' should be read as molecules, complexes of multiple molecules, oligomers, polymers, polypeptides, proteins, or particles, or fragments thereof. In this context, contacting a cell with a substance or a composition can comprise adding such a substance or composition to a medium in which a cell is cultured. Contacting a cell with a substance or a composition can also comprise adding such a substance or composition to a medium, buffer, or solution in which a cell is suspended, or which covers a cell. Other preferred methods of contacting a cell comprise injecting a cell with a substance or composition, or exposing a cell to a material comprising a substance or composition.
Preferably, said method is for determining the nephrotoxicity of said substance, and preferably further comprises the subsequent analysis of cell viability, preferably by analysis of cellular dehydrogenase capacity, more preferably by a tetrazolium (MTT) assay. Cell viability assays are known to the person skilled in the art and the person skilled in the art is capable of selecting a proper assay. In this context, nephrotoxicity is to be interpreted as a poisonous effect of substances on the kidneys of a subject, or on kidney cells such as PTEC or ciPTEC. There are various forms of toxicity, and this term is well-understood in the art. Nephrotoxins are substances displaying nephrotoxicity. If a substance causes a decrease in kidney cell, PTEC, or ciPTEC viability or activity when it is contacted with a kidney cell, PTEC, or ciPTEC, this can be construed as said substance being a nephrotoxin.
Preferably, said method is for the functional analysis of the interaction of said substance with a transporter, preferably a renal transporter, and wherein said contacting preferably is in the presence of a labeled anionic transporter substrate, preferably a radiolabeled (e.g. ³H or ¹⁴C) or fluorescently labeled anionic transporter substrate, more preferably the fluorescent substrate is selected from the group consisting of fluorescein, ASP (preferably 4-(4-(didecylamino)styryl)-N-methylpyridinium iodide), and calcein. Appropriate labels and substrates are known to the person skilled in the art and the person skilled in the art is capable of selecting a proper label and/or substrate. In this context, a substrate or a transporter substrate is a substance that has known transport efficiency or uptake affinity or export affinity in relation to specific transporters. In other words, a substrate has known interaction with a transporter. Interaction of a substance with a reporter, in this context, is to be construed as a substance being recruited by a transporter, being transported by a transporter, inhibiting or decreasing the activity of a transporter (either permanently, through competition, or allosterically), or promoting or increasing the activity of a transporter. Non-limiting examples of parameters that define aspects of interaction of a substance with a transporter are IC₅₀, K_{I}, and Cₘₐₓ/IC₅₀. These parameters are known to a skilled person. Functional analysis of interaction can be interpreted as the determination of such parameters as they relate to a specific interaction.
Preferably, said method according to the invention further comprises determining the drug-drug interaction of said substance. Preferably, the method further comprises determining whether said substance is a substrate or an inhibitor of a transporter involved in a clinically relevant drug-drug interaction.
Drug-drug interaction (DDI) is the effect that the presence of a first substance has an effect on the properties of a second substance. For example, a first substance, in interacting with a transporter, might alter the interaction of said transporter with a second substance. A non-limiting example of such behaviour is that para-aminohippuric acid inhibits the uptake of fluorescein by OAT1, and thus it alters the characteristics of fluorescein by lowering its rate of transport by OAT1. In undesirable cases of DDI, a first drug could interact with a transporter in such a way that a second drug undergoes a change in its adsorption, distribution, metabolism, or excretion, all of which might alter the toxicity of the second drug. This could result in the second drug losing its efficacy or losing its therapeutic value.
Existing drugs are known to sometimes be withdrawn from the market, denied regulatory approval, or discontinued from clinical development because of their so-called victim potential (which means they are the object of a drug-drug interaction - their characteristics are undesirably changed by the effect of a second drug) or their so-called perpetrator potential (which means they are the precipitating cause of the drug-drug interaction - their presence undesirable affects the characteristics of a second drug). Regardless of a drug being a perpetrator or a victim, nephrotoxicity of a compound as a result of DDI is a significant cause of drug candidate attrition during pharmaceutical development, because it is often recognized only during the clinical stages of development: the translation from in vitro and animal studies known in the prior art to human studies lacks sufficient predictivity. Methods of the present invention can overcome this problem.

In a fourth aspect, the invention provides for the use of a cell according to the invention in analysis of a substance, comprising contacting said cell with said substance, preferably using a monolayer of a multiplicity of said cell, more preferably a mature monolayer of a multiplicity of said cell. In an embodiment, said analysis is for determining the nephrotoxicity of said substance. In an embodiment, said analysis is for the functional analysis of the interaction of said substance with a transporter, preferably a renal transporter, and wherein said contacting preferably is in the presence of a labeled or fluorescent anionic transporter substrate, preferably fluorescein. The features of the use are preferably the features described in the first or second aspect of the invention. Preferably, said analysis further comprises determining the drug-drug interaction of said substance. Preferably, said analysis further comprises determining whether said substance is a substrate or an inhibitor of a transporter involved in a clinically relevant drug-drug interaction.

### Kit

In a fifth aspect, the present invention provides for a kit of parts comprising a cell according to the invention and instructions for use.

In an embodiment, said kit of parts is for use in any method described elsewhere herein and for any use described elsewhere herein.

### General definitions

Each nucleotide sequence or amino acid sequence described herein by virtue of its identity or similarity percentage (at least 50%) with a given nucleotide sequence or amino acid sequence respectively has in a further preferred embodiment an identity or a similarity of at least 55%, 65%. 65%, 70%, 75%, 80%, 85%, 90%, 95%, 97%, 98%, 99% or more identity or similarity with the given nucleotide or amino acid sequence respectively. In a preferred embodiment, sequence identity or similarity is determined by comparing the whole length of the sequences as identified herein.
"Sequence identity" is herein defined as a relationship between two or more amino acid (polypeptide or protein) sequences or two or more nucleic acid (polynucleotide) sequences, as determined by comparing the sequences. In a preferred embodiment, sequence identity is calculated based on the full length of two given SEQ ID NO or on part thereof. Part thereof preferably means at least 50%, 60%, 70%, 80%, 90%, or 100% of both SEQ ID NO. In the art, "identity" also means the degree of sequence relatedness between amino acid or nucleic acid sequences, as the case may be, as determined by the match between strings of such sequences.
"Similarity" between two amino acid sequences is determined by comparing the amino acid sequence and its conserved amino acid substitutes of one polypeptide to the sequence of a second polypeptide. "Identity" and "similarity" can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heine, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48:1073 (1988).
Preferred methods to determine identity are designed to give the largest match between the sequences tested. Methods to determine identity and similarity are codified in publicly available computer programs. Preferred computer program methods to determine identity and similarity between two sequences include e.g. the GCG program package (Devereux, J., et al., Nucleic Acids Research 12 (1): 387 (1984)), BestFit, BLASTP, BLASTN, and FASTA (Altschul, S. F. et al., J. Mol. Biol. 215:403-410 (1990). The BLAST X program is publicly available from NCBI and other sources (BLAST Manual, Altschul, S., et al., NCBI NLM NIH Bethesda, MD 20894; Altschul, S., et al., J. Mol. Biol. 215:403-410 (1990). The well-known Smith Waterman algorithm may also be used to determine identity.
Preferred parameters for polypeptide sequence comparison include the following: Algorithm: Needleman and Wunsch, J. Mol. Biol. 48:443-453 (1970); Comparison matrix: BLOSSUM62 from Hentikoff and Hentikoff, Proc. Natl. Acad. Sci. USA. 25 89:10915-10919 (1992); Gap Penalty: 12; and Gap Length Penalty: 4. A program useful with these parameters is publicly available as the "Ogap" program from Genetics Computer Group, located in Madison, WI. The aforementioned parameters are the default parameters for amino acid comparisons (along with no penalty for end gaps). Preferred parameters for nucleic acid comparison include the following: Algorithm: Needleman and Wunsch, J. Mol. Biol. 48:443-453 (1970); Comparison matrix: matches=+10, mismatch=0; Gap Penalty: 50; Gap Length Penalty: 3. Available as the Gap program from Genetics Computer Group, located in Madison, Wis. Given above are the default parameters for nucleic acid comparisons.
Optionally, in determining the degree of amino acid similarity, the skilled person may also take into account so-called "conservative" amino acid substitutions, as will be clear to the skilled person. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulphur-containing side chains is cysteine and methionine. Preferred conservative amino acids substitution groups are: valine-leucine-isoleucine, phenylalanine-tyrosine, lysine-arginine, alaninevaline, and asparagine-glutamine. Substitutional variants of the amino acid sequence disclosed herein are those in which at least one residue in the disclosed sequences has been removed and a different residue inserted in its place. Preferably, the amino acid change is conservative. Preferred conservative substitutions for naturally occurring amino acids are as follows, and can be inverted: Ala to Ser; Arg to Lys; Asn to Gln or His; Asp to Glu; Cys to Ser or Ala; Gln to Asn; Glu to Asp; Gly to Pro; His to Asn or Gln; Ile to Leu or Val; Leu to Ile or Val; Lys to Arg; Gln or Glu; Met to Leu or Ile; Pro to Gly; Phe to Met, Leu or Tyr; Ser to Thr; Thr to Ser; Trp to Tyr; Tyr to Trp or Phe; and, Val to Ile or Leu.

In this document and in its claims, the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".
The word "about" or "approximately" when used in association with a numerical value (e.g. about 10) preferably means that the value may be the given value (of 10) more or less 0.1% of the value.

The sequence information as provided herein should not be so narrowly construed as to require inclusion of erroneously identified bases. The skilled person is capable of identifying such erroneously identified bases and knows how to correct for such errors. In case of sequence errors, the sequence of the gene products obtainable by expression of the genes as represented by SEQ ID NO's 2, 4, 6, 8, 10, 12, 14, 16, 18, 20, 22, 24, 25, and 26 encoding polynucleotide sequences should prevail.

**Table 1. Michaelis-Menten parameters for OAT-mediated fluorescein uptake in ciPTEC-OAT1 and in ciPTEC-OAT3. Data are expressed as mean±SEM, n=4.**

| | **ciPTEC-OAT1** | **ciPTEC-OAT3** |
|---|---|---|
| Kₘ (µM) | 0.8±0.1 | 3.7±0.5 |
| Vₘₐₓ (RFU) | 695±84 | 384±103 |
| K_{d} (RFU^{∗}µmol/L) | 2.4±1.2 | 4.3±0.9 |

**Table 2. Inhibitory potencies of substrates and/or inhibitors of fluorescein uptake in ciPTEC-OAT1 and ciPTEC-OAT3 and a selection of reference values. Data are expressed as mean±SEM, n=4.**

| **Present study** | | | | |
|---|---|---|---|---|
| | | | **Cell line** | **IC₅₀ (µM)** |
| para-aminohippuric acid (PAH) | | | ciPTEC-OAT1 | 18±4 |
| | | | ciPTEC-OAT3 | 152±3 |
| estrone sulfate (ES) | | | ciPTEC-OAT1 | 54±13 |
| | | | ciPTEC-OAT3 | 2.1±0.3 |
| Probenecid (pbc) | | | ciPTEC-OAT1 | 12.7±0.5 |
| | | | ciPTEC-OAT3 | 1.9±0.6 |
| Furosemide (fsm) | | | ciPTEC-OAT1 | 25±4 |
| | | | ciPTEC-OAT3 | 2.3±0.4 |
| Cimetidine (cmd)(Apparent IC₅₀ values due to partial inhibition) | | | ciPTEC-OAT1 | 654±291 |
| | | | ciPTEC-OAT3 | 215±162 |
| Diclofenac (dfn) | | | ciPTEC-OAT1 | 5±1 |
| | | | ciPTEC-OAT3 | 3±1 |
| **Literature** | | | | |

| **IC₅₀ (µM)** | **K_{I} (µM)** | **Substrate (inhibitor)** | **Cell line** | **Reference** |
|---|---|---|---|---|
| 8.8 | | 6-carboxyfluorescein (PAH) | CHO-OAT1 | 28 |
| | 6.02 | ochratoxin A (PAH) | S2-OAT1 | 47 |
| | 19.6 | ochratoxin A (PAH) | S2-OAT3 | 47 |
| | 100 | Benzylpenicillin (PAH) | HEK293-hOAT1 | 48 |
| >100 | | PAH (ES) | S2-OAT1 | 49 |
| 3 | | estrone sulfate (ES) | Xenopus-OAT3 | 50 |
| | 4.29 | ochratoxin A (pbc) | S2-OAT1 | 47 |
| 6.3 | | 6-carboxyfluorescein (pbc) | CHO-OAT1 | 28 |
| | 12.1 | PAH (pbc) | S2-OAT1 | 15 |
| 3.1 | | Cimetidine (pbc) | CHO-OAT3 | 51 |
| | 4.41 | ochratoxin A (pbc) | S2-OAT3 | 47 |
| 18 | | PAH (fsm) | S2-OAT1 | 52 |
| 17.31 | | estrone sulfate (fsm) | S2-OAT3 | 52 |
| 1.7 | | Sitagliptin (fsm) | CHO-OAT3 | 51 |
| 492 | | PAH (cmd) | S2-OAT1 | 34 |
| 79 | | Sitagliptin (cmd) | CHO-OAT3 | 51 |
| 53 | | estrone sulfate (cmd) | Xenopus-OAT3 | 53 |
| 4.46 | | PAH (dfn) | S2-OAT1 | 54 |
| 4 | | Adefovir (dfn) | CHO-OAT1 | 55 |
| 7.78 | | estrone sulfate (dfn) | S2-OAT3 | 54 |

**Table 3. Inhibitory potencies of antivirals on fluorescein uptake using ciPTEC-OAT1 and ciPTEC-OAT3 compared with a selection of reference values. In the current study, fluorescein inhibition by the model compounds was measured. For references, the competitive substrate is provided. Data are expressed as mean±SEM, n=3.**

| **Present study** | | | | | | | | | | |
|---|---|---|---|---|---|---|---|---|---|---|
| **Antiviral** | **Cell line** | | | | **IC₅₀ (□M)** | | | | | |
| Adefovir | ciPTEC-OAT1 | | | | 23±4 | | | | | |
| | ciPTEC-OAT3 | | | | N.A. | | | | | |
| Cidofovir | ciPTEC-OAT1 | | | | 71±34 | | | | | |
| | ciPTEC-OAT3 | | | | N.A. | | | | | |
| Tenofovir | ciPTEC-OAT1 | | | | 42±8 | | | | | |
| | ciPTEC-OAT3 | | | | N.A. | | | | | |
| Zidovudine | ciPTEC-OAT1 | | | | 14±7 | | | | | |
| | ciPTEC-OAT3 | | | | 21±4 | | | | | |

| **Literature** | | | | | | | | **DDI index** | | |
|---|---|---|---|---|---|---|---|---|---|---|
| **Antiviral** | **IC₅₀ (µM)** | | **K_{I} (µM)** | **Substrate** | | **Cell line** | **Ref.** | **Cₘₐₓ** | **Cₘₐₓ/ IC₅₀** | **Ref.** |
| Adefovir | 8.1 | | | PAH | | HeLa-OAT1 | 29 | 1.6 | 0.18 | 56 |
| | 28 | | | 6-carboxyfluorescein | | CHO-OAT1 | 28 | 38.8 | 4.2 | 57 |
| | | | 23.8 | | | CHO-OAT1 | | | | |
| Cidofovir | 60 | | | 6-carboxyfluorescein | | CHO-OAT1 | 28 | 15.8 | 0.53 | 58 |
| | | | 58 | | | CHO-OAT1 | | 26.3 | 0.88 | |
| Tenofovir | 29.3 | | | PAH | | HeLa-OAT1 | 29 | 0.52 | 0.014 | 60 |
| | | | 33.8 | | | | 35 | 0.72 | 0.019 | 61 |
| Zidovudin e | | | 45.9 | | | S2-OAT1 | | 5.5 | 0.55 | 62 |
| | | | | | | | | | 0.66 | |
| | | | 145 | | | S2-OAT1 | | 6.6 | 0.69 | 62 |
| | | | | | | | | | 0.83 | |

**Table 4. Inhibitory potencies of antivirals on cell viability as measured by MTT assay using ciPTEC-OAT1 and a selection of values as found in literature for cells that express OAT without also expressing all of the other functional transporters that are expressed in ciPTEC. Data are expressed as mean±SEM, n≥3.**

| | ciPTEC-OAT1 | | | | | |
|---|---|---|---|---|---|---|
| | Present Study | | | Literature | | |
| | 24h | 48h | 72h | 48h | 120h | Ref |
| Adefovir | 462±52 | 303±38 | 230±37 | 0.22±0.08 | 1.4±0.7 | 35, 37 |
| Cidofovir | 613±384 | 130±58 | 69±2 | 0.5±0.2 | 3±1 | 35, 37 |
| Tenofovir | 114±25 | 189±48 | 223±67 | 10±2 | 21±7 | 35, 37 |

**Table 5. Sequences**

| SEQ ID NO | Nucleic acid / Polypeptide | Sequence |
|---|---|---|
| 1 | SLC22A6 PRT (OAT1) | |
| 2 | SLC22A6 NA | |
| | | |
| 3 | SLC22A8 PRT (OAT3) | |
| 4 | SLC22A8 NA | |
| 5 | SLC22A2 PRT (OCT2) | |
| 6 | SLC22A2 NA | |
| | | |
| 7 | SLCO4C1 PRT (OATP-H) | |
| 8 | SLCO4C1 NA | |
| | | |
| 9 | ABCB1 PRT (PgP) | |
| 10 | ABCB1 NA | |
| | | |
| 11 | ABCG2 PRT (BCRP) | |
| | | |
| 12 | ABCG2 NA | |
| 13 | ABCC2 PRT (MRP2) | |
| | | |
| 14 | ABCC2 NA | |
| | | |
| 15 | ABCC4 PRT (MRP4) | |
| 16 | ABCC4 NA | |
| | | |
| | | |
| 17 | SLC47A1 PRT (MATE1) | |
| 18 | SLC47 A1 NA | |
| 19 | SLC47A2 PRT (MATE-2K) | |
| | | |
| 20 | SLC47A2 NA | |
| 21 | SLC34A1 PRT (NaPi IIa) | |
| 22 | SLC34A1 NA | |
| | | |
| 23 | SLC34A3 PRT (NaPi IIc) | |
| 24 | SLC34A3 NA | |
| | | |
| 25 | pLenti4/V 5-EX-CMV-TetO2-hOAT1 | |
| | | |
| | | |
| 26 | pLenti4/V 5-EX-CMV-TetO2-hOAT3 | |
| | | |
| | | |
| | | |
| 27 | Primer Cla1-CMV-TetO2 | GCCGCCATCGATGCCGCCGTTGACATTGATTATTGACT |
| 28 | Primer EcoRI-CMV-TetO2 | GGCGGCGAATTCGGCGGCCGGAGGCTGGATCGGTCCCGG |

### FIGURE LEGENDS

**Figure 1****.** Schematic overview of transduction procedure to obtain ciPTEC-OAT1 and ciPTEC-OAT3.
   (A) ciPTEC parent was transduced with OAT1 or OAT3 lentiviral constructs and enriched by FACS using OATs' capacity to transport fluorescein. Further subcloning using radiated 3T3 fibroblasts as feeder cells resulted in a homogeneous ciPTEC-OAT1 or ciPTEC-OAT3 cell line.
   (B), (C) and (D) show histograms obtained by flow cytometry of ciPTEC parent (B), and of ciPTEC-OAT1 (C) or ciPTEC-OAT3 (D) exposed to fluorescein (1µM, 10 min, dashed line), fluorescein and para-aminohippuric acid (100 µM, dotted line), or untreated cells (continuous line). Parent cells exposed to fluorescein did not show increased fluorescence intensity, while ciPTEC-OAT1 and ciPTEC-OAT3 both showed a sub-population with increased fluorescence, which is indicative for OAT functionality. Fluorescence increase in ciPTEC-OAT1 was sensitive to inhibition induced by para-aminohippuric acid, as evidenced by the shift of the subpopulation.
   (E) shows a scatter plot showing forward scatter (y-axis) and fluorescein intensity (x-axis) of transduced ciPTEC-OAT1 exposed to 1 µM fluorescein for 10 min. The population with high fluorescence intensity indicated by gate PI (8.3 % of total population) was sorted to enrich successfully transduced ciPTEC-OAT1. Transduction with OAT3 was more efficient than OAT1, represented by the larger positive subpopulation in figure ID compared to figure 1C, making the enrichment protocol redundant for ciPTEC-OAT3.
   (F) shows a histogram of enriched ciPTEC-OAT1 exposed to fluorescein (1 µM, 10 min) in presence (dotted line) or absence (dashed line) of the competitor para-aminohippuric acid (100 µM). It demonstrates increased fluorescence intensity upon exposure to fluorescein as compared to non-enriched ciPTEC, but it also indicates a heterogeneous population that is sensitive to para-aminohippuric acid, pointing towards the requirement of subcloning of the enriched cells.

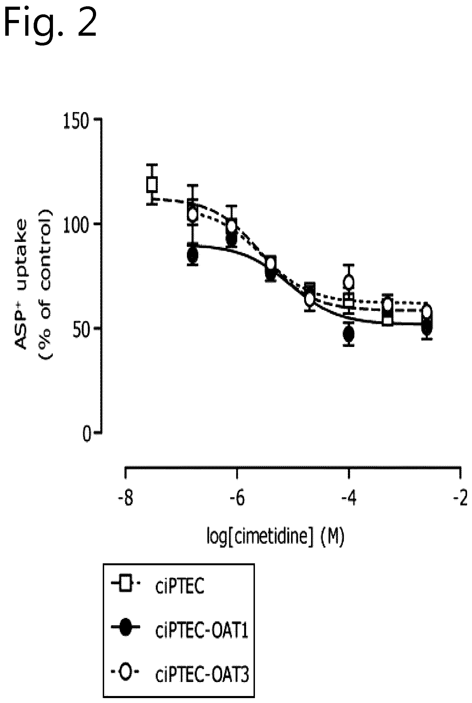

**Figure 2****.** Uptake of (4-(4-(dimethylamino)styryl)-*N*-methylpyridinium (ASP+) (1 µM) by ciPTEC parent, ciPTEC-OAT1, or ciPTEC-OAT3, each when co-incubated with OCT2-substrate cimetidine for 60 min in Hank's Balanced Salt Solution (HBSS) at 37° C, relative to uptake without inhibitor. The lines represent the fit according to a one-site competition model. Values are expressed as ±SEM, (ciPTEC, n=3; ciPTEC-OAT1, n=4; ciPTEC-OAT3, n=2. Analysis using Two-way ANOVA indicated significant inhibition of ASP+ uptake at OCT2 with cimetidine, resulted in similar IC₅₀ (p>0.05).

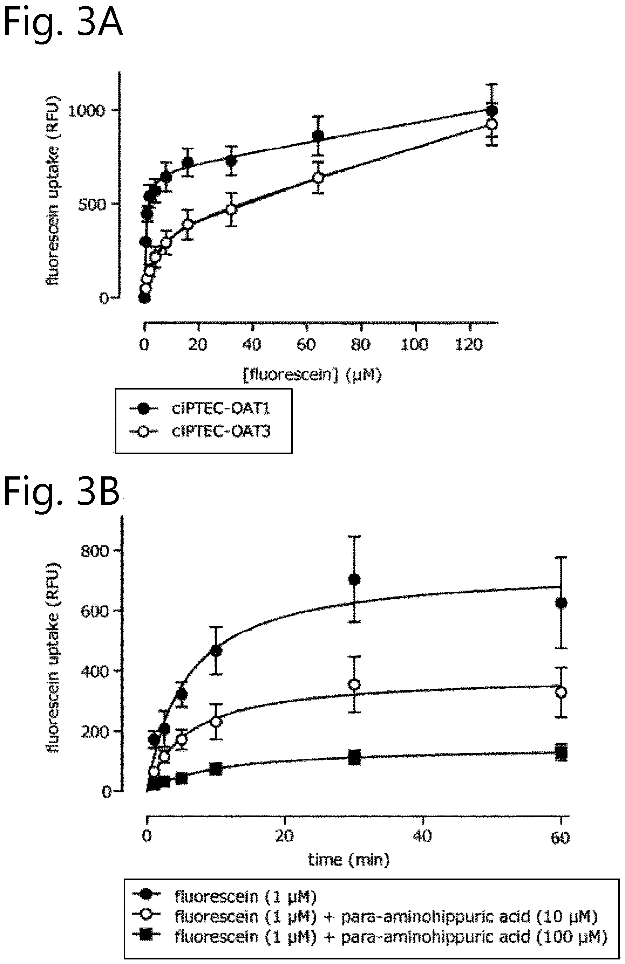

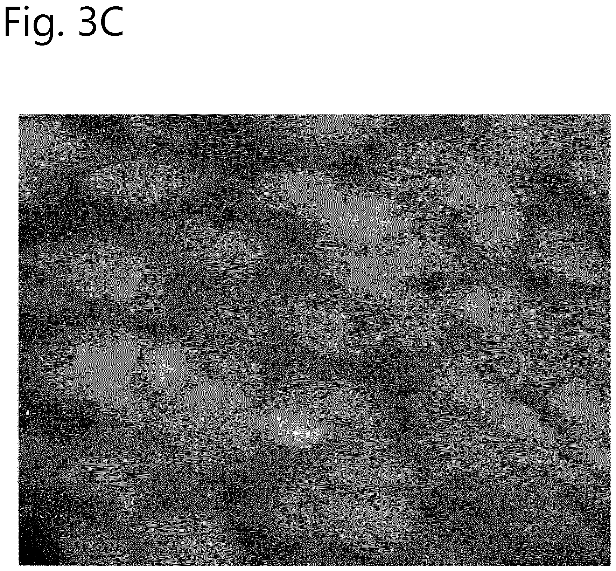

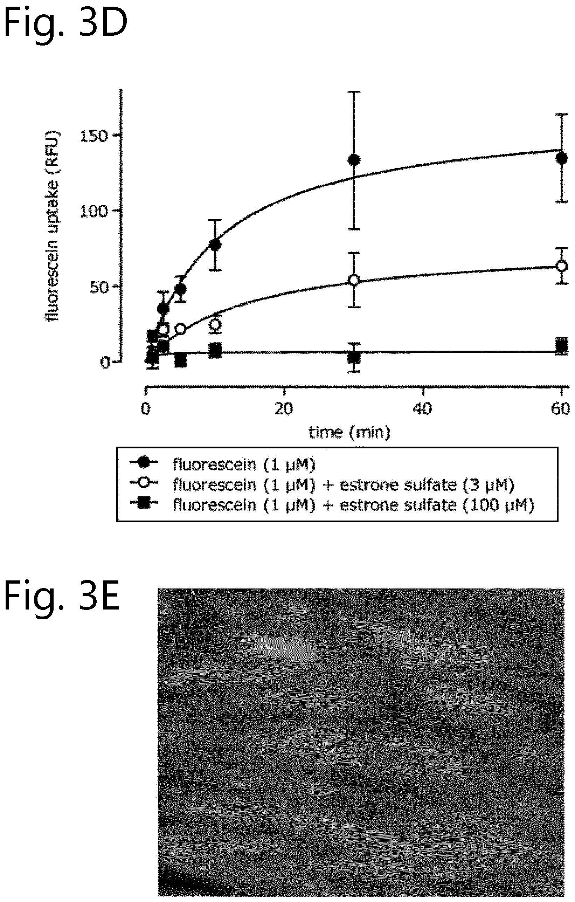

**Figure 3****.** OAT-mediated fluorescein uptake in ciPTEC-OAT1 and ciPTEC-OAT3. (A) Concentration-dependent OAT1 and OAT3 mediated uptake of fluorescein after 10 min incubation in ciPTEC-OAT1 and ciPTEC-OAT3. The curve was fitted (n=4) according to a Michaelis-Menten model in combination with linear diffusion.
   (B, C) Fluorescein uptake (1 µM) by ciPTEC-OAT1 and (D, E) ciPTEC-OAT3 up to 60 min in absence or presence of two concentrations of the typical inhibitors para-aminohippuric acid (PAH, for ciPTEC-OATl) or estrone sulfate (ES, for ciPTEC-OAT3). (B, D) The curves were fitted (n=4) to a standard saturation model after background subtraction. Analysis using two-way ANOVA indicated significantly decreased uptake curves in both ciPTEC-OAT1 (10µM and 100µM PAH, p<0.001)) and ciPTEC-OAT3 (3 µM ES, p<0.01; 100 µM ES, ***p<0.001). (C, E) Representative images of fluorescein uptake (1 µM) by ciPTEC-OAT1 (C) and ciPTEC-OAT3 (E) after 10 min (magnification 20x).

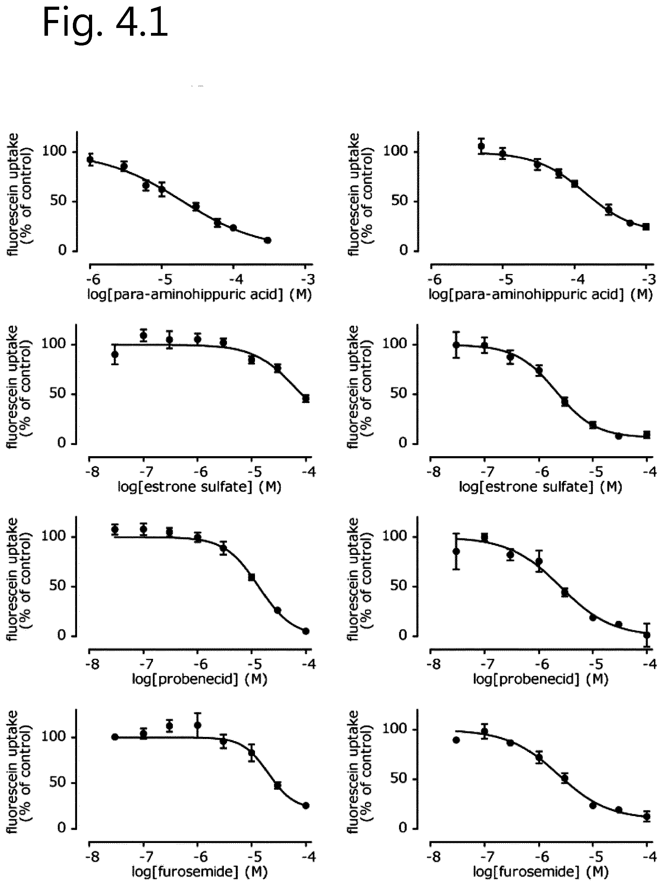

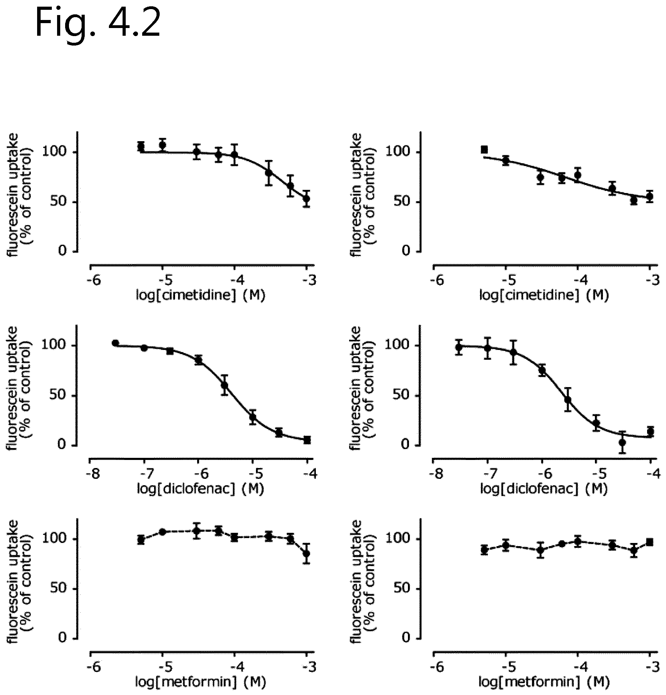

**Figure 4****.** Inhibition of OAT-mediated fluorescein uptake by a panel of OAT-perpetrators. Fluorescein uptake (1 µM) by ciPTEC-OAT1 (left set of graphs) and ciPTEC-OAT3 (right set of graphs) when co-incubated with any one of para-aminohippuric acid, estrone sulfate, probenecid, furosemide, cimetidine, diclofenac, or metformin for 10 min in HBSS at 37° C, relative to uptake without inhibitor. The line represents the fit according to a one-site competition model with variable slope, except for metformin. Values are derived from experiments performed at passage x+8, x+11, x+14 and x+29 upon transduction (n=4).

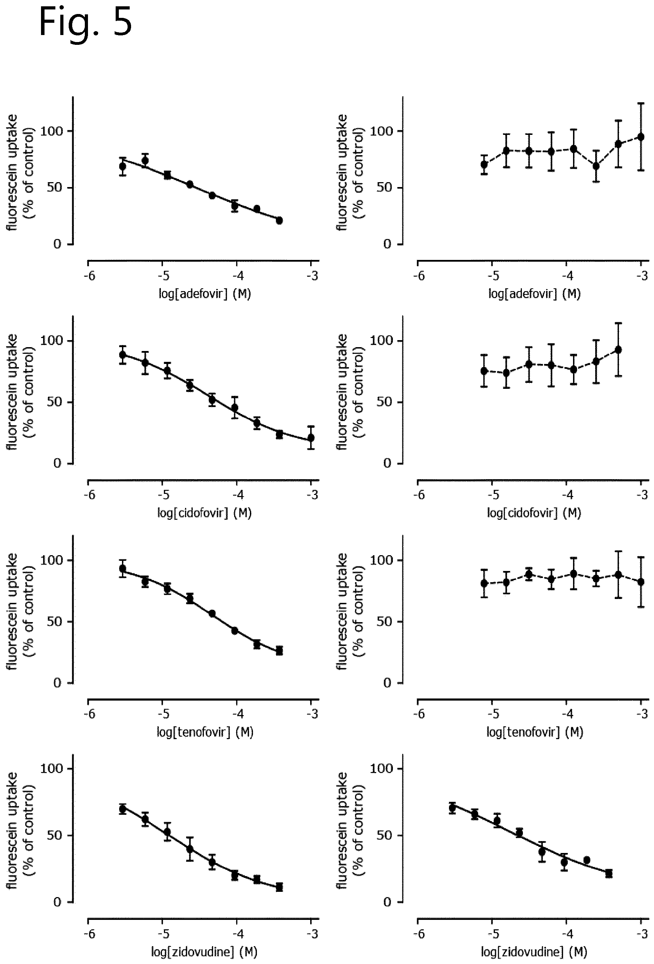

**Figure 5****.** Inhibition of OAT-mediated fluorescein uptake by adefovir, cidofovir, tenofovir, or zidovudine. Fluorescein uptake (1 µM) by ciPTEC-OAT1 (left set of graphs) and ciPTEC-OAT3 (right set of graphs) when co-incubated with any one of the antivirals for 10 min in HBSS at 37° C, relative to uptake without inhibitor. The line represents the fit according to a one-site competition model with variable slope (n=4).

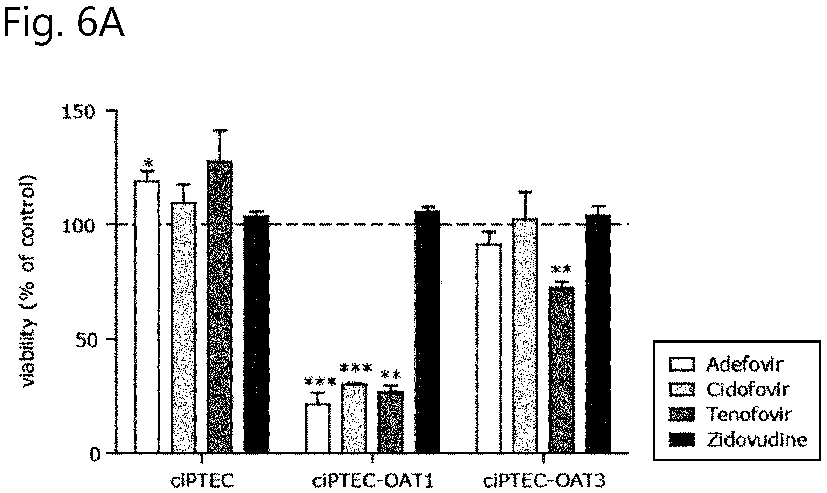

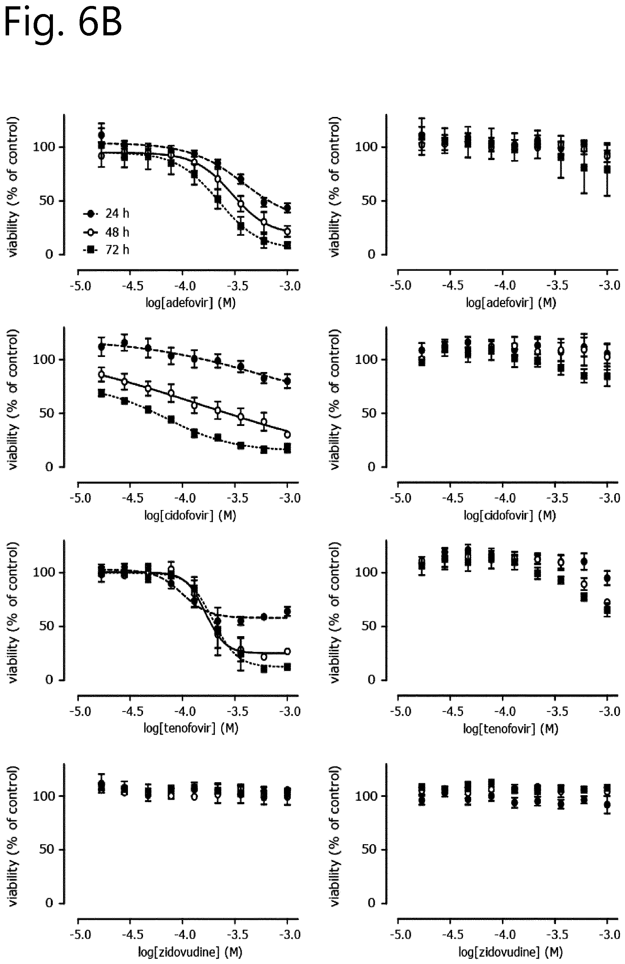

**Figure 6****.** Antiviral-induced toxicity in ciPTEC-OAT1 and ciPTEC-OAT3.
   (A) Viability of ciPTEC parent, ciPTEC-OAT1 and ciPTEC-OAT3 after exposure to antiviral agent (1 mM) for 48 h in serum free medium relative to cell viability without exposure, as measured with the MTT assay (n=3). **p<0.01, ***p<0.001.
   (B) Viability of ciPTEC-OAT1 (left set of graphs) and ciPTEC-OAT3 (right set of graphs) upon tenofovir, adefovir, cidofovir, or zidovudine exposure for 24, 48 and 72 h in serum free medium, relative to cell viability without exposure. The line represents the fit according to a one-site competition model with variable slope (n ≥3).

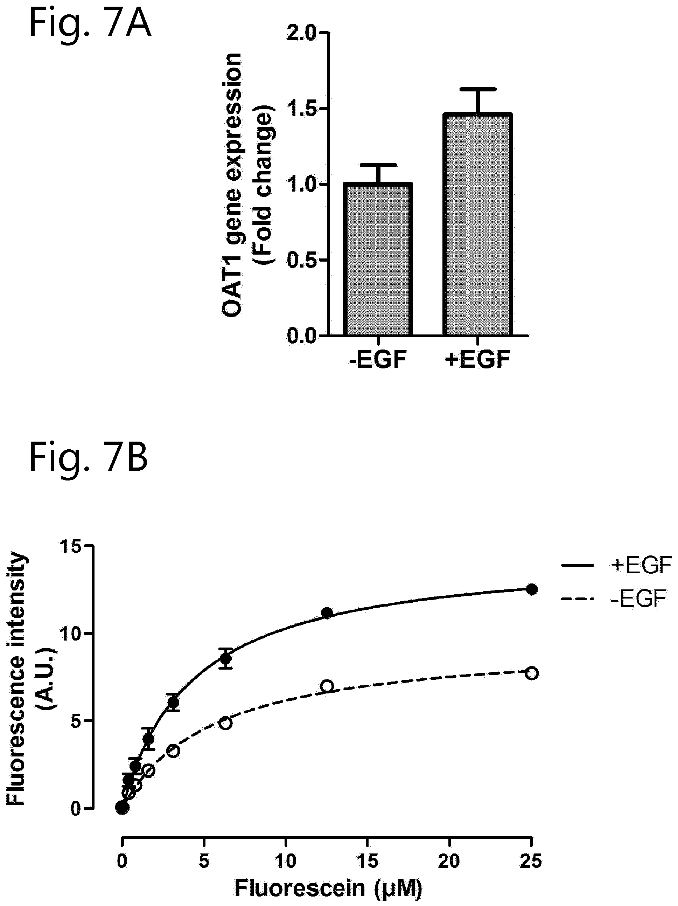

**Figure 7****.** Expression and transport activity of OAT1 in ciPTEC-OAT1 is regulated by EGF.
   OAT1 gene expression (A) and transport activity (B) was determined in isolated RNA fractions and cultured ciPTEC-OAT1, respectively. The activity of OAT1 was determined using a fluorescent OAT1 substrate (fluorescein); OAT1 expression and activity were increased upon exposure to EGF. Data are presented as mean values +/-SEM, n=3. Statistical analysis was performed via unpaired Student's *t*-test.

### EXAMPLES

The present invention is further described by the following examples which should not be construed as limiting the scope of the invention.

Unless stated otherwise, the practice of the invention will employ standard conventional methods of molecular biology, virology, microbiology or biochemistry. Such techniques are described in Sambrook et al. (1989) Molecular Cloning, A Laboratory Manual (2nd edition), Cold Spring Harbor Laboratory, Cold Spring Harbor Laboratory Press; in Sambrook and Russell (2001) Molecular Cloning: A Laboratory Manual, Third Edition, Cold Spring Harbor Laboratory Press, NY; in Volumes 1 and 2 of Ausubel et al. (1994) Current Protocols in Molecular Biology, Current Protocols, USA; and in Volumes I and II of Brown (1998) Molecular Biology LabFax, Second Edition, Academic Press (UK); Oligonucleotide Synthesis (N. Gait editor); Nucleic Acid Hybridization (Hames and Higgins, eds.).

In the examples here below, ciPTEC (also referred to as ciPTEC cells, ciPTEC parent, parent ciPTEC or ciPTEC parent cells) is DSM ACC 3019; ciPTEC OAT1 is ciPTEC.OAT1.4B2 DSM ACC3279 and ciPTEC OAT3 is ciPTEC.OAT3.3C1 DSM ACC3280.

### Example 1. - material and methods

### Cell culture

Conditionally immortalized proximal tubule epithelial cells (ciPTEC) were developed as described by Wilmer et al. with informed consent of the donors in accordance with the approved guidelines of the Radboud Institutional Review Board. (Wilmer et al., 2010) Cells were seeded 7 days prior to the experiment at their corresponding density (55,000 cells/cm² for ciPTEC parent cells, 63,000 cells/cm² for ciPTEC-OAT1 and 82,000 cells/cm² for ciPTEC-OAT3) and grown for 1 day at 33° C and 5% v/v CO₂ to allow proliferation, enabled by the temperature-sensitive mutant of SV large T antigen (SV40T). Next, cells were cultured for 6 days at 37° C and 5% v/v CO₂ to stimulate differentiation and formation of an epithelial monolayer, described as 'maturation'. Cells were cultured using Dulbecco's modified eagle medium (DMEM HAM's F12, Life Technologies, Paisly, UK), 5 µg/ml insulin, 5 µg/ml transferrin, 5 µg/ml selenium, 35 ng/ml hydrocortisone, 10 ng/ml epidermal growth factor (EGF), 40 pg/ml triiodothyronine (Sigma, St. Louis, USA) and 10% fetal calf serum (FCS, Greiner Bio One, Kremsmuenster, Austria). Medium was refreshed every second day, supplemented with 1% penicillin/streptomycin (pen/strep, Invitrogen, Carlsbad, USA) at 33° C and without pen/strep at the maturation temperature of 37° C. 3T3 mouse-fibroblast (3T3) cells were cultured at 37° C and used as feeder cells for sub-cloning procedures upon transduction, as described (Wilmer et al., 2010).

### Vector construction

Vector construction was performed using Gateway Cloning Technology (Invitrogen), according to the manufacturer's instructions. Commercially obtained vectors containing OAT1 (pENTR201-hOAT1, Harvard Plasmids HsCD00044153) and OAT3 (pENTR201-hOAT3, HsCD00044090) were transferred into a commercially available pLenti4/V5-DEST vector by LR recombinant reaction, resulting in expression vectors pLenti4/V5-EX-hOAT1 and pLenti4/V5-EX-hOAT3. The inducible CMV-TetO2 promoter was replicated from pcDNA5-FRT-TO (Invitrogen) using two primers, one primer that introduces a ClaI restriction site (forward Cla1-CMV-TetO2: GCCGCCATCGATGCCGCCGTTGACATTGATTATTGACT - SEQ ID NO: 27) and one primer that introduces an EcoRI restriction site (reverse EcoRI-CMV-TetO2: GGCGGCGAATTCGGCGGCCGGAGGCTGGATCGGTCCCGG - SEQ ID NO: 28). The resulting PCR product (ClaI-CMV-TetO2-EcoRI) was purified using the High Pure PCR Product Purification kit (Roche, Basel, Switzerland). Both PCR product and expression vectors were digested by ClaI and EcoRI (New England Biolabs, Ipswich, USA) for 1 hour at 37° C and, after purification, ligation was performed with a 1:3 (insert:vector) unit ratio using T4 ligase (Invitrogen) for 2 h at 37° C, resulting in the pLenti expression constructs (pLenti4/V5-EX-CMV-TetO2-hOAT1 (SEQ ID NO: 25) and pLenti4/V5-EX-CMV-TetO2-hOAT3 (SEQ ID NO: 26)).

### OAT transduction in ciPTEC

To obtain lentiviral particles containing the OAT constructs, lentiviral stock was produced by transfecting the pLenti expression constructs with packaging plasmid mix into the HEK293FT cell line using ViraPower Lentiviral Gateway Expression Systems (Invitrogen), according to the manufacturer's instructions. ciPTEC were cultured to 50-70% confluency and exposed to lentiviral particles for 24 h. Both ciPTEC-OAT1 and ciPTEC-OAT3 were selected and subcloned to obtain homogeneous cell populations. To this end, transduced ciPTEC-OAT3 cells were plated into 3 separate culture flasks (100, 300 and 900 cells) containing irradiated (30 Gy) 3T3-cells as described (Saleem et al., 2002). After 2-3 weeks, single cell colonies of ciPTEC-OAT3 were picked and cultured. Transduction efficiency for ciPTEC-OAT1 was too low for immediate subcloning. Therefore, the heterogeneous cell population was enriched by positive selection of fluorescein transporting cells. Only successfully transduced ciPTEC express functional OAT; hence, positive selection could be performed upon exposure to fluorescein, which is an OAT substrate, using a BD FACSAria SORP flow cytometer (BD biosciences, San Jose, USA). 20 million ciPTEC-OAT1 cells were suspended in Hank's Balanced Salt Solution (HBSS, Invitrogen) containing 1 µM fluorescein and incubated for 10 min at 37° C before fluorescence-activated cell sorting (FACS). Enriched ciPTEC-OAT1 cells were subcloned as described for ciPTEC-OAT3. Both ciPTEC-OAT1 and ciPTEC-OAT3 were cultured for up to 30 passages after transduction to study stability of OAT 1 and OAT3 expression.

### OAT-mediated fluorescein uptake

To evaluate OAT transporter function and to evaluate the inhibition properties of several known OAT substrates, fluorescein uptake was measured by flow cytometry and multiplate reader assays. Mature monolayers of sub-cloned ciPTEC were co-incubated with fluorescein (1 µM, unless stated otherwise) and a test compound in HBSS for 10 min at 37° C. Compounds known for their inhibitory effect on OAT-mediated transport were tested. The following is a list of tested compounds: para-aminohippuric acid (PAH), estrone sulfate, probenecid, furosemide, cimetidine, diclofenac, adefovir, cidofovir, tenofovir, and zidovudine. The organic cation metformin was included as a negative control. All chemicals were obtained from Sigma, unless stated otherwise. Uptake was stopped by washing 3 times with cold HBSS (4° C). For flow cytometry, samples were harvested following fluorescein exposure using trypsin-EDTA, then washed, fixed using 0.5% paraformaldehyde, and measured using FACS calibur (Becton Dickinson, Franklin Lakes, USA). For 96 well plate assay, cells were lysed by 200 µl 0.1 M NaOH for 10 min at 37° C and fluorescence was measured (excitation 485 nm, emission 535 nm) using the multiplate reader Victor X3 (Perkin Elmer, Waltham, USA).

### Viability assays

To evaluate toxicity induced by antivirals, viability of ciPTEC was evaluated by an MTT assay (Moghadasali et al., 2013). Briefly, monolayers of ciPTEC (96-wells) were exposed to antivirals in serum-free medium (SFM) on day 6 of maturation. Cell toxicity was analyzed further in presence of multidrug resistance protein (MRP) efflux inhibitor MK571 (5 µM) and breast cancer resistance protein (BCRP) efflux inhibitor KO143 (10 µM). After incubation for 24, 48 and 72 h at 37° C, ciPTEC were washed and incubated with 0.5 mg/ml thiazolyl blue tetrazolium bromide (MTT, Sigma) for 3 h at 37° C in absence of antivirals. Formazan crystals formed in viable cells were dissolved in dimethyl sulfoxide (DMSO, Merck, Whitehouse Station, USA) and optical density was measured (560 nm, background at 670 nm was subtracted) using Benchmark Plus (BioRad, Hercules, USA).

### Gene expressions in ciPTEC

Total RNA was isolated from matured ciPTEC (6-well plates) using TRIzol (Life Technologies Europe BV) and chloroform extraction. cDNA was synthesised using M-MLV Reverse Transcriptase (Promega, Madison, USA), according to the manufacturer's instructions. The mRNA expression levels were evaluated using gene-specific primer-probe sets obtained from Life Technologies: OAT1 (SLC22A6, hs00537914), OAT3 (SLC22A8, hs00188599), GAPDH (hs99999905) and TaqMan Universal PCR Master Mix (Applied Biosystems). The quantitative PCR reactions were performed using CFX96-Touch Real Time PCR System (BioRad) and analyzed using BioRad CFX Manager (version 1.6). Fold differences in mRNA-levels for ciPTEC-OAT1 and ciPTEC-OAT3 were calculated using GAPDH as a reference gene and normalized to parent ciPTEC.

### Data analysis

A Michaelis-Menten equation was combined with linear diffusion to fit fluorescein uptake data after background subtraction with GraphPad Prism (version 5.03). For calculation of IC₅₀ values, log (concentration inhibitor) versus fluorescein uptake was plotted after background subtraction using GraphPad Prism.

For MTT and fluorescein inhibition assays, data were normalized to the viability or activity of untreated control cells. Non-linear regression with variable slope constraining the top to 100% was used to fit the data after background subtraction with GraphPad Prism. Statistics was performed by two-way ANOVA (two-tailed, α=0.05) using GraphPad Prism as well. All data is presented as mean±SEM of at least three separate experiments (n=3) performed in triplicate, unless stated otherwise.

### Example 2. - Functional OAT expression in ciPTEC

The absence of endogenous OAT1 and OAT3 expression in ciPTEC was demonstrated by exposure to fluorescein (1 µM) for 10 min, which did not increase the intracellular fluorescence intensity as measured by flow cytometry (Figure 1B, dashed line). Therefore, OAT transporters were introduced separately by lentiviral transduction. A schematic overview of the experimental approach is provided in Figure 1A. The transporter genes SLC22A6 and SLC22A8 were cloned under regulation of a CMV promoter and a TetO2 site to conditionally induce their expression. Remarkably, basal expression and function upon transduction of both OAT transporters was positive without tetracycline induction, and was not influenced by this inducer (data not shown). Fluorescein uptake capacity (without induction by tetracycline) was used to discriminate between successfully transduced cells and non-transduced cells, reflected by two subpopulations in the flow cytometer histograms (ciPTEC-OAT1, Figure 1C, ciPTEC-OAT3, Figure 1D). When exposed to 1 µM fluorescein for 10 min, a small cell population accumulated the fluorescent substrate, which was immediately selected using FACS. The fraction of OAT1 positive cells selected (Figure IE) accounted for only 8.3% of the total population, suggesting that a down-regulation in OAT expression upon culturing can be part of a survival mechanism. The enriched population accumulated fluorescein efficiently, and was sensitive to inhibition by para-aminohippuric acid, a known OAT1 substrate and/or inhibitor (Figure IF). The ciPTEC-OAT1 population enriched by FACS and the non-enriched ciPTEC-OAT3 population were subcloned to obtain homogeneous cell populations with high functional OAT transporter expression, demonstrated by qPCR. Expression of OAT1 and OAT3 in the respective cell lines was semi-quantified in relation to GAPDH expression and appeared to be 0.8±0.1 for ciPTEC-OAT1 and 0.09±0.01 for ciPTEC-OAT3, which was comparable to the relative levels in human kidney tissue homogenates (1.0±0.1 and 0.2±0.01 for OAT1 and OAT3, respectively; experiments performed in duplicate). Intact tubular phenotype was further demonstrated by functionally active OCT2, for which a drug-interaction with cimetidine was shown to be similar to the parent cell line (Figure 2B).

### Example 3 - Drug-interaction at the site of OAT1 and OAT3

Pharmacokinetics of OAT-mediated fluorescein transport was investigated by studying the time- and concentration-dependent uptake of the substrate. Fluorescein uptake demonstrated partial saturation in OAT1 and OAT3 expressing cells (Figure 3A, B and D) for which a Kₘ and a Vₘₐₓ value were determined, taking a passive diffusion component k_{d} into account (Table 1). Fluorescein affinity was approximately 5-fold higher for OAT1 than for OAT3. Upon fluorescein exposure (10 min, 1 µM), confocal fluorescent imaging confirmed uptake in ciPTEC-OAT1 and ciPTEC-OAT3 (Figure 3C and E). To demonstrate that the uptake was indeed transporter mediated, specific inhibition of fluorescein uptake in the presence of two concentrations of para-aminohippuric acid (10 µM or 100 µM) or estrone sulfate (3 µM or 100 µM) in ciPTEC-OAT1 and ciPTEC-OAT3 respectively, was studied (Figure 3B and 3D). CiPTEC-OAT1 and ciPTEC-OAT3 were validated further by determination of IC₅₀ values using concentration-dependent inhibition of fluorescein uptake in presence of any one of para-aminohippuric acid, estrone sulfate, probenecid, furosemide, cimetidine, or diclofenac (Figure 4, Table 2). Overall, IC₅₀ values calculated in these models are in close agreement with previously reported values (Table 2). Further confirmation of specificity was obtained by using metformin, which did not affect OAT-mediated fluorescein uptake in both ciPTEC-OAT1 and ciPTEC-OAT3, as metformin is an OCT substrate and not an OAT substrate (Kimura et al., 2005). The experiments depicted in Figure 4 were performed in cells spanning 29 passages after transduction. The small variations in this data and the maintained fluorescein uptake both indicate stable transduction and high robustness of transporter function in ciPTEC-OAT1 and in ciPTEC-OAT3.

### Example 4 - OATs mediate antiviral-induced toxicity

Toxicity of antivirals was reported to be associated with renal tubular uptake mediated by OAT1 and OAT3 (Kohler et al., 2011; Takeda et al., 2002; Ciglar et al., 1999). Therefore, the effects of antivirals on OAT function and on cell viability was investigated upon drug exposures. Concentration-dependent inhibition of fluorescein uptake via OAT1 was observed by adefovir, cidofovir, tenofovir and zidovudine, while OAT3 was only associated with zidovudine-fluorescein interactions (Figure 5, Table 3). Next, the DDI indices were determined. The United States Food and Drug Administration (FDA) draft DDI guideline (Huang & Zhang, 2012) states that a ratio between unbound plasma concentration and IC₅₀ (C_{max,u} /IC₅₀) higher than 0.1 corresponds to a high chance of clinical drug interaction and a low potential for false negative results. For adefovir, cidofovir, and zidovudine, the IC₅₀ value was less than 10 times the maximal free plasma concentration (C_{max,u}/IC₅₀>0.1), and, therefore, at clinically relevant plasma concentrations, inhibition of OAT1 is likely and DDI with OAT1 transporter substrates were defined as clinically relevant in this study.

Next, cytotoxicity caused by all four antivirals was evaluated after exposure of ciPTEC to the drugs for 24-72 h. As a measure of cytotoxicity, cell viability was analyzed by cellular dehydrogenase capacity, metabolizing MTT into purple formazan. In the parent ciPTEC, viability was not affected by any of the antivirals (48 hr, 1 mM), while adefovir, cidofovir and tenofovir significantly affected cell viability in ciPTEC-OATl and only tenofovir slightly decreased ciPTEC-OAT3 viability (Figure 6A). Antiviral-induced toxicity was evaluated in more detail, demonstrating a concentration- and time-dependent decrease in viability by adefovir, cidofovir and tenofovir in ciPTEC-OATl, while the effect was less pronounced in ciPTEC-OAT3 (Figure 6B and Table 4). These findings indicate the direct involvement of the OAT transporters in antiviral-mediated nephrotoxicity, although IC₅₀ values found in the current study are higher compared to those obtained in previous studies (Table 4). In this regard, it should be noted that the present system involves a highly relevant set of functional transporters, whereas previous studies often only overexpressed OATs or at least did not express all transporters that are active in ciPTEC-OAT cells, which makes ciPTEC-OAT a more relevant model system. The cytotoxic effect of the antivirals correlated nicely with the inhibitory effect on fluorescein uptake shown in Figure 5, except for zidovudine. Despite a clear inhibition of fluorescein uptake by zidovudine, which suggests OAT-mediated uptake, this compound did not affect cell viability as determined by the MTT assay. To investigate a potential protective effect via intact efflux transporters in ciPTEC, cells were exposed to zidovudine at 10x Cₘₐₓ (50 µM) in the presence of the MRP4 inhibitor MK571, and the BCRP inhibitor KO143. This did not affect cell viability in ciPTEC, ciPTEC-OATl, or ciPTEC-OAT3, indicating that efflux transporters did not counteract intracellular exposure to zidovudine, thus that efflux transporters did not reduce the cytotoxic potential of zidovudine.

Antiviral-induced nephrotoxicity was shown to be associated with OAT-mediated uptake and further evaluated in the present disclosure (Izzedine et al., 2009; Kohler et al., 2011; Cihlar et al., 2009; Zhang et al., 2015). It is demonstrated here that OAT1 or OAT3 expression is required for induction of toxicity by adefovir, cidofovir and tenofovir in ciPTEC. The relation between OAT1 transporter affinity and toxicity was described earlier using HeLa cells that transiently expressed hOAT1, in which cidofovir showed a higher affinity as well as a higher toxicity compared to tenofovir (Mandikova et al., 2013). In agreement, when the cytotoxic potential of NtRTIs in ciPTEC-OATl at 72 h of exposure was ranked, it was found that cidofovir has a higher potency compared to tenofovir and adefovir (Cihlar et al., 2009; Zhang et al., 2015). On the other hand, the low potency of adefovir shown in the present disclosure contrasts to the cytotoxicity reported for other cell models (Khamdang et al., 2004; Wang & Sweet, 2012). In general, the toxic potency of the antivirals in ciPTEC is lower as compared to hOAT1-CHO and HEK-OAT1, possibly due to the presence of functional metabolic enzymes and an intact efflux machinery in ciPTEC (Cihlar et al., 2009; Zhang et al., 2015; Imaoka et al., 2007). Activity of phase I and phase II metabolizing enzymes was demonstrated in ciPTEC of which the UGT2B7 subfamily is a possible cause of the tolerance for zidovudine observed in the present study (Mutsaers et al., 2013). While adefovir, cidofovir and tenofovir are largely excreted unchanged by the kidneys, only 23% of zidovudine is eliminated via the urine without metabolic alterations (Varma et al., 2009). Zidovudine undergoes either phase II metabolism into the non-toxic 5'-zidovudine-O-glucuronide or the antiviral is phosphorylated resulting in mitochondrial toxicity (Blum et al., 1988; Lewis et al., 2003). As both glucuronidation and phosphorylation take place at the same functional group of zidovudine (5'-OH), the low toxicity of zidovudine suggests a favour for glucuronidation in ciPTEC. Although glucuronidation predominantly takes place in the liver, UGT2B7 expression in ciPTEC likely contributes to zidovudine detoxification. The efflux pumps MRP4 and BCRP in ciPTEC, which can minimize intracellular exposure, appeared to be of minor importance, as efflux inhibition did not reduce viability of ciPTEC upon antiviral exposure.

### Example 5 - Regulated OAT expression in ciPTEC

### Results and conclusion

Epidermal growth factor (EGF) dependent regulation of OAT1 drug transport expression and activity was determined in a representative ciPTEC-OATl (ciPTEC.OAT1.4B2 DSM ACC3279). The expression of OAT1 was significantly increased in the presence of EGF (Figure 7A). As a result, the uptake of fluorescein (an OAT1 substrate) was significantly increased upon EGF exposure (calculated Vmax of 14.6 vs 9.7 A.U.; Fig 7B). These data depict that OAT1 expression and transport activity is regulated in ciPTEC-OATl by EGF, likely via the EGF receptor. These data demonstrates that the human renal background of ciPTEC-OATl provides a physiological relevant model with intact regulation of OAT transport.

### METHODS

### Cell culture and EGF exposure

ciPTEC-OATl. 4B2 DSM ACC3279 was cultured in phenol red-free DMEM/F12 (Invitrogen, Breda, The Netherlands), as described in example 1 here above. Cells were seeded at a density of 63,000 cell/cm², cultured for 24 hours at 33°C and subsequently at 37°C for 7 days. To study the effects of EGF, matured ciPTEC-OATl were treated for 48 hours in the presence or absence of EGF (10 ng/mL).

### Fluorescein uptake

The uptake of fluorescein was used to determine the changes in activity of OAT1 mediated transport. Cells were washed twice before incubation at 37°C for 10 minutes with fluorescein. After incubation, plates were washed twice and cells were lysed with 0.1 M NaOH. Subsequently, intracellular fluorescence was measured via an Ascent Fluoroskan FL microplate reader (excitation: 494 nm, emission: 512 nm). To calculate Vmax, a Michaelis-Menten equation was combined with linear diffusion to fit fluorescein uptake data after background subtraction with GraphPad Prism (version 5.03).

### Gene expression

OAT1 gene expression profiling was performed by isolating total RNA from cells grown in six-well plates, using an RNeasy Mini kit (Qiagen, Venlo, The Netherlands), according to the manufacturers specifications. Subsequently, cDNA was synthesized using the Omniscript RT-kit (Qiagen). Subsequently, quantitative PCR was performed in a CFX96 Real-Time PCR detection system (Bio-rad, Veenendaal, The Netherlands) according to the manufacturers conditions. GAPDH was used as reference gene for normalization and relative expression levels were calculated as fold change using the 2^{-ΔΔCT} method. The primer-probe sets for the quantitative PCR were obtained from Applied Biosystems: GAPDH - hs99999905_m1 and OAT1 - hs00537914.

### REFERENCE LIST

1. Tiong, H. Y. et al. Drug-induced nephrotoxicity: clinical impact and preclinical in vitro models. Molecular pharmaceutics 11, 1933-1948, doi:10.1021/mp400720w (2014).
2. Redfern, W. S. et al. Impact and frequency of different toxicities throughout the pharmaceutical life cycle. The Toxicologist, 1081 (2010).
3. Guengerich, F. P. Mechanisms of drug toxicity and relevance to pharmaceutical development. Drug metabolism and pharmacokinetics 26, 3-14 (2011).
4. Gundert-Remy, U. et al. Extrahepatic metabolism at the body's internal-external interfaces. Drug metabolism reviews 46, 291-324, doi:10.3109/03602532.2014.900565 (2014).
5. Wang, L. & Sweet, D. H. Renal organic anion transporters (SLC22 family): expression, regulation, roles in toxicity, and impact on injury and disease. The AAPS journal 15, 53-69, doi:10.1208/s12248-012-9413-y (2013).
6. Burckhardt, G. & Burckhardt, B. C. In vitro and in vivo evidence of the importance of organic anion transporters (OATs) in drug therapy. Handbook of experimental pharmacology, 29-104, doi:10.1007/978-3-642-14541-4_2 (2011).
7. Masereeuw, R. & Russel, F. G. Therapeutic implications of renal anionic drug transporters. Pharmacology & therapeutics 126, 200-216, doi:10.1016/j.pharmthera.2010.02.007 (2010).
8. Motohashi, H. & Inui, K. Organic cation transporter OCTs (SLC22) and MATEs (SLC47) in the human kidney. The AAPS journal 15, 581-588, doi:10.1208/s12248-013-9465-7 (2013).
9. Konig, J., Muller, F. & Fromm, M. F. Transporters and drug-drug interactions: important determinants of drug disposition and effects. Pharmacological reviews 65, 944-966, doi:10.1124/pr.113.007518 (2013).
10. Izzedine, H., Harris, M. & Perazella, M. A. The nephrotoxic effects of HAART. Nature reviews. Nephrology 5, 563-573, doi:10.1038/nrneph.2009.142 (2009).
11. Lewis, W., Day, B. J. & Copeland, W. C. Mitochondrial toxicity of NRTI antiviral drugs: an integrated cellular perspective. Nature reviews. Drug discovery 2, 812-822, doi:10.1038/nrd1201 (2003).
12. De Clercq, E. Antiviral drugs in current clinical use. Journal of clinical virology : the official publication of the Pan American Society for Clinical Virology 30, 115-133, doi:10.1016/j.jcv.2004.02.009 (2004).
13. Tourret, J., Deray, G. & Isnard-Bagnis, C. Tenofovir effect on the kidneys of HIV-infected patients: a double-edged sword? Journal of the American Society of Nephrology : JASN 24, 1519-1527, doi:10.1681/ASN.2012080857 (2013).
14. Kohler, J. J. et al. Tenofovir renal proximal tubular toxicity is regulated by OAT1 and MRP4 transporters. Laboratory investigation; a journal of technical methods and pathology 91, 852-858, doi:10.1038/labinvest.2011.48 (2011).
15. Takeda, M. et al. Human organic anion transporters and human organic cation transporters mediate renal antiviral transport. The Journal of pharmacology and experimental therapeutics 300, 918-924 (2002).
16. Cihlar, T. et al. The antiviral nucleotide analogs cidofovir and adefovir are novel substrates for human and rat renal organic anion transporter 1. Molecular pharmacology 56, 570-580 (1999).
17. Lacy, S. A., Hitchcock, M. J., Lee, W. A., Tellier, P. & Cundy, K. C. Effect of oral probenecid coadministration on the chronic toxicity and pharmacokinetics of intravenous cidofovir in cynomolgus monkeys. Toxicological sciences : an official journal of the Society of Toxicology 44, 97-106, doi:10.1006/toxs.1998.2481 (1998).
18. Kearney, B. P., Flaherty, J. F. & Shah, J. Tenofovir disoproxil fumarate: clinical pharmacology and pharmacokinetics. Clinical pharmacokinetics 43, 595-612, doi:10.2165/00003088-200443090-00003 (2004).
19. Vigouroux, C., Bastard, J. P. & Capeau, J. Emerging clinical issues related to management of multiorgan comorbidities and polypharmacy. Current opinion in HIV and AIDS 9, 371-378, doi:10.1097/COH.0000000000000068 (2014).
20. Wilmer, M. J. et al. Novel conditionally immortalized human proximal tubule cell line expressing functional influx and efflux transporters. Cell and tissue research 339, 449-457, doi:10.1007/s00441-009-0882-y (2010).
21. Schophuizen, C. M. et al. Cationic uremic toxins affect human renal proximal tubule cell functioning through interaction with the organic cation transporter. Pflugers Archiv : European journal of physiology 465, 1701-1714, doi:10.1007/s00424-013-1307-z (2013).
22. Mutsaers, H. A. et al. Uremic toxins inhibit renal metabolic capacity through interference with glucuronidation and mitochondrial respiration. Biochimica et biophysica acta 1832, 142-150, doi:10.1016/j.bbadis.2012.09.006 (2013).
23. Jansen, J. et al. A morphological and functional comparison of proximal tubule cell lines established from human urine and kidney tissue. Experimental cell research 323, 87-99, doi:10.1016/j.yexcr.2014.02.011 (2014).
24. Saleem, M. A. et al. A conditionally immortalized human podocyte cell line demonstrating nephrin and podocin expression. Journal of the American Society of Nephrology : JASN 13, 630-638 (2002).
25. Moghadasali, R. et al. Mesenchymal stem cell-conditioned medium accelerates regeneration of human renal proximal tubule epithelial cells after gentamicin toxicity. Experimental and toxicologic pathology : official journal of the Gesellschaft fur Toxikologische Pathologie 65, 595-600, doi:10.1016/j.etp.2012.06.002 (2013).
26. Kimura, N. et al. Metformin is a superior substrate for renal organic cation transporter OCT2 rather than hepatic OCT1. Drug metabolism and pharmacokinetics 20, 379-386 (2005).
27. Huang, S. H., Zhang, L. Guidance for Industry (ed Health and Human Services) 75 (Silver Spring, MD, 2012).
28. Cihlar, T. & Ho, E. S. Fluorescence-based assay for the interaction of small molecules with the human renal organic anion transporter 1. Analytical biochemistry 283, 49-55, doi:10.1006/abio.2000.4633 (2000).
29. Mandikova, J. et al. Interactions with selected drug renal transporters and transporter-mediated cytotoxicity in antiviral agents from the group of acyclic nucleoside phosphonates. Toxicology 311, 135-146, doi:10.1016/j.tox.2013.07.004 (2013).
30. Lash, L. H., Putt, D. A. & Cai, H. Membrane transport function in primary cultures of human proximal tubular cells. Toxicology 228, 200-218, doi:10.1016/j.tox.2006.08.035 (2006).
31. Brown, C. D. et al. Characterisation of human tubular cell monolayers as a model of proximal tubular xenobiotic handling. Toxicology and applied pharmacology 233, 428-438, doi:10.1016/j.taap.2008.09.018 (2008).
32. Wieser, M. et al. hTERT alone immortalizes epithelial cells of renal proximal tubules without changing their functional characteristics. American journal of physiology. Renal physiology 295, F1365-1375, doi:10.1152/ajprenal.90405.2008 (2008).
33. Aschauer, L., Carta, G., Vogelsang, N., Schlatter, E. & Jennings, P. Expression of xenobiotic transporters in the human renal proximal tubule cell line RPTEC/TERT1. Toxicology in vitro : an international journal published in association with BIBRA, doi:10.1016/j.tiv.2014.12.003 (2014).
34. Khamdang, S. et al. Interactions of human- and rat-organic anion transporters with pravastatin and cimetidine. Journal of pharmacological sciences 94, 197-202 (2004).
35. Cihlar, T. et al. Novel nucleotide human immunodeficiency virus reverse transcriptase inhibitor GS-9148 with a low nephrotoxic potential: characterization of renal transport and accumulation. Antimicrobial agents and chemotherapy 53, 150-156, doi:10.1128/AAC.01183-08 (2009).
36. Wang, L. & Sweet, D. H. Potential for food-drug interactions by dietary phenolic acids on human organic anion transporters 1 (SLC22A6), 3 (SLC22A8), and 4 (SLC22A11). Biochemical pharmacology 84, 1088-1095, doi:http://dx.doi.org/10.1016/j.bcp.2012.07.027 (2012).
37. Zhang, X., Wang, R., Piotrowski, M., Zhang, H. & Leach, K. L. Intracellular concentrations determine the cytotoxicity of adefovir, cidofovir and tenofovir. Toxicology in Vitro 29, 251-258, doi:http://dx.doi.org/10.1016/j.tiv.2014.10.019 (2015).
38. Imaoka, T. et al. Functional involvement of multidrug resistance-associated protein 4 (MRP4/ABCC4) in the renal elimination of the antiviral drugs adefovir and tenofovir. Molecular pharmacology 71, 619-627, doi:10.1124/mol.106.028233 (2007).
39. Varma, M. V. et al. Physicochemical determinants of human renal clearance. Journal of medicinal chemistry 52, 4844-4852, doi:10.1021/jm900403j (2009).
40. Blum, M. R., Liao, S. H., Good, S. S. & de Miranda, P. Pharmacokinetics and bioavailability of zidovudine in humans. The American journal of medicine 85, 189-194 (1988).
41. Nigam, S. K. What do drug transporters really do? Nature reviews. Drug discovery 14, 29-44, doi:10.1038/nrd4461 (2015).
42. EMA. (ed European Medicines Agency) 38 (London, UK, 2010).
43. International Transporter, C. et al. Membrane transporters in drug development. Nature reviews. Drug discovery 9, 215-236, doi:10.1038/nrd3028 (2010).
44. McGuinness, L. CRACK IT Challenge winners awarded £4.9 million to further their research <https://www.nc3rs.org.uk/crackit-news/crack-it-challenge-winners-awarded-%C2%A349-million-further-their-research> (2014).
45. Chu, X., Bleasby, K. & Evers, R. Species differences in drug transporters and implications for translating preclinical findings to humans. Expert opinion on drug metabolism & toxicology 9, 237-252, doi:10.1517/17425255.2013.741589 (2013).
46. Bhatia, S. N. & Ingber, D. E. Microfluidic organs-on-chips. Nature biotechnology 32, 760-772, doi:10.1038/nbt.2989 (2014).
47. Jung, K. Y. et al. Characterization of ochratoxin A transport by human organic anion transporters. Life sciences 69, 2123-2135 (2001).
48. Deguchi, T. et al. Characterization of uremic toxin transport by organic anion transporters in the kidney. Kidney international 65, 162-174, doi:10.1111/j.1523-1755.2004.00354.x (2004).
49. Srimaroeng, C., Jutabha, P., Pritchard, J. B., Endou, H. & Chatsudthipong, V. Interactions of stevioside and steviol with renal organic anion transporters in S2 cells and mouse renal cortical slices. Pharmaceutical research 22, 858-866, doi:10.1007/s11095-005-4580-5 (2005).
50. Cha, S. H. et al. Identification and characterization of human organic anion transporter 3 expressing predominantly in the kidney. Molecular pharmacology 59, 1277-1286 (2001).
51. Chu, X. Y. et al. Transport of the dipeptidyl peptidase-4 inhibitor sitagliptin by human organic anion transporter 3, organic anion transporting polypeptide 4C1, and multidrug resistance P-glycoprotein. The Journal of pharmacology and experimental therapeutics 321, 673-683, doi:10.1124/jpet.106.116517 (2007).
52. Hasannejad, H. et al. Interactions of human organic anion transporters with diuretics. The Journal of pharmacology and experimental therapeutics 308, 1021-1029, doi:10.1124/jpet.103.059139 (2004).
53. Motohashi, H., Uwai, Y., Hiramoto, K., Okuda, M. & Inui, K. Different transport properties between famotidine and cimetidine by human renal organic ion transporters (SLC22A). European journal of pharmacology 503, 25-30, doi:10.1016/j.ejphar.2004.09.032 (2004).
54. Khamdang, S. et al. Interactions of human organic anion transporters and human organic cation transporters with nonsteroidal anti-inflammatory drugs. The Journal of pharmacology and experimental therapeutics 303, 534-539, doi:10.1124/jpet.102.037580 (2002).
55. Mulato, A. S., Ho, E. S. & Cihlar, T. Nonsteroidal anti-inflammatory drugs efficiently reduce the transport and cytotoxicity of adefovir mediated by the human renal organic anion transporter 1. The Journal of pharmacology and experimental therapeutics 295, 10-15 (2000).
56. Barditch-Crovo, P. et al. Anti-human immunodeficiency virus (HIV) activity, safety, and pharmacokinetics of adefovir dipivoxil (9-[2-(bis-pivaloyloxymethyl)-phosphonylmethoxyethyl]adenine) in HIV-infected patients. The Journal of infectious diseases 176, 406-413 (1997).
57. Cundy, K. C. et al. Clinical pharmacokinetics of adefovir in human immunodeficiency virus type 1-infected patients. Antimicrobial agents and chemotherapy 39, 2401-2405 (1995).
58. Wachsman, M. et al. Pharmacokinetics, safety and bioavailability of HPMPC (cidofovir) in human immunodeficiency virus-infected subjects. Antiviral research 29, 153-161 (1996).
59. Cundy, K. C. et al. Clinical pharmacokinetics of cidofovir in human immunodeficiency virus-infected patients. Antimicrobial agents and chemotherapy 39, 1247-1252 (1995).
60. Kearney, B. P., Ramanathan, S., Cheng, A. K., Ebrahimi, R. & Shah, J. Systemic and renal pharmacokinetics of adefovir and tenofovir upon coadministration. Journal of clinical pharmacology 45, 935-940, doi:10.1177/0091270005278949 (2005).
61. Flynn, P. M. et al. Pharmacokinetics and safety of single-dose tenofovir disoproxil fumarate and emtricitabine in HIV-1-infected pregnant women and their infants. Antimicrobial agents and chemotherapy 55, 5914-5922, doi:10.1128/aac.00544-11 (2011).
62. Moore, K. H. et al. Pharmacokinetics and bioavailability of zidovudine and its glucuronidated metabolite in patients with human immunodeficiency virus infection and hepatic disease (AIDS Clinical Trials Group protocol 062). Antimicrobial agents and chemotherapy 39, 2732-2737 (1995).
63. Ahlin G, Hilgendorf C, Karlsson J, Szigyarto CA, Uhlen M, Artursson P (2009) Endogenous gene and protein expression of drug-transporting proteins in cell lines routinely used in drug discovery programs. Drug Metab Dispos 37:2275-2283
64. J.H. Miller, Sodium-sensitive, probenecid-insensitive p-amino-hippuric acid uptake in cultured renal proximal tubule cells of the rabbit, Proc. Soc. Exp. Biol. Med. 199 (1992) 298-304.

## Claims

1. A human proximal tubule epithelial cell (PTEC) that stably expresses a functional organic anion transporter (OAT) when cultured, wherein the cell is ciPTEC.OAT1.4B2 DSM ACC3279 or a passage up to the 29^{th} passage thereof or wherein the cell is ciPTEC.OAT3.3C1 DSM ACC3280 or a passage up to the 29th passage thereof.

2. A method for the production of a human proximal tubule epithelial cell that stably expresses an organic anion transporter when cultured, said method comprising:
i) transducing a population of proximal tubule epithelial cells by a lentiviral particle comprising an expression construct that comprises a nucleotide sequence having at least 50% sequence identity with SEQ ID NO: 2 or with SEQ ID NO: 4,
ii) optionally enriching the transduced population obtained in (i), preferably by using fluorescence activated cell sorting (FACS), and
iii) subcloning the transduced population obtained in (i) of (ii) by selecting and isolating single cells and expanding these by culture
wherein stable expression of the organic anion transporter is expression that leads to the detectable presence or activity of the organic anion transporter for at least ten passages of said cell.

3. The method according to claim 2, wherein said expression construct has at least 50% sequence identity with an expression construct selected from the group consisting of:
i) pLenti4/V5-EX-CMV-TetO2-hOAT1 (SEQ ID NO: 25), and
ii) pLenti4/V5-EX-CMV-TetO2-hOAT3 (SEQ ID NO: 26).

4. An *in vitro* or *ex vivo* method for analysis of a substance, comprising contacting said substance with at least one cell according to claim 1, preferably with a mature monolayer of said cells.

5. The method according to claim 4, wherein said method is for determining the nephrotoxicity of said substance, and preferably further comprises the subsequent analysis of cell viability, preferably by analysis of cellular dehydrogenase capacity.

6. The method according to claim 4, wherein said method is for the functional analysis of the interaction of said substance with a transporter, preferably a renal transporter, and wherein said contacting preferably is in the presence of a labeled anionic transporter substrate, preferably a radiolabeled or a fluorescently labeled anionic transporter substrate.

7. The method according to any one of claims 4 or 6, wherein said method further comprises determining the drug-drug interaction of said substance.

8. The method according to any one of claims 4, 6, or 7, wherein said method further comprises determining whether said substance is a substrate or an inhibitor of a transporter involved in a clinically relevant drug-drug interaction.

9. A kit of parts comprising a cell as described in claim 1 and instructions for use.

## Patentansprüche

1. Humane proximale Tubulusepithelzelle (PTEC), die bei der Kultivierung einen funktionellen organischen Anionentransporter (OAT) stabil exprimiert, wobei die Zelle ciPTEC.OAT1.4B2 DSM ACC3279 oder eine Passage bis zu ihrer 29. Passage ist oder wobei die Zelle ciPTEC.OAT3.3C1 DSM ACC3280 oder eine Passage bis zu ihrer 29. Passage ist.

2. Verfahren zum Herstellen einer humanen proximalen Tubulusepithelzelle, die bei der Kultivierung einen organischen Anionentransporter stabil exprimiert, wobei das Verfahren Folgendes umfasst:
i) Transduzieren einer Population von proximalen Tubulusepithelzellen durch ein lentivirales Partikel, umfassend ein Expressionskonstrukt, das eine Nukleotidsequenz mit mindestens 50%iger Sequenzidentität mit SEQ ID NO: 2 oder mit SEQ ID NO: 4 umfasst,
ii) optional Anreichern der in (i) erhaltenen transduzierten Population, vorzugsweise unter Verwendung der fluoreszenzaktivierten Zellsortierung (FACS), und
iii) Subklonieren der in (i) von (ii) erhaltenen transduzierten Population durch Auswählen und Isolieren einzelner Zellen und Expandieren dieser durch die Kultur,
wobei die stabile Expression des organischen Anionentransporters eine Expression ist, die zu der nachweisbaren Präsenz oder Aktivität des organischen Anionentransporters für mindestens zehn Passagen der Zelle führt.

3. Verfahren nach Anspruch 2, wobei das Expressionskonstrukt mindestens 50 % Sequenzidentität mit einem Expressionskonstrukt aufweist, das aus der Gruppe ausgewählt ist, bestehend aus:
i) pLenti4/V5-EX-CMV-TetO2-hOAT1 (SEQ ID NO: 25), und
ii) pLenti4/V5-EX-CMV-TetO2-hOAT3 (SEQ ID NO: 26).

4. *In-vitro-* oder *ex-vivo-*Verfahren zum Analysieren einer Substanz, umfassend das Inkontaktbringen der Substanz mit mindestens einer Zelle nach Anspruch 1, vorzugsweise mit einer reifen Monoschicht der Zellen.

5. Das Verfahren nach Anspruch 4, wobei das Verfahren zum Bestimmen der Nephrotoxizität der Substanz dient und vorzugsweise weiterhin das anschließende Analysieren der Zelllebensfähigkeit, vorzugsweise durch das Analysieren der zellulären Dehydrogenasekapazität, umfasst.

6. Das Verfahren nach Anspruch 4, wobei das Verfahren zum funktionellen Analysieren der Wechselwirkung der Substanz mit einem Transporter, vorzugsweise einem renalen Transporter, dient, und wobei das Inkontaktbringen vorzugsweise in Gegenwart eines markierten anionischen Transportersubstrats, vorzugsweise eines radioaktiv markierten oder eines fluoreszenzmarkierten anionischen Transportersubstrats, erfolgt.

7. Das Verfahren nach einem der Ansprüche 4 oder 6, wobei das Verfahren ferner das Bestimmen der Arzneimittelwechselwirkung der Substanz umfasst.

8. Das Verfahren nach einem der Ansprüche 4, 6 oder 7, wobei das Verfahren ferner das Bestimmen umfasst, ob die Substanz ein Substrat oder ein Inhibitor eines Transporters ist, der an einer klinisch relevanten Arzneimittelwechselwirkung beteiligt ist.

9. Teilesatz, umfassend eine Zelle nach Anspruch 1 und eine Gebrauchsanweisung.

## Revendications

1. Cellule épithéliale de tubule proximal humain (PTEC) qui exprime de manière stable un transporteur d'anions organiques fonctionnel (OAT) lorsqu'elle est cultivée, dans laquelle la cellule est ciPTEC.OAT1.4B2 DSM ACC3279 ou un passage jusqu'au 29ème passage de celle-ci ou dans laquelle la cellule est ciPTEC.OAT3.3C1 DSM ACC3280 ou un passage jusqu'au 29^{ème} passage de celle-ci.

2. Méthode de production d'une cellule épithéliale de tubule proximal humain qui exprime de manière stable un transporteur d'anions organiques lorsqu'elle est cultivée, ladite méthode comprenant les étapes consistant à :
i) effectuer une transduction d'une population de cellules épithéliales de tubule proximal par une particule lentivirale comprenant une construction d'expression qui comprend une séquence nucléotidique ayant au moins 50 % d'identité de séquence avec SEQ ID NO : 2 ou avec SEQ ID NO : 4,
ii) facultativement enrichir la population transduite obtenue en (i), de préférence en utilisant un tri cellulaire activé par fluorescence (FACS), et
iii) sous-cloner la population transduite obtenue en (i) de (ii) en sélectionnant et en isolant des cellules uniques et en les développant par culture
dans laquelle l'expression stable du transporteur d'anions organiques est une expression qui conduit à la présence ou à l'activité détectable du transporteur d'anions organiques pour au moins dix passages de ladite cellule.

3. La méthode selon la revendication 2, dans laquelle ladite construction d'expression a au moins 50 % d'identité de séquence avec une construction d'expression choisie dans le groupe consistant en :
i) pLenti4/V5-EX-CMV-TetO2-hOAT1 (SEQ ID NO : 25), et
ii) pLenti4/V5-EX-CMV-TetO2-hOAT3 (SEQ ID NO : 26).

4. Méthode d'analyse *in vitro* ou *ex vivo* d'une substance, comprenant la mise en contact de ladite substance avec au moins une cellule selon la revendication 1, de préférence avec une monocouche mature desdites cellules.

5. La méthode selon la revendication 4, dans laquelle ladite méthode consiste à déterminer la néphrotoxicité de ladite substance, et comprend en outre de préférence l'analyse ultérieure de la viabilité cellulaire, de préférence par l'analyse de la capacité de déshydrogénase cellulaire.

6. La méthode selon la revendication 4, dans laquelle ladite méthode est pour l'analyse fonctionnelle de l'interaction de ladite substance avec un transporteur, de préférence un transporteur rénal, et dans laquelle ladite mise en contact se fait de préférence en présence d'un substrat de transporteur anionique marqué, de préférence un substrat de transporteur anionique radiomarqué ou marqué par fluorescence.

7. La méthode selon l'une quelconque des revendications 4 ou 6, dans laquelle ladite méthode comprend en outre la détermination de l'interaction médicament-médicament de ladite substance.

8. La méthode selon l'une quelconque des revendications 4, 6 ou 7, dans laquelle ladite méthode comprend en outre la détermination que ladite substance est un substrat ou un inhibiteur d'un transporteur impliqué dans une interaction médicament-médicament cliniquement pertinente.

9. Kit de pièces comprenant une cellule telle que décrite la revendication 1 et des instructions d'utilisation.
